# EUROPEAN PATENT SPECIFICATION

(11) **EP 2 997 133 B1**
(45) Date of publication and mention of the grant of the patent: **23.08.2023**
(21) Application number: 14727627.3
(22) Date of filing: 13.05.2014
(51) Int. Cl.: C12N 5/0783, C07K 16/28, C07K 14/735, A61P 31/12, A61P 35/00, A61P 35/02, A61P 35/04

(54) **METHODS FOR ENGINEERING HIGHLY ACTIVE T CELL FOR IMMUNOTHERAPY**
VERFAHREN ZUR ZÜCHTUNG HOCHAKTIVER T-ZELLEN ZUR IMMUNTHERAPIE
PROCÉDÉS DE PRODUCTION, PAR GÉNIE GÉNÉTIQUE, D'UN LYMPHOCYTE T HAUTEMENT ACTIF À VOCATION IMMUNOTHÉRAPEUTIQUE

(30) Priority: 13.05.2013 US 201313892805; 13.05.2013 WO PCT/US2013/040755; 13.05.2013 WO PCT/US2013/040766; 22.11.2013 US 201361907858 P
(43) Date of publication of application: 23.03.2016
(73) Proprietor: Cellectis, 75013 Paris (FR)
(72) Inventor: GALETTO, Roman, 75014 Paris (FR); GOUBLE, Agnes, 75017 Paris (FR); GROSSE, Stephanie, 77750 Saint Cyr Sur Morin (FR); SCHIFFER-MANNIOUI, Cécile, 94350 Villiers sur Marne (FR); POIROT, Laurent, 75018 Paris (FR); SCHARENBERG, Andrew, Seattle Washington 98177 (US); SMITH, Julianne, 92350 Le Plessis Robinson (FR)
(74) Representative: Santarelli
(86) International application number: PCT/IB2014/061412
(87) International publication number: WO 2014/184744

(56) References cited:
- MIZOGUCHI E ET AL: "Pathogenic role of IL-4, but not IFN-gamma in colitis of TCRalpha knockout mice", GASTROENTEROLOGY, ELSEVIER, PHILADELPHIA, PA, vol. 114, 15 April 1998 (1998-04-15), page A1041, XP027469136, ISSN: 0016-5085, DOI: 10.1016/S0016-5085(98)84235-2 [retrieved on 1998-04-15]
- H. TORIKAI ET AL: "A foundation for universal T-cell based immunotherapy: T cells engineered to express a CD19-specific chimeric-antigen-receptor and eliminate expression of endogenous TCR", BLOOD, vol. 119, no. 24, 14 June 2012 (2012-06-14), pages 5697-5705, XP055071623, ISSN: 0006-4971, DOI: 10.1182/blood-2012-01-405365
- ZACHARY A. COOPER ET AL: "Combining checkpoint inhibitors and BRAF-targeted agents against metastatic melanoma", ONCOIMMUNOLOGY, vol. 2, no. 5, 1 May 2013 (2013-05-01), page e24320, XP055133315, ISSN: 2162-4011, DOI: 10.4161/onci.24320
- JEFFREY C MILLER ET AL: "A TALE nuclease architecture for efficient genome editing", NATURE BIOTECHNOLOGY, NATURE PUBLISHING GROUP, UNITED STATES, vol. 29, no. 2, 1 February 2011 (2011-02-01), pages 143-148, XP002685898, ISSN: 1546-1696, DOI: 10.1038/NBT.1755 [retrieved on 2010-12-22]
- M. M. MAHFOUZ ET AL: "De novo-engineered transcription activator-like effector (TALE) hybrid nuclease with novel DNA binding specificity creates double-strand breaks", PROCEEDINGS OF THE NATIONAL ACADEMY OF SCIENCES, vol. 108, no. 6, 8 February 2011 (2011-02-08), pages 2623-2628, XP055007615, ISSN: 0027-8424, DOI: 10.1073/pnas.1019533108
- PARTOW KEBRIAEI ET AL: "Infusing CD19-Directed T Cells to Augment Disease Control in Patients Undergoing Autologous Hematopoietic Stem-Cell Transplantation for Advanced B-Lymphoid Malignancies", HUMAN GENE THERAPY, vol. 23, no. 5, 1 May 2012 (2012-05-01), pages 444-450, XP055130799, ISSN: 1043-0342, DOI: 10.1089/hum.2011.167
- UTTENTHAL B J ET AL: "Challenges in T cell receptor gene therapy", JOURNAL OF GENE MEDICINE, JOHN WILEY & SONS, INC, US, vol. 14, no. 6, 1 June 2012 (2012-06-01), pages 386-399, XP002760096, ISSN: 1099-498X, DOI: 10.1002/JGM.2637 [retrieved on 2012-06-27]
- Michael S Magee ET AL: "Immunotherapeutic strategies to target prognostic and predictive markers of cancer", Biomarkers in Medicine, vol. 7, no. 1, 1 February 2013 (2013-02-01), pages 23-35, XP55486731, UK ISSN: 1752-0363, DOI: 10.2217/bmm.12.110
- JEFFREY WEBER: "Immune Checkpoint Proteins: A New Therapeutic Paradigm for Cancer-Preclinical Background: CTLA-4 and PD-1 Blockade", SEMINARS IN ONCOLOGY, W.B. SAUNDERS, US, vol. 37, no. 5, 1 October 2010 (2010-10-01), pages 430-439, XP009177527, ISSN: 0093-7754, DOI: 10.1053/J.SEMINONCOL.2010.09.005

## Description

### Field of the invention

The present invention relates to methods for developing engineered T-cells for immunotherapy and more specifically to methods for modifying T-cells by inactivating, immune checkpoint genes, to circumvent inhibitory mechanisms and enhance T-cell immune activity. This method involves the use of specific rare cutting endonucleases, in particular TALE-nucleases (TAL effector endonuclease) and polynucleotides encoding such polypeptides, to precisely target a selection of key genes in T-cells, which are available from donors or from culture of primary cells. The invention opens the way to highly efficient adoptive immunotherapy strategies for treating cancer and viral infections.

### Background of the invention

Adoptive immunotherapy, which involves the transfer of autologous antigen-specific T cells generated *ex vivo,* is a promising strategy to treat viral infections and cancer. The T cells used for adoptive immunotherapy can be generated either by expansion of antigen-specific T cells or redirection of T cells through genetic engineering (Park, Rosenberg et al. 2011). Transfer of viral antigen specific T cells is a well-established procedure used for the treatment of transplant associated viral infections and rare viralrelated malignancies. Similarly, isolation and transfer of tumor specific T cells has been shown to be successful in treating melanoma.

Novel specificities in T cells have been successfully generated through the genetic transfer of transgenic T cell receptors or chimeric antigen receptors (CARs) (Jena, Dotti et al. 2010). CARs are synthetic receptors consisting of a targeting moiety that is associated with one or more signaling domains in a single fusion molecule. In general, the binding moiety of a CAR consists of an antigen-binding domain of a single-chain antibody (scFv), comprising the light and variable fragments of a monoclonal antibody joined by a flexible linker. Binding moieties based on receptor or ligand domains have also been used successfully. The signaling domains for first generation CARs are derived from the cytoplasmic region of the CD3zeta or the Fc receptor gamma chains. CARs have successfully allowed T cells to be redirected against antigens expressed at the surface of tumor cells from various malignancies including lymphomas and solid tumors (Jena, Dotti et al. 2010).

With the aim of generating universal allogeneic TAA-specific T cells from one donor that might be administered to multiple recipients, Torikai et al engineered CAR⁺TCR^{neg} T cells by inactivating one gene, encoding a TCR chain (TCRct orTCRβ) (Torikai et al, 2012).

In a review article on T cell receptor gene therapy, Uttenthal et al disclose T-cells for immunotherapy, wherein a TCR gene is disrupted; this article does not disclose inactivation of a gene encoding an immune checkpoint protein (Uttenthal et al, 2012).

T-cell mediated immunity includes multiple sequential steps regulated by a balance between co-stimulatory and inhibitory signals that fine-tune the immune response. The inhibitory signals referred to as immune checkpoints are crucial for the maintenance of self-tolerance and also to limit immune-mediated collateral tissue damage. The ability of tumours to co-opt these inhibitory pathways represents an important mechanism in immune resistance and limits the success of immunotherapy. One of the promising approaches to activate a therapeutic T-cell immune response is the blockade of these immune checkpoints (Pardoll 2012). Immune checkpoints represent significant barriers to activation of functional cellular immunity in cancer, and antagonistic antibodies specific for inhibitory ligands on T cells including CTLA4 and programmed death-1 (PD-1) are examples of targeted agents being evaluated in the clinics.

Cytotoxic-T-lymphocyte-associated antigen 4 (CTLA-4; also known as CD152) downregulates the amplitude of T cell activation and treatment with antagonist CTLA4 antibodies (ipilimumab) has shown a survival benefit in patients with melanoma (Robert and Mateus 2011). Programmed cell death protein 1 (PD1 or PDCD1 also known as CD279) represents another very promising target for immunotherapy (Pardoll and Drake 2012; Pardoll 2012). In contrast to CTLA-4, PD1 limits T cell effector functions in peripheral tissue at the time of an inflammatory response to infection and limits autoimmunity. The first clinical trial with PD1 antibody shows some cases of tumour regression (Brahmer, Drake et al. 2010). Multiple additional immune checkpoint proteins represent promising targets for therapeutic blockade based on recent studies.

Although the precise molecular pathways by which these immune checkpoint proteins signal are poorly understood, immune-checkpoint proteins seem to use distinct mechanisms to inhibit T-cell activation and are potentially non-redundant. Use of at least two different antibodies to block multiple immune checkpoint ligands or receptors has been shown to produce synergic antitumor activities. Nevertheless, the rate of adverse events was increased among patients treated with a combination CTLA-4/PD-1 antibody therapy (Wolchok, Kluger et al.).

To avoid the adverse events of antibody tumor treatments, the inventors sought to engineer highly active T-cells for immunotherapy by inactivating at least two genes encoding immune-checkpoint proteins. Contrary to the immune-checkpoint blockade mediated by antibody treatment, checkpoint signal pathways are blocked specifically in engineered T-cells used for immunotherapy and the adverse events due to non-specific effects of the antibodies on the patient's immune system are avoided.

In particular, this was made possible by gene inactivation using specific TALE-nucleases directed against at least two immune checkpoint proteins, such as PD1 and CTLA-4. Inactivation of immune checkpoint genes in T lymphocytes permits significant proliferation and activity of the introduced lymphocytes. Thus, these modified T cells are expected to be highly active in patient's blood, where they can target tumor cells or infected cells.

In addition to the above conception of genetically modified T cells, which can be highly active, the inventors, by the use and design of specific TALE-nucleases, have concomitantly inactivated different genes in T-cells, thereby obtaining at least double mutants. As a matter of fact, double gene targeting by double-strand break has been so far unachieved in T cells due to the difficulty of yielding and maintaining T-cells in culture over time, to their low transformation rates, and loss during selection procedures. These difficulties result in a low probability of success for obtaining such cells.

Thus, one significant part of the invention is to have designed specific TALE-nucleases, allowing higher rates of DSB events within T-cells, which are well tolerated by the cells (especially upon co-transfection) and are able to target the selection of genes according to the invention. By using rare-cutting endonucleases, such as the TALE-nucleases described therein, the probability of obtaining double inactivation of the genes in the transfected T-cells was significantly increased, so that it now appears possible to produce engineered T cells available from donors on a regular basis, using standard procedures.

### Summary of the invention

The invention is defined in the appended claims. The summary of the disclosure and the detailed technical disclosure set out below may in some respects go beyond the disclosure of the invention per se, and may also provide technical background for related technical developments. It will be appreciated that this technical disclosure is not intended to define the invention as such (which is defined exclusively by the appended claims), but rather to place it in a broader technical context. Accordingly, it will be appreciated that the various spects described are not intended to portray as part of the invention subject-matter that does not fall within the scope of the appended claims. In one aspect, the present invention discloses methods to engineer T cells to make them suitable for immunotherapy purposes. The methods of the present invention more particularly allow the precise modification of the genome of cells relevant for immunotherapy by inactivating a gene encoding immune checkpoint proteins. The modified cells relevant for immunotherapy further comprise exogenous recombinant polynucleotides encoding Chimeric Antigen Receptors (CAR) for specific cell recognition. In another aspect, the present disclosure relates to the polypeptides and the polynucleotides, which encode the rare-cutting endonucleases, to precisely target the above genes encoding immune checkpoint proteins, thereby enabling the genetic modification of the Tcells for immunotherapy. The present disclosure provides more particularly specific target sequences within these genes and TALE-nucleases designed to respectively target those genes.

The present disclosure also relates to the isolated cells or cell lines comprising any of the proteins, polypeptides or vectors described herein. The T cells of the present invention comprise an inactivated immune checkpoint gene for their use in immunotherapy

According to the invention, the modified T cells are used as a therapeutic product, as an "off the shelf" product. In this respect, the T-cells according to the invention are further genetically modified, by inactivating at least one gene encoding a component of TCR (T cell receptor), to make the cells allogeneic and thereby suited for treating different patients from one donor's sample..

In a further aspect, the present disclosure concerns the method for treating or preventing cancer or infections in the patient by administrating an engineered T-cell obtainable by the above methods.

### Brief description of the figures and Tables

In addition to the preceding features, the invention further comprises other features which will emerge from the description which follows, as well as to the appended drawings. A more complete appreciation of the invention and many of the attendant advantages thereof will be readily obtained as the same becomes better understood by reference to the following Figures in conjunction with the detailed description below.

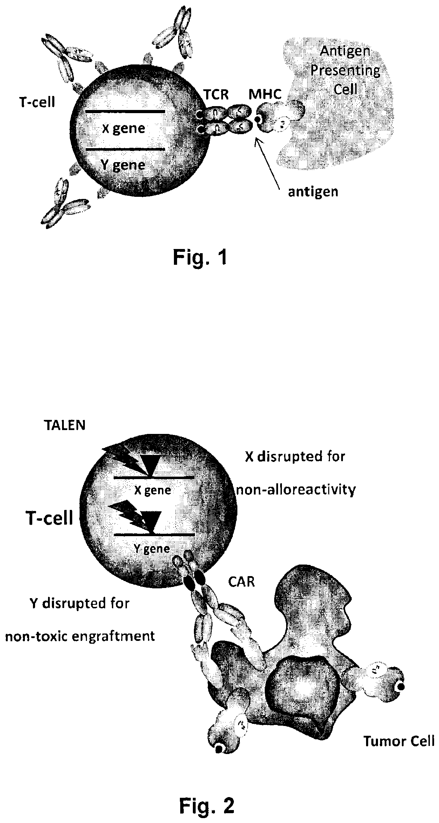

**Figure 1****:** Schematic representation of the normal relationship between T-cells and antigen presenting cell.
**Figure 2****:** Schematic representation of the genetically modified therapeutic T-cells according to the disclosure and the patient's tumor cells.
**Figure 3****:** Schematic representation of multi-chain CAR.
**Figure 4****:** Schematic of different versions of multi-chain CARs. A. Schematic of the FcεRl receptor. B-C Different versions of multi-chain CARs (csm1 to csm10) comprising a scFv and a CD8 stalk region fused to the transmembrane domain of FcεRl alpha chain. At least one 41BB, CD28 and/or CD3 zeta domains can be fused to a FcεRl alpha, beta and/or gamma chain.
**Figure 5****:** Schematic representation of one example of the method of engineering human allogenic cells for immunotherapy
**Figure 6****:** Concentration in cells per milliliter of live CD52-positive or CD52-negative cells after treatment with anti-CD52 antibody (CAMPATH1-H) with complement or controls.
**Figure 7****:** Comparison of the forward side scatter (FSC) distribution, an indicator of cell size, between TCR-positive and TCR-negative cells, or between CD52-positive and CD52-negative cells, and non activated cells as control.

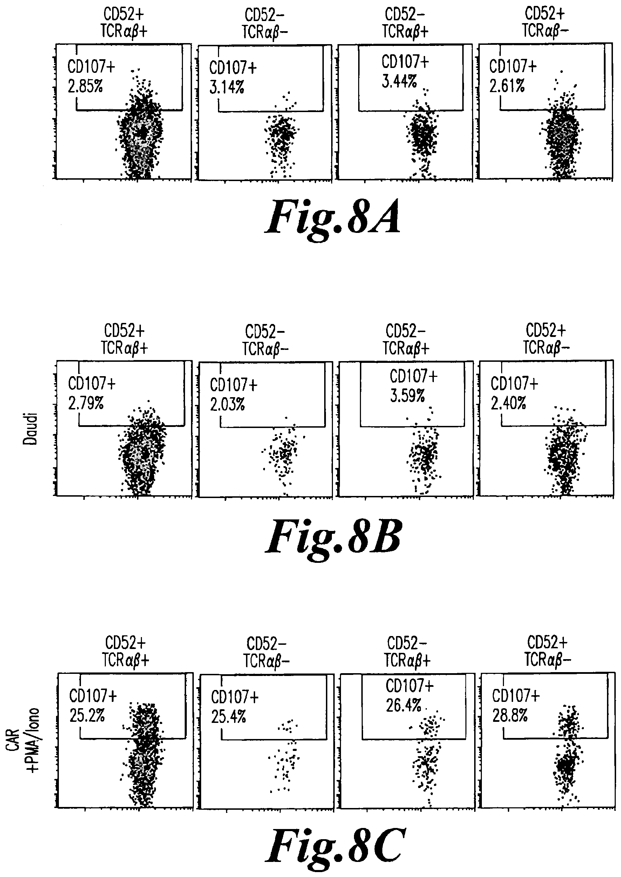

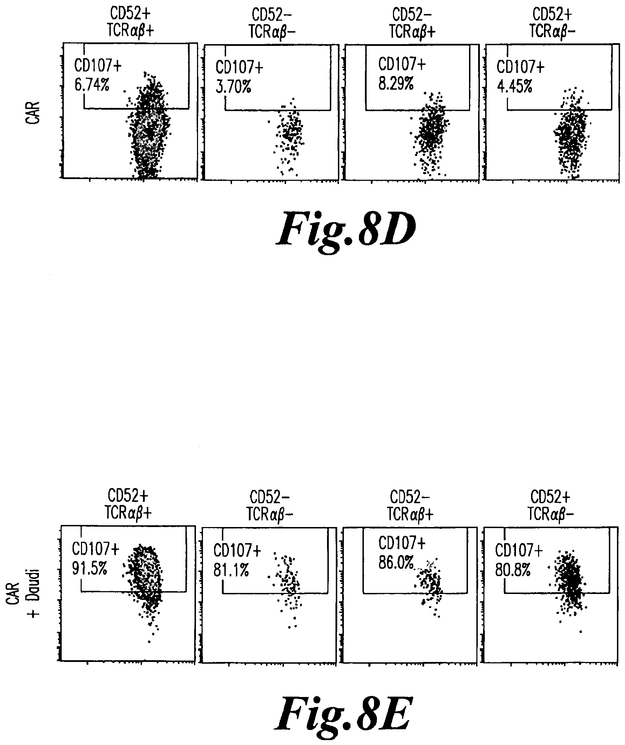

**Figure 8****:** Flow cytometry analysis of CD107a expression (marker of degranulation) on targeted CD52 and TCRalpha inactivated T cells. CD107 expression is analyzed on CD52+TCRαβ+ cells (first column), CD52-TCRαβ- cells (second column), CD52-TCRαβ+ cells (third column) and CD52+TCRαβ- cells (fourth column) before (A) and after incubation with Daudi cells (B); C) represents flow cytometry analysis of T cells further transfected with a CAR and incubated with Daudi cells; D) represents flow cytometry analysis of T cells transfected with a CAR but not incubated with Daudi cells and E) represents flow cytometry analysis of T cells transfected with a CAR and treated to PMA/ionomycin (positive control).
**Figure 9****:** Deep sequencing analysis of CD52 and TRAC TALE-nucleases potential off-site targets.
**Figure 10****:** Analysis of PDCD1 and CTLA-4 genomic locus by T7-endonuclease assay. Arrows point to digested PCR products.
**Figure 11****:** Schematic representation of some examples of preTalpha constructs.
**Figure 12****:** Flow cytometry analysis of transduction efficiency (% BFP+ cells) and activity of the FL, Δ18, Δ48 pTalpha constructs (% CD3 surface expression) in TCR alpha inactivated Jurkat cells.
**Figure 13****:** Schematic representation of a lentiviral construct coding for pTalpha protein (preTCRα).
**Figure 14****: A.** Representation of the experimental protocol. **B.**Flow cytometry analysis of TCR alpha/beta, CD3 expression and BFP expression on TCRalpha inactivated T cells (KO) transduced with either BFP-2A-pTalphaΔ48 (KO/Δ48) or control BFP lentiviral vector (KO/BFP) before and after purification. **C.** Flow cytometry analysis of TCR alpha/beta and CD3 expression on purified TCR alpha inactivated cells transduced (BFPpos) or not (BFPneg) with BFP-2A-pTalphaΔ48 lentiviral vector. NEP represents non electroporated cells with TRAC TALE-nucleases.

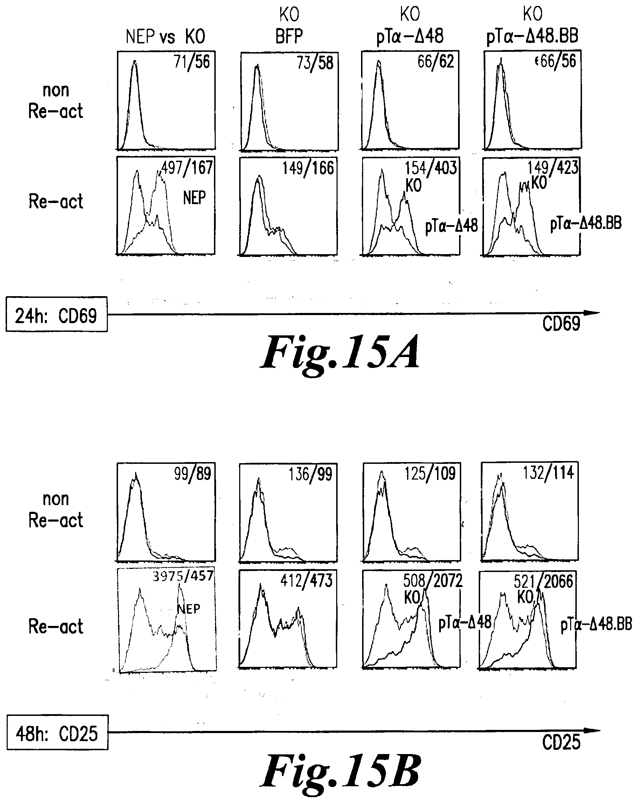

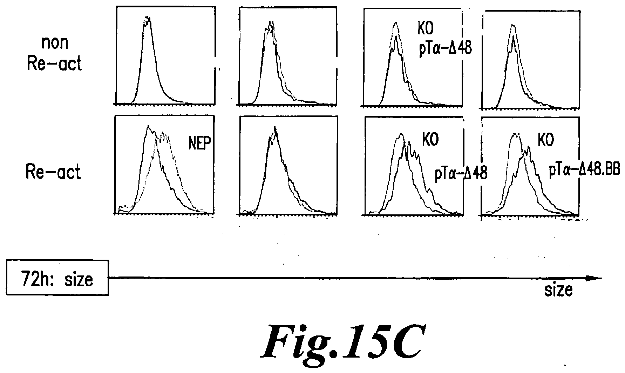

**Figure 15****: A-B.** Flow cytometry analysis of early activation marker CD69 (A), late activation marker CD25 (B) expression 24 and 48 hours after re-activation with anti-CD3/CD28 beads respectively on non electroporated cells (NEP) and TCRalpha inactivated cells (KO) transduced with BFP-2A-pTα-Δ48 lentiviral vector (pTα-Δ48), BFP-2A-pTα-Δ48.41BB lentiviral vector (pTα-Δ48.BB) or control BFP vector (BFP). pTα-Δ48 histograms correspond to the signal detected in TCR inactivated cells expressing pTα-Δ48 (BFP+ cells) while the KO histograms correspond to TCRalpha inactivated cells which do not express pTα-Δ48 (BFP-cells) pTα-Δ48.BB histograms correspond to the signal detected in TCR inactivated cells expressing pTα-Δ48.41BB (BFP+ cells) while the KO histograms correspond to TCRalpha inactivated cells which do not express pTα-Δ48.41BB (BFP- cells). NEP (non electroporated) histograms correspond to signal detected in non engineered cells. C. Flow cytometry analysis of the size of cells 72 hours after re-activation with anti-CD3/CD28 beads on non electroporated cells (NEP) and TCRalpha inactivated cells (KO) transduced with BFP-2A-pTα-Δ48 lentiviral vector (pTα-Δ48), BFP-2A-pTα-Δ48.41BB lentiviral vector (pTα-Δ48.BB) or control BFP vector (BFP). The values indicated in the upper part of each graph correspond to the geometrical mean of the fluorescence of each population.

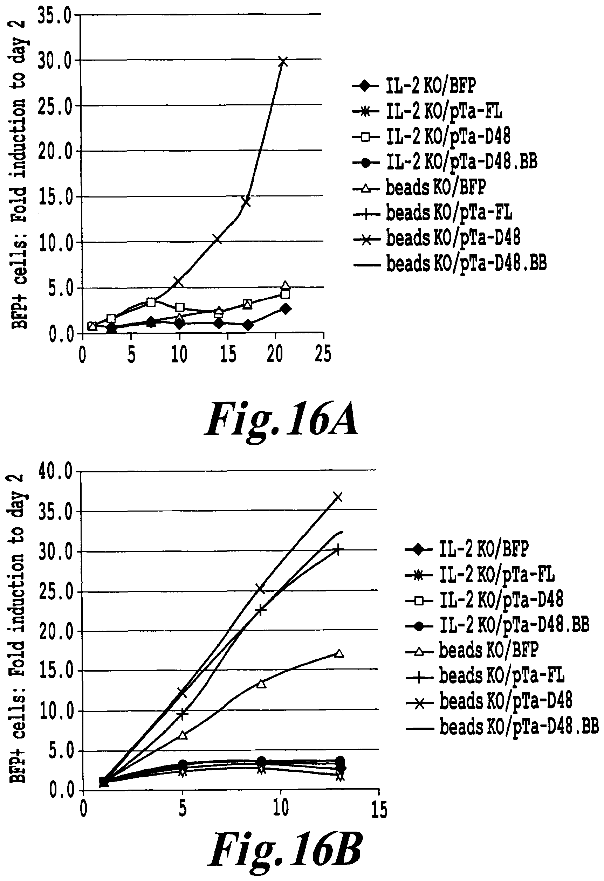

**Figure 16****:** Cell growth analysis of TCR alpha inactivated cells (KO) transduced with pTalpha-Δ48 (pTaΔ48) or control BFP vector (BFP) maintained in IL2 or in IL2 with anti- CD3/CD28 beads at different time points (x-axis). The BFP+ cells number is estimated at different time points for each condition and the fold induction of these cells (y-axis) was estimated with respect to the value obtained at day 2 post re-activation. The results are obtained from two independent donors. For the second donor, cell growth was also determined for cells transduced with pTalpha-Δ48.41BB (pTa-Δ48.BB) and full-length pTalpha- (pTa-FL).
**Figure 17****:** Flow cytometry analysis of GFP positive cells on PBMCs electroporated with the five different Cytopulse programs. The upper line corresponds to transfection of 6×10⁶ cells per cuvette, while the lower line corresponds to transfection of 3×10⁶ cells per cuvette.
**Figure 18****:** Flow cytometry analysis of purified T cell mortality using viability dye (eFluor-450) and of GFP positive cells among the viable population after electroporation with GFP mRNA, GFP DNA and control pUC DNA. NEP corresponds to cells that were maintained in electroporation buffer but were not electroporated and NT corresponds to non electroporated cells maintained in culture medium.
**Figure 19****:** Flow cytometry analysis of TCR alpha/beta and CD3 expression on human primary T cells following TRAC TALE-nuclease mRNA electroporation (top). Deep sequencing analysis of genomic DNA extracted from human primary T cells following TRAC TALE-nuclease mRNA electroporation (bottom).
**Figure 20****: A.** Flow cytometry analysis of CAR expression (anti F(ab')2 ) after electroporation of T cells with or without mRNA encoding a single chain CAR. **B**. Flow cytometry analysis of CD107a expression (marker of degranulation) on electroporated T cells cocultured with daudi cells.
**Figure 21****: A.** Representation of mRNA encoding a multi-chain CAR. **B.** Flow cytometry analysis of CAR expression (anti F(ab')2 ) on viable T cells electroporated with or without a polycistronic mRNA encoding a multi-chain CAR. **C.** Flow cytometry analysis of CD107a expression (marker of degranulation) on electroporated T cells cocultured with daudi cells.

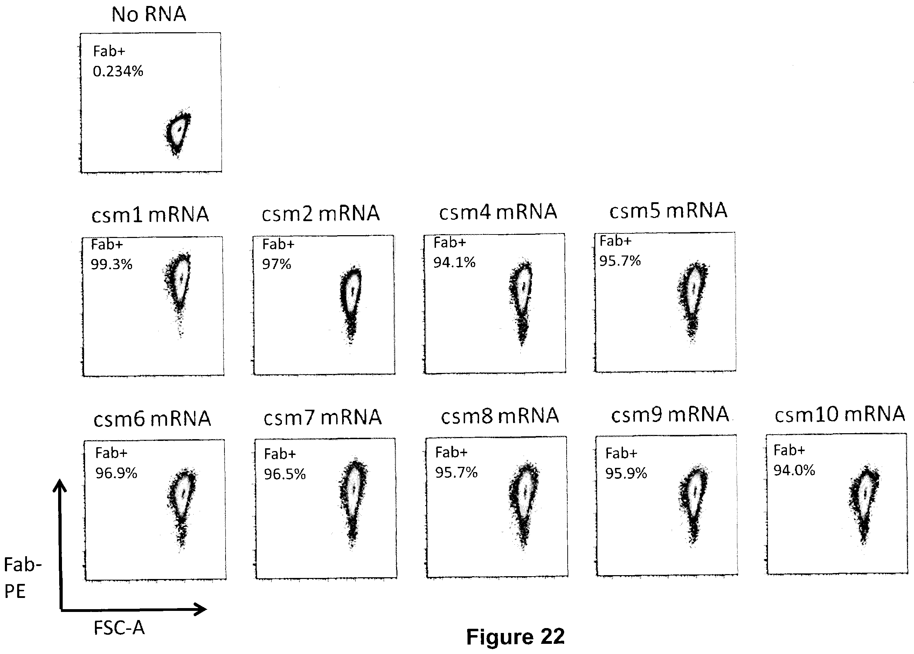

**Figure 22****:** Multi-chain CARs expression in human T cells after electroporation of polycistronic mRNAs.

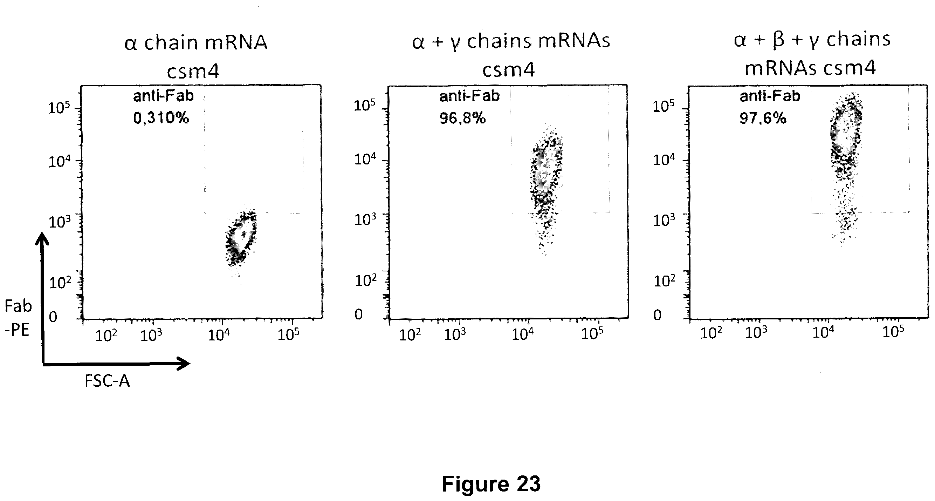

**Figure 23****:** The expression of the multi-subunit CARs is conditioned by the expression of the three chains: α, β and γ.

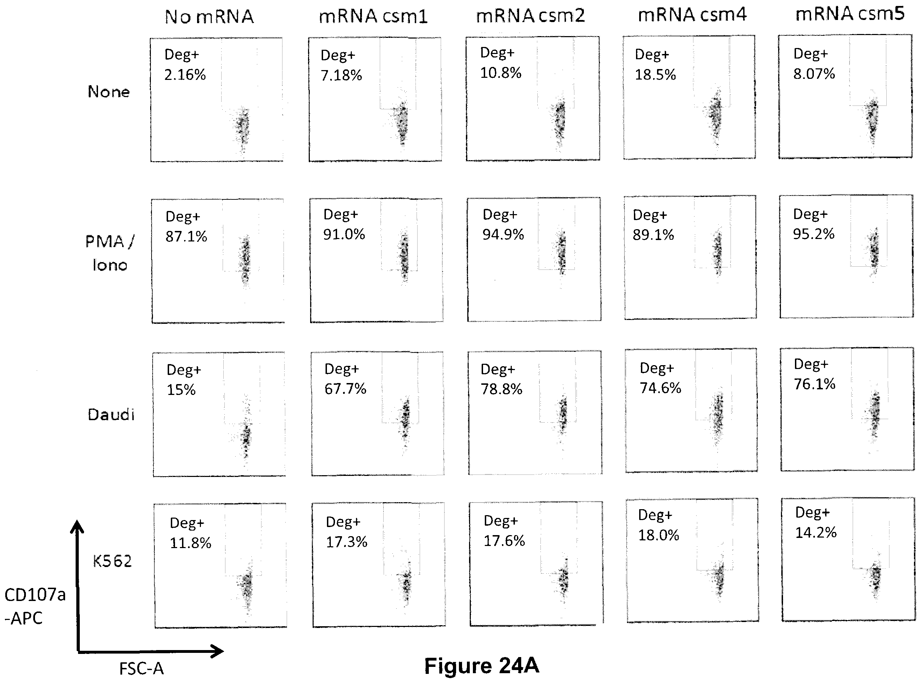

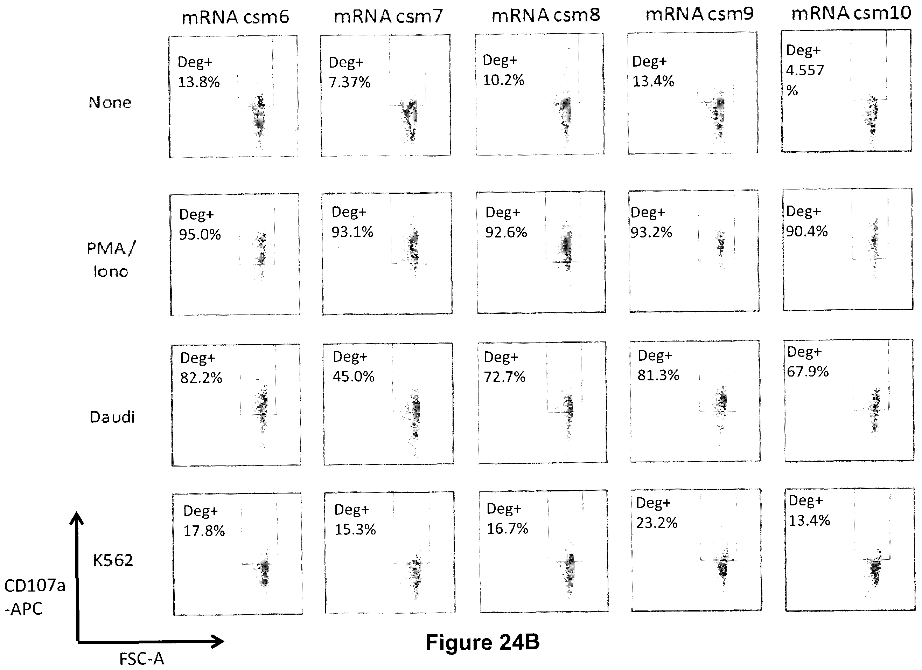

**Figure 24****:** The human T cells transiently expressing the multi-chain CARs degranulate following coculture with target cells. A: csm1 to csm5 CAR constructs. B: csm6 to csm10 CAR constructs.

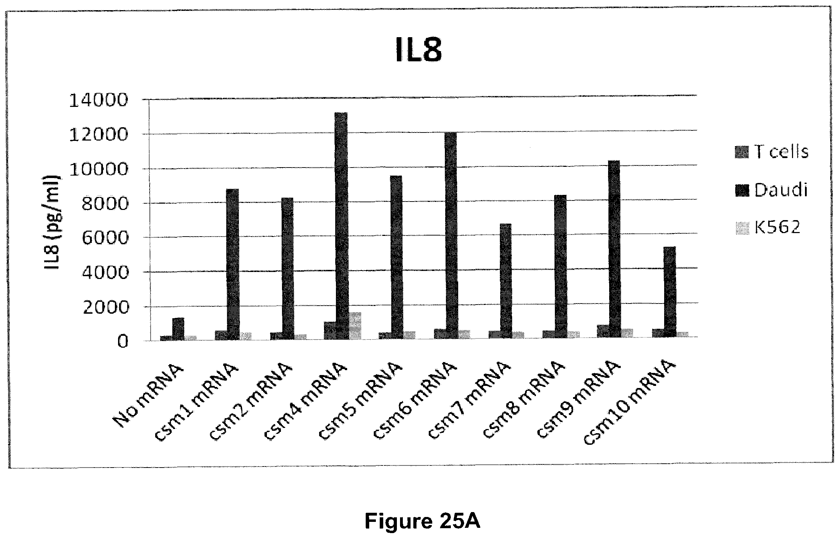

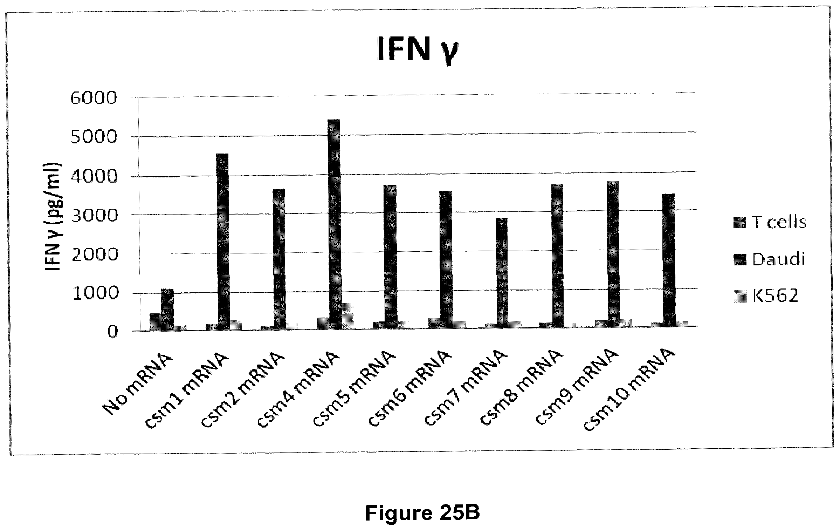

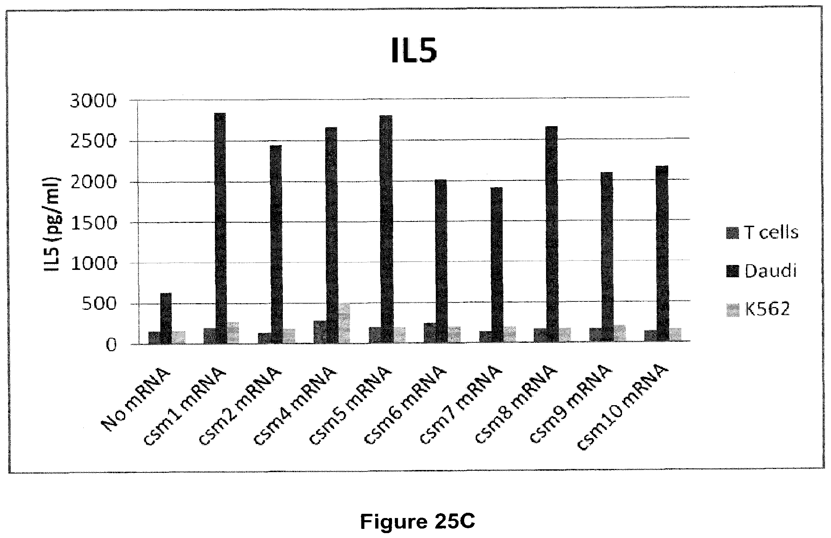

**Figure 25****:** The human T cells transiently expressing the multi-chain CARs secrete cytokines following coculture with target cells (Tcells vs. Daudi cells or K562). A:IL8 release. B:IFNy release. C:IL5 release.

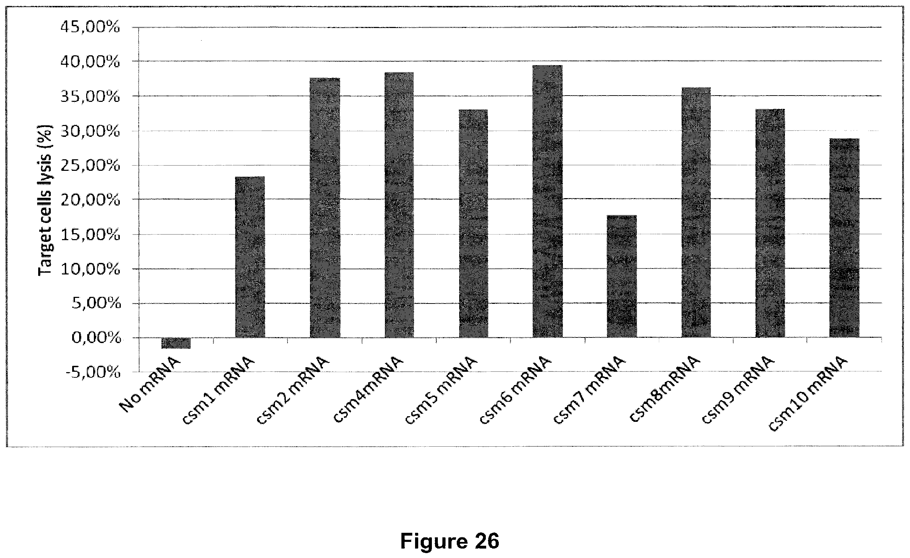

**Figure 26****:** The human T cells transiently expressing the multi-chain CARs (semi to csm10 constructs) lyse target cells.
Figure 27: CTLA4 inactivation in primary T cells measured by intracellular staining using fluorescent antibody and flow cytometry analysis.
**Figure 28****:** distribution of fluorescent T-cells expressing CTLA4 upon transfection with TALENs T1, T2 and T3.Proportion of cells expressing CTLA4 is dramatically reduced with respect to control cells.
**Figure 29****:** PD1 inactivation in primary T cells measured by intracellular staining using fluorescent antibody and flow cytometry analysis. Proportion of cells expressing PD1 is dramatically reduced with respect to control cells.
**Figure 30****:** Diagram showing deletions frequencies observed in T-cells upon transfection with TALEN T01 and T03 targeting PD1 gene.
**Figure 31****:** Diagram showing that cytotoxic activity is enhanced in T-cells disrupted for PD1 as per the experiment described in Example 3.

**Table 1:** List of immune checkpoint genes identified by the inventors as appropriate to make allogeneic T-cells more active for immunotherapy.
**Table2:** Description of the GR TALE-nucleases and sequences of the TALE-nucleases target sites in the human GR gene.
**Table 3:** Cleavage activity of the GR TALE-nucleases in yeast. Values are comprised between 0 and 1. Maximal value is 1.
**Table 4:** Percentage of targeted mutagenesis at endogenous TALE-nuclease target sites in 293 cells.
**Table 5:** Percentage of targeted mutagenesis at endogenous TALE-nuclease target sites in primary T lymphocytes.
**Table 6:** Description of the CD52, TRAC and TRBC TALE-nucleases and sequences of the TALE-nucleases target sites in the human corresponding genes.
**Table 7:** Additional target sequences for TRAC and CD52 TALE-nucleases.
**Table 8:** Percentage of indels for TALE-nuclease targeting CD52_T02, TRAC_T01, TRBC_T01 and TRBC_T02 targets.
**Table 9:** Percentages of CD52- negative, TCR-negative and CD52/TCR-double negative T lymphocytes after transfection of corresponding TALE-nuclease-expressing polynucleotides.
**Table 10:** Percentages of TCR-negative T lymphocytes after transfection of TRBC TALE-nuclease-expressing polynucleotides.
**Table 11:** Description of the CTLA4 and PDCD1 TALE-nucleases and sequences of the TALE-nucleases target sites in the human corresponding genes.
**Table 12:** Description of a subset of pTalpha constructs.
**Table 13:** Activity of the different pTalpha constructs in Jurkat TCR alpha inactivated cell.
Activity was measured by flow cytometry analysis of CD3 expression on jurkat TCR alpha inactivated cell transfected with the different preTalpha constructs.
**Table 14:** Different cytopulse programs used to determine the minimal voltage required for electroporation in PBMC derived T-cells.
**Table 15:** Cytopulse program used to electroporate purified T-cells.
**Table 16:** List of genes encoding immune checkpoint proteins.

### Detailed description of the invention

Unless specifically defined herein, all technical and scientific terms used have the same meaning as commonly understood by a skilled artisan in the fields of gene therapy, biochemistry, genetics, and molecular biology.

All methods and materials similar or equivalent to those described herein can be used in the practice or testing of the present disclosure, with suitable methods and materials being described herein. The materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In a general aspect, the present disclosure relates to methods for new adoptive immunotherapy strategies in treating cancer and infections.

### Highly active T cells for immunotherapy

According to a first aspect of the present disclosure, the inventors have identified new genes encoding so-called "immune checkpoints" function that can be individually disrupted to enhance the efficiency of T cells in adoptive immunotherapy. Table 1, below, lists these new targeted genes, which are more particularly involved into the functions of co-inhibitory receptor function, cell death, cytokine signaling, arginine tryptophan starvation, TCR signaling, Induced T-reg repression, transcription factors controlling exhaustion or anergy, and hypoxia mediated tolerance. As previously mentioned, the targeting of one of these later functions, through disruption or expression control of one these genes, can be made as part of an autologous or allogeneic scheme treatment

The invention thus more particularly relates to engineered T-cells, in which at least one of the genes having identity with one mentioned in Table 1 is disrupted or repressed for the purpose of performing immunotherapy.

T cell-mediated immunity includes multiple sequential steps involving the clonal selection of antigen specific cells, their activation and proliferation in secondary lymphoid tissue, their trafficking to sites of antigen and inflammation, the execution of direct effector function and the provision of help (through cytokines and membrane ligands) for a multitude of effector immune cells. Each of these steps is regulated by counterbalancing stimulatory and inhibitory signals that fine-tune the response. Although the molecular mechanism for inhibitory signals in most cases has not been defined, studies suggest that negative regulatory components might attenuate T-cell response in more than one way. For example, CTLA-4 can compete with CD28 for costimulatory ligands. In contrast, inhibitory motif-containing molecules such as PD-1 can recruit phosphatases (such as SHP-1, SHP-2 or SHIP) to TCR-proximal signaling complexes and attenuate signaling. Both PD-1 and CTLA-4 can inhibit signaling by the serine-threonine kinase Akt but seem to do so by different molecular mechanisms. Thus, inactivation of one of the genes can be compensated by another negative regulatory pathway. To circumvent this, inactivation of at least two genes encoding immune-checkpoint proteins to sufficiently augment the activity of T-cells for immunotherapy is described herein. Based on the classification shown in table 1, different genes encoding proteins preferably involved in different inhibitory pathways that negatively regulate the immune response are chosen.

The present disclosure relates to a method for preparing T-cells for immunotherapy by inactivating in T-cells at least two of the genes encoding immune-checkpoint proteins to sufficiently increase the activity of T-cells for immunotherapy. In particular, the present disclosure relates to a method of preparing T-cells for immunotherapy comprising:
(a) Modifying T-cells by inactivating at least two genes encoding immune checkpoint proteins;
(b) Expanding said cells.

It will be understood by those of ordinary skill in the art, that the term "immune checkpoints" means a group of molecules expressed by T cells. These molecules effectively serve as "brakes" to down-modulate or inhibit an immune response. Immune checkpoint molecules include, but are not limited to Programmed Death 1 (PD-1, also known as PDCD1 or CD279, accession number: NM_005018), Cytotoxic T-Lymphocyte Antigen 4 (CTLA-4, also known as CD152, GenBank accession number AF414120.1), LAG3 (also known as CD223, accession number: NM_002286.5), Tim3 (also known as HAVCR2, GenBank accession number: JX049979.1), BTLA (also known as CD272, accession number: NM_181780.3), BY55 (also known as CD160, GenBank accession number: CR541888.1), TIGIT (also known as IVSTM3, accession number: NM_173799), LAIR1 (also known as CD305, GenBank accession number: CR542051.1, (Meyaard, Adema et al. 1997)), SIGLEC10 (GeneBank accession number: AY358337.1), 2B4 (also known as CD244, accession number: NM_001166664.1), PPP2CA, PPP2CB, PTPN6, PTPN22, CD96, CRTAM, SIGLEC7 (Nicoll, Ni et al. 1999), SIGLEC9 (Zhang, Nicoll et al. 2000; Ikehara, Ikehara et al. 2004), TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF (Quigley, Pereyra et al. 2010), GUCY1A2, GUCY1A3, GUCY1B2, GUCY1B3 which directly inhibit immune cells. For example, CTLA-4 is a cellsurface protein expressed on certain CD4 and CD8 T cells; when engaged by its ligands (B7-1 and B7-2) on antigen presenting cells, T-cell activation and effector function are inhibited. Thus the present invention relates to a method of engineering T-cells, especially for immunotherapy, comprising genetically modifying T-cells by inactivating at least one protein involved in the immune check-point, in particular PD1 and/or CTLA-4.

Table 1 below, without being exhaustive, shows immune checkpoint genes that can be inactivated according to the teaching of the present invention in order to improve the efficiency and fitness of the engineered T-cells. The immune checkpoints genes are preferably selected from such genes having identity to those listed in this table involved into co-inhibitory receptor function, cell death, cytokine signaling, arginine tryptophan starvation, TCR signaling, Induced T-reg repression, transcription factors controlling exhaustion or anergy, and hypoxia mediated tolerance.

**Table 1: Immune checkpoint genes appropriate to make allogeneic T-cells more active for immunotherapy**

| **Pathway** | **Genes that can be inactivated in pathway** | **NCBI database gene ID** (*Homo sapiens*) on May 13^{th}, 2014 |
|---|---|---|
| Co-inhibitory receptors | LAG3 (CD223) | 3902 |
| | HAVCR2 (TIM3) | 84868 |
| | BTLA (CD272) | 151888 |
| | CD160 (NK1) | 11126 |
| | TIGIT (VSIG9) | 201633 |
| | CD96 (TACTILE) | 10225 |
| | CRTAM (CD355) | 56253 |
| | LAIR1 (CD305) | 3903 |
| | SIGLEC7 (CD328) | 27036 |
| | A2A (IGKV2-29) | 28882 |
| | SIGLEC9 (CD329) | 27180 |
| | CD244 (2B4)) | 51744 |
| Cell death | TNFRSF10B (CD262) | 8795 |
| | TNFRSF10A (CD261) | 8797 |
| | CASP3 | 836 |
| | CASP6 | 839 |
| | CASP7 | 840 |
| | CASP8 | 841 |
| | CASP10 | 843 |
| | Arhgap5 (GFI2) | 394 |
| | Akap8i | 10270 |
| | FADD (GIG3) | 8772 |
| | FAS (RP11) | 355 |
| | Stk17b (DRAK2) | 9262 |
| Cytokine signalling | TGFBRII (AAT3) | 7048 |
| | TGFBRI | 7046 |
| | SMAD2 (JV18) | 4087 |
| | SMAD3 | 4088 |
| | SMAD4 | 4089 |
| | SMAD10 (SMAD7) | 394331 |
| | SKI (SGS) | 6497 |
| | SKIL (SNO) | 6498 |
| | TGIF1 (HPE4) | 7050 |
| | IL10RA (CD210) | 3587 |
| | IL10RB | 3588 |
| | HMOX2 (HO-2) | 3163 |
| | Jun (AP1) | 3725 |
| | Ppp3cc | 5533 |
| | Ppm1g | 5496 |
| | Socs1 | 8651 |
| | Soc3 | 9021 |
| | IL6R (CD126) | 3570 |
| | IL6ST (CD130) | 3572 |
| | Lck | 3932 |
| | Fyn | 2534 |
| | ADAP (FYB) | 2533 |
| | Carma1 (CARD11) | 84433 |
| | Bcl10 | 8915 |
| | Malt1 (IMD12) | 10892 |
| | TAK1(NR2C2) | 7182 |
| arginine/tryptophan starvation | EIF2AK4 (GCN2) | 440275 |
| | Nuak2 | 81788 |
| TCR signalling | CSK | 1445 |
| | PAG1 (CBP) | 55824 |
| | SIT1 | 27240 |
| | CRTAM (CD355) | 56253 |
| | Egr2 (AT591) | 1959 |
| | DGK-a (DAGK) | 1606 |
| | DGK-z | 8525 |
| | Cblb | 868 |
| | Inpp5b | 3633 |
| | Ptpn2 (PTN2) | 5771 |
| | Vamp7 | 6845 |
| | Mast2 | 23139 |
| | tnk1 | 8711 |
| | stk17b (DRAK2) | 9262 |
| | Mdfic (HIC) | 29969 |
| | F11r (CD321) | 50848 |
| Induced Treg | FOXP3 (JM2) | 50943 |
| | Entpd1 (CD39) | 953 |
| Transcription factors controlling exhaustion/anergy | PRDM1 (blimp1) | 12142 |
| | BATF | 10538 |
| | Ypel2 | 388403 |
| | Ppp2r2d | 55844 |
| | Rock1 | 6093 |
| | Sbf1 | 6305 |
| | Hipk1 (MYAK) | 204851 |
| | Map3k3 | 4215 |
| | Grk6 | 2870 |
| | Eif2ak3 (PEK) | 9451 |
| | Fyn | 2534 |
| | NFAT1(NFATC2) | 4773 |
| Hypoxia mediated tolerance | GUCY1A2 | 2977 |
| | GUCY1A3 | 2982 |
| | GUCY1B2 | 2974 |
| | GUCY1B3 | 2983 |

For example, said two genes encoding immune checkpoint proteins are selected from the group consisting of: CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3.

Said two genes encoding immune checkpoint proteins can be selected from the group consisting of: BTLA and BATF ; CASP10 and BATF ; CASP10 and BTLA ; CASP3 and BATF ; CASP3 and BTLA ; CASP3 and CASP10 ; CASP6 and BATF ; CASP6 and BTLA ; CASP6 and CASP10 ; CASP6 and CASP3 ; CASP7 and BATF ; CASP7 and BTLA ; CASP7 and CASP10 ; CASP7 and CASP3 ; CASP7 and CASP6 ; CASP8 and BATF ; CASP8 and BTLA ; CASP8 and CASP10 ; CASP8 and CASP3 ; CASP8 and CASP6 ; CASP8 and CASP7 ; CD160 and BATF ; CD160 and BTLA ; CD160 and CASP10 ; CD160 and CASP3 ; CD160 and CASP6 ; CD160 and CASP7 ; CD160 and CASP8 ; CD244 and BATF ; CD244 and BTLA ; CD244 and CASP10 ; CD244 and CASP3 ; CD244 and CASP6 ; CD244 and CASP7 ; CD244 and CASP8 ; CD244 and CD160 ; CD96 and BATF ; CD96 and BTLA ; CD96 and CASP10 ; CD96 and CASP3 ; CD96 and CASP6 ; CD96 and CASP7 ; CD96 and CASP8 ; CD96 and CD160 ; CD96 and CD244 ; CRTAM and BATF ; CRTAM and BTLA ; CRTAM and CASP10 ; CRTAM and CASP3 ; CRTAM and CASP6 ; CRTAM and CASP7 ; CRTAM and CASP8 ; CRTAM and CD160 ; CRTAM and CD244 ; CRTAM and CD96 ; CSK and BATF ; CSK and BTLA ; CSK and CASP10 ; CSK and CASP3 ; CSK and CASP6 ; CSK and CASP7 ; CSK and CASP8 ; CSK and CD160 ; CSK and CD244 ; CSK and CD96 ; CSK and CRTAM ; CTLA4 and BATF ; CTLA4 and BTLA ; CTLA4 and CASP10 ; CTLA4 and CASP3 ; CTLA4 and CASP6 ; CTLA4 and CASP7 ; CTLA4 and CASP8 ; CTLA4 and CD160 ; CTLA4 and CD244 ; CTLA4 and CD96 ; CTLA4 and CRTAM ; CTLA4 and CSK ; EIF2AK4 and BATF ; EIF2AK4 and BTLA ; EIF2AK4 and CASP10 ; EIF2AK4 and CASP3 ; EIF2AK4 and CASP6 ; EIF2AK4 and CASP7 ; EIF2AK4 and CASP8 ; EIF2AK4 and CD160 ; EIF2AK4 and CD244 ; EIF2AK4 and CD96 ; EIF2AK4 and CRTAM ; EIF2AK4 and CSK ; EIF2AK4 and CTLA4 ; FADD and BATF ; FADD and BTLA ; FADD and CASP10 ; FADD and CASP3 ; FADD and CASP6 ; FADD and CASP7 ; FADD and CASP8 ; FADD and CD160 ; FADD and CD244 ; FADD and CD96 ; FADD and CRTAM ; FADD and CSK ; FADD and CTLA4 ; FADD and EIF2AK4 ; FAS and BATF ; FAS and BTLA ; FAS and CASP10 ; FAS and CASP3 ; FAS and CASP6 ; FAS and CASP7 ; FAS and CASP8; FAS and CD160 ; FAS and CD244 ; FAS and CD96 ; FAS and CRTAM ; FAS and CSK; FAS and CTLA4 ; FAS and EIF2AK4 ; FAS and FADD ; FOXP3 and BATF ; FOXP3 and BTLA ; FOXP3 and CASP10 ; FOXP3 and CASP3 ; FOXP3 and CASP6 ; FOXP3 and CASP7 ; FOXP3 and CASP8 ; FOXP3 and CD160 ; FOXP3 and CD244 ; FOXP3 and CD96 ; FOXP3 and CRTAM ; FOXP3 and CSK ; FOXP3 and CTLA4 ; FOXP3 and EIF2AK4 ; FOXP3 and FADD ; FOXP3 and FAS ; GUCY1A2 and BATF ; GUCY1A2 and BTLA ; GUCY1A2 and CASP10 ; GUCY1A2 and CASP3 ; GUCY1A2 and CASP6 ; GUCY1A2 and CASP7 ; GUCY1A2 and CASP8 ; GUCY1A2 and CD160 ; GUCY1A2 and CD244 ; GUCY1A2 and CD96 ; GUCY1A2 and CRTAM ; GUCY1A2 and CSK ; GUCY1A2 and CTLA4 ; GUCY1A2 and EIF2AK4 ; GUCY1A2 and FADD ; GUCY1A2 and FAS ; GUCY1A2 and FOXP3 ; GUCY1A3 and BATF ; GUCY1A3 and BTLA ; GUCY1A3 and CASP10 ; GUCY1A3 and CASP3 ; GUCY1A3 and CASP6 ; GUCY1A3 and CASP7 ; GUCY1A3 and CASP8 ; GUCY1A3 and CD160 ; GUCY1A3 and CD244 ; GUCY1A3 and CD96 ; GUCY1A3 and CRTAM ; GUCY1A3 and CSK ; GUCY1A3 and CTLA4 ; GUCY1A3 and EIF2AK4 ; GUCY1A3 and FADD ; GUCY1A3 and FAS ; GUCY1A3 and FOXP3 ; GUCY1A3 and GUCY1A2 ; GUCY1B2 and BATF ; GUCY1B2 and BTLA ; GUCY1B2 and CASP10 ; GUCY1B2 and CASP3 ; GUCY1B2 and CASP6 ; GUCY1B2 and CASP7 ; GUCY1B2 and CASP8 ; GUCY1B2 and CD160 ; GUCY1B2 and CD244 ; GUCY1B2 and CD96 ; GUCY1B2 and CRTAM ; GUCY1B2 and CSK ; GUCY1B2 and CTLA4 ; GUCY1B2 and EIF2AK4 ; GUCY1B2 and FADD ; GUCY1B2 and FAS ; GUCY1B2 and FOXP3 ; GUCY1B2 and GUCY1A2 ; GUCY1B2 and GUCY1A3 ; GUCY1B3 and BATF ; GUCY1B3 and BTLA ; GUCY1B3 and CASP10 ; GUCY1B3 and CASP3 ; GUCY1B3 and CASP6 ; GUCY1B3 and CASP7 ; GUCY1B3 and CASP8 ; GUCY1B3 and CD160 ; GUCY1B3 and CD244 ; GUCY1B3 and CD96 ; GUCY1B3 and CRTAM ; GUCY1B3 and CSK ; GUCY1B3 and CTLA4 ; GUCY1B3 and EIF2AK4 ; GUCY1B3 and FADD ; GUCY1B3 and FAS ; GUCY1B3 and FOXP3 ; GUCY1B3 and GUCY1A2 ; GUCY1B3 and GUCY1A3 ; GUCY1B3 and GUCY1B2 ; HAVCR2 and BATF ; HAVCR2 and BTLA ; HAVCR2 and CASP10 ; HAVCR2 and CASP3 ; HAVCR2 and CASP6 ; HAVCR2 and CASP7 ; HAVCR2 and CASP8 ; HAVCR2 and CD160 ; HAVCR2 and CD244 ; HAVCR2 and CD96 ; HAVCR2 and CRTAM ; HAVCR2 and CSK ; HAVCR2 and CTLA4 ; HAVCR2 and EIF2AK4 ; HAVCR2 and FADD ; HAVCR2 and FAS ; HAVCR2 and FOXP3 ; HAVCR2 and GUCY1A2 ; HAVCR2 and GUCY1A3 ; HAVCR2 and GUCY1B2 ; HAVCR2 and GUCY1B3 ; HMOX2 and BATF ; HMOX2 and BTLA ; HMOX2 and CASP10 ; HMOX2 and CASP3 ; HMOX2 and CASP6 ; HMOX2 and CASP7 ; HMOX2 and CASP8 ; HMOX2 and CD160 ; HMOX2 and CD244 ; HMOX2 and CD96 ; HMOX2 and CRTAM ; HMOX2 and CSK ; HMOX2 and CTLA4 ; HMOX2 and EIF2AK4; HMOX2 and FADD ; HMOX2 and FAS ; HMOX2 and FOXP3 ; HMOX2 and GUCY1A2 ; HMOX2 and GUCY1A3 ; HMOX2 and GUCY1B2 ; HMOX2 and GUCY1B3 ; HMOX2 and HAVCR2 ; IL10RA and BATF ; IL10RA and BTLA ; IL10RA and CASP10 ; IL10RA and CASP3 ; IL10RA and CASP6 ; IL10RA and CASP7 ; IL10RA and CASP8 ; IL10RA and CD160 ; IL10RA and CD244 ; IL10RA and CD96 ; IL10RA and CRTAM ; IL10RA and CSK ; IL10RA and CTLA4 ; IL10RA and EIF2AK4 ; IL10RA and FADD ; IL10RA and FAS ; IL10RA and FOXP3 ; IL10RA and GUCY1A2 ; IL10RA and GUCY1A3 ; IL10RA and GUCY1B2 ; IL10RA and GUCY1B3 ; IL10RA and HAVCR2 ; IL10RA and HMOX2 ; IL10RB and BATF ; IL10RB and BTLA ; IL10RB and CASP10 ; IL10RB and CASP3 ; IL10RB and CASP6 ; IL10RB and CASP7 ; IL10RB and CASP8 ; IL10RB and CD160 ; IL10RB and CD244; IL10RB and CD96 ; IL10RB and CRTAM ; IL10RB and CSK; IL10RB and CTLA4 ; IL10RB and EIF2AK4 ; IL10RB and FADD ; IL10RB and FAS ; IL10RB and FOXP3 ; IL10RB and GUCY1A2 ; IL10RB and GUCY1A3 ; IL10RB and GUCY1B2 ; IL10RB and GUCY1B3 ; IL10RB and HAVCR2 ; IL10RB and HMOX2 ; IL10RB and IL10RA ; IL6R and BATF ; IL6R and BTLA ; IL6R and CASP10 ; IL6R and CASP3 ; IL6R and CASP6 ; IL6R and CASP7 ; IL6R and CASP8 ; IL6R and CD160 ; IL6R and CD244 ; IL6R and CD96 ; IL6R and CRTAM ; IL6R and CSK; IL6R and CTLA4 ; IL6R and EIF2AK4; IL6R and FADD ; IL6R and FAS ; IL6R and FOXP3 ; IL6R and GUCY1A2 ; IL6R and GUCY1A3 ; IL6R and GUCY1B2 ; IL6R and GUCY1B3 ; IL6R and HAVCR2 ; IL6R and HMOX2 ; IL6R and IL10RA ; IL6R and IL10RB ; IL6ST and BATF ; IL6ST and BTLA ; IL6ST and CASP10 ; IL6ST and CASP3 ; IL6ST and CASP6 ; IL6ST and CASP7 ; IL6ST and CASP8 ; IL6ST and CD160 ; IL6ST and CD244 ; IL6ST and CD96 ; IL6ST and CRTAM ; IL6ST and CSK ; IL6ST and CTLA4 ; IL6ST and EIF2AK4 ; IL6ST and FADD ; IL6ST and FAS ; IL6ST and FOXP3 ; IL6ST and GUCY1A2 ; IL6ST and GUCY1A3 ; IL6ST and GUCY1B2 ; IL6ST and GUCY1B3 ; IL6ST and HAVCR2 ; IL6ST and HMOX2 ; IL6ST and IL10RA ; IL6ST and IL10RB ; IL6ST and IL6R ; LAG3 and BATF ; LAG3 and BTLA ; LAG3 and CASP10 ; LAG3 and CASP3 ; LAG3 and CASP6 ; LAG3 and CASP7 ; LAG3 and CASP8 ; LAG3 and CD160 ; LAG3 and CD244 ; LAG3 and CD96 ; LAG3 and CRTAM ; LAG3 and CSK; LAG3 and CTLA4 ; LAG3 and EIF2AK4 ; LAG3 and FADD ; LAG3 and FAS ; LAG3 and FOXP3 ; LAG3 and GUCY1A2 ; LAG3 and GUCY1A3 ; LAG3 and GUCY1B2 ; LAG3 and GUCY1B3 ; LAG3 and HAVCR2 ; LAG3 and HMOX2 ; LAG3 and IL10RA; LAG3 and IL10RB ; LAG3 and IL6R ; LAG3 and IL6ST; LAIR1 and BATF ; LAIR1 and BTLA; LAIR1 and CASP10 ; LAIR1 and CASP3 ; LAIR1 and CASP6 ; LAIR1 and CASP7 ; LAIR1 and CASP8 ; LAIR1 and CD160 ; LAIR1 and CD244 ; LAIR1 and CD96 ; LAIR1 and CRTAM ; LAIR1 and CSK ; LAIR1 and CTLA4 ; LAIR1 and EIF2AK4 ; LAIR1 and FADD ; LAIR1 and FAS ; LAIR1 and FOXP3 ; LAIR1 and GUCY1A2 ; LAIR1 and GUCY1A3 ; LAIR1 and GUCY1B2 ; LAIR1 and GUCY1B3 ; LAIR1 and HAVCR2 ; LAIR1 and HMOX2 ; LAIR1 and IL10RA ; LAIR1 and IL10RB ; LAIR1 and IL6R ; LAIR1 and IL6ST ; LAIR1 and LAG3 ; PAG1 and BATF ; PAG1 and BTLA ; PAG1 and CASP10 ; PAG1 and CASP3 ; PAG1 and CASP6 ; PAG1 and CASP7 ; PAG1 and CASP8 ; PAG1 and CD160 ; PAG1 and CD244 ; PAG1 and CD96 ; PAG1 and CRTAM ; PAG1 and CSK; PAG1 and CTLA4; PAG1 and EIF2AK4 ; PAG1 and FADD ; PAG1 and FAS ; PAG1 and FOXP3 ; PAG1 and GUCY1A2 ; PAG1 and GUCY1A3 ; PAG1 and GUCY1B2 ; PAG1 and GUCY1B3 ; PAG1 and HAVCR2 ; PAG1 and HMOX2 ; PAG1 and IL10RA; PAG1 and IL10RB ; PAG1 and IL6R ; PAG1 and IL6ST ; PAG1 and LAG3 ; PAG1 and LAIR1 ; PDCD1 and BATF ; PDCD1 and BTLA ; PDCD1 and CASP10 ; PDCD1 and CASP3 ; PDCD1 and CASP6 ; PDCD1 and CASP7 ; PDCD1 and CASP8 ; PDCD1 and CD160 ; PDCD1 and CD244 ; PDCD1 and CD96 ; PDCD1 and CRTAM ; PDCD1 and CSK ; PDCD1 and CTLA4 ; PDCD1 and EIF2AK4 ; PDCD1 and FADD ; PDCD1 and FAS ; PDCD1 and FOXP3 ; PDCD1 and GUCY1A2 ; PDCD1 and GUCY1A3 ; PDCD1 and GUCY1B2 ; PDCD1 and GUCY1B3 ; PDCD1 and HAVCR2 ; PDCD1 and HMOX2 ; PDCD1 and IL10RA ; PDCD1 and IL10RB ; PDCD1 and IL6R ; PDCD1 and IL6ST ; PDCD1 and LAG3 ; PDCD1 and LAIR1 ; PDCD1 and PAG1 ; PPP2CA and BATF ; PPP2CA and BTLA ; PPP2CA and CASP10 ; PPP2CA and CASP3 ; PPP2CA and CASP6 ; PPP2CA and CASP7 ; PPP2CA and CASP8 ; PPP2CA and CD160 ; PPP2CA and CD244 ; PPP2CA and CD96 ; PPP2CA and CRTAM ; PPP2CA and CSK ; PPP2CA and CTLA4 ; PPP2CA and EIF2AK4 ; PPP2CA and FADD ; PPP2CA and FAS ; PPP2CA and FOXP3 ; PPP2CA and GUCY1A2 ; PPP2CA and GUCY1A3 ; PPP2CA and GUCY1B2 ; PPP2CA and GUCY1B3 ; PPP2CA and HAVCR2 ; PPP2CA and HMOX2 ; PPP2CA and IL10RA ; PPP2CA and IL10RB ; PPP2CA and IL6R ; PPP2CA and IL6ST ; PPP2CA and LAG3 ; PPP2CA and LAIR1 ; PPP2CA and PAG1 ; PPP2CA and PDCD1 ; PPP2CB and BATF ; PPP2CB and BTLA ; PPP2CB and CASP10 ; PPP2CB and CASP3 ; PPP2CB and CASP6 ; PPP2CB and CASP7 ; PPP2CB and CASP8 ; PPP2CB and CD160 ; PPP2CB and CD244 ; PPP2CB and CD96 ; PPP2CB and CRTAM ; PPP2CB and CSK ; PPP2CB and CTLA4 ; PPP2CB and EIF2AK4 ; PPP2CB and FADD ; PPP2CB and FAS ; PPP2CB and FOXP3 ; PPP2CB and GUCY1A2 ; PPP2CB and GUCY1A3 ; PPP2CB and GUCY1B2 ; PPP2CB and GUCY1B3 ; PPP2CB and HAVCR2 ; PPP2CB and HMOX2 ; PPP2CB and IL10RA ; PPP2CB and IL10RB ; PPP2CB and IL6R ; PPP2CB and IL6ST ; PPP2CB and LAG3 ; PPP2CB and LAIR1 ; PPP2CB and PAG1 ; PPP2CB and PDCD1 ; PPP2CB and PPP2CA ; PRDM1 and BATF ; PRDM1 and BTLA ; PRDM1 and CASP10 ; PRDM1 and CASP3 ; PRDM1 and CASP6 ; PRDM1 and CASP7 ; PRDM1 and CASP8 ; PRDM1 and CD160 ; PRDM1 and CD244 ; PRDM1 and CD96 ; PRDM1 and CRTAM ; PRDM1 and CSK ; PRDM1 and CTLA4 ; PRDM1 and EIF2AK4 ; PRDM1 and FADD ; PRDM1 and FAS ; PRDM1 and FOXP3 ; PRDM1 and GUCY1A2 ; PRDM1 and GUCY1A3 ; PRDM1 and GUCY1B2 ; PRDM1 and GUCY1B3 ; PRDM1 and HAVCR2 ; PRDM1 and HMOX2 ; PRDM1 and IL10RA ; PRDM1 and IL10RB ; PRDM1 and IL6R ; PRDM1 and IL6ST ; PRDM1 and LAG3 ; PRDM1 and LAIR1 ; PRDM1 and PAG1 ; PRDM1 and PDCD1 ; PRDM1 and PPP2CA ; PRDM1 and PPP2CB ; PTPN22 and BATF ; PTPN22 and BTLA ; PTPN22 and CASP10 ; PTPN22 and CASP3 ; PTPN22 and CASP6 ; PTPN22 and CASP7 ; PTPN22 and CASP8 ; PTPN22 and CD160 ; PTPN22 and CD244 ; PTPN22 and CD96 ; PTPN22 and CRTAM ; PTPN22 and CSK ; PTPN22 and CTLA4 ; PTPN22 and EIF2AK4 ; PTPN22 and FADD ; PTPN22 and FAS ; PTPN22 and FOXP3 ; PTPN22 and GUCY1A2 ; PTPN22 and GUCY1A3 ; PTPN22 and GUCY1B2 ; PTPN22 and GUCY1B3 ; PTPN22 and HAVCR2 ; PTPN22 and HMOX2 ; PTPN22 and IL10RA ; PTPN22 and IL10RB ; PTPN22 and IL6R ; PTPN22 and IL6ST ; PTPN22 and LAG3 ; PTPN22 and LAIR1 ; PTPN22 and PAG1 ; PTPN22 and PDCD1 ; PTPN22 and PPP2CA ; PTPN22 and PPP2CB ; PTPN22 and PRDM1 ; PTPN6 and BATF ; PTPN6 and BTLA ; PTPN6 and CASP10 ; PTPN6 and CASP3 ; PTPN6 and CASP6 ; PTPN6 and CASP7 ; PTPN6 and CASP8 ; PTPN6 and CD160 ; PTPN6 and CD244 ; PTPN6 and CD96 ; PTPN6 and CRTAM ; PTPN6 and CSK; PTPN6 and CTLA4 ; PTPN6 and EIF2AK4 ; PTPN6 and FADD ; PTPN6 and FAS ; PTPN6 and FOXP3 ; PTPN6 and GUCY1A2 ; PTPN6 and GUCY1A3 ; PTPN6 and GUCY1B2 ; PTPN6 and GUCY1B3 ; PTPN6 and HAVCR2 ; PTPN6 and HMOX2 ; PTPN6 and IL10RA ; PTPN6 and IL10RB ; PTPN6 and IL6R; PTPN6 and IL6ST; PTPN6 and LAG3 ; PTPN6 and LAIR1; PTPN6 and PAG1; PTPN6 and PDCD1 ; PTPN6 and PPP2CA ; PTPN6 and PPP2CB ; PTPN6 and PRDM1 ; PTPN6 and PTPN22 ; SIGLEC7 and BATF ; SIGLEC7 and BTLA ; SIGLEC7 and CASP10 ; SIGLEC7 and CASP3 ; SIGLEC7 and CASP6 ; SIGLEC7 and CASP7 ; SIGLEC7 and CASP8 ; SIGLEC7 and CD160 ; SIGLEC7 and CD244 ; SIGLEC7 and CD96 ; SIGLEC7 and CRTAM ; SIGLEC7 and CSK; SIGLEC7 and CTLA4 ; SIGLEC7 and EIF2AK4 ; SIGLEC7 and FADD ; SIGLEC7 and FAS ; SIGLEC7 and FOXP3 ; SIGLEC7 and GUCY1A2 ; SIGLEC7 and GUCY1A3 ; SIGLEC7 and GUCY1B2 ; SIGLEC7 and GUCY1B3 ; SIGLEC7 and HAVCR2 ; SIGLEC7 and HMOX2 ; SIGLEC7 and IL10RA ; SIGLEC7 and IL10RB ; SIGLEC7 and IL6R ; SIGLEC7 and IL6ST ; SIGLEC7 and LAG3 ; SIGLEC7 and LAIR1 ; SIGLEC7 and PAG1 ; SIGLEC7 and PDCD1 ; SIGLEC7 and PPP2CA ; SIGLEC7 and PPP2CB ; SIGLEC7 and PRDM1 ; SIGLEC7 and PTPN22 ; SIGLEC7 and PTPN6 ; SIGLEC9 and BATF ; SIGLEC9 and BTLA ; SIGLEC9 and CASP10 ; SIGLEC9 and CASP3 ; SIGLEC9 and CASP6 ; SIGLEC9 and CASP7 ; SIGLEC9 and CASP8 ; SIGLEC9 and CD160 ; SIGLEC9 and CD244 ; SIGLEC9 and CD96 ; SIGLEC9 and CRTAM ; SIGLEC9 and CSK; SIGLEC9 and CTLA4 ; SIGLEC9 and EIF2AK4 ; SIGLEC9 and FADD ; SIGLEC9 and FAS ; SIGLEC9 and FOXP3 ; SIGLEC9 and GUCY1A2 ; SIGLEC9 and GUCY1A3 ; SIGLEC9 and GUCY1B2 ; SIGLEC9 and GUCY1B3 ; SIGLEC9 and HAVCR2 ; SIGLEC9 and HMOX2 ; SIGLEC9 and IL10RA ; SIGLEC9 and IL10RB ; SIGLEC9 and IL6R ; SIGLEC9 and IL6ST ; SIGLEC9 and LAG3 ; SIGLEC9 and LAIR1 ; SIGLEC9 and PAG1 ; SIGLEC9 and PDCD1 ; SIGLEC9 and PPP2CA ; SIGLEC9 and PPP2CB ; SIGLEC9 and PRDM1; SIGLEC9 and PTPN22 ; SIGLEC9 and PTPN6 ; SIGLEC9 and SIGLEC7 ; SIT1 and BATF ; SIT1 and BTLA ; SIT1 and CASP10 ; SIT1 and CASP3 ; SIT1 and CASP6 ; SIT1 and CASP7 ; SIT1 and CASP8 ; SIT1 and CD160 ; SIT1 and CD244 ; SIT1 and CD96 ; SIT1 and CRTAM ; SIT1 and CSK; SIT1 and CTLA4 ; SIT1 and EIF2AK4 ; SIT1 and FADD ; SIT1 and FAS ; SIT1 and FOXP3 ; SIT1 and GUCY1A2 ; SIT1 and GUCY1A3 ; SIT1 and GUCY1B2 ; SIT1 and GUCY1B3 ; SIT1 and HAVCR2 ; SIT1 and HMOX2 ; SIT1 and IL10RA ; SIT1 and IL10RB ; SIT1 and IL6R ; SIT1 and IL6ST; SIT1 and LAG3 ; SIT1 and LAIR1 ; SIT1 and PAG1 ; SIT1 and PDCD1 ; SIT1 and PPP2CA ; SIT1 and PPP2CB ; SIT1 and PRDM1 ; SIT1 and PTPN22 ; SIT1 and PTPN6 ; SIT1 and SIGLEC7 ; SIT1 and SIGLEC9 ; SKI and BATF ; SKI and BTLA ; SKI and CASP10 ; SKI and CASP3 ; SKI and CASP6 ; SKI and CASP7 ; SKI and CASP8 ; SKI and CD160 ; SKI and CD244 ; SKI and CD96 ; SKI and CRTAM ; SKI and CSK ; SKI and CTLA4 ; SKI and EIF2AK4 ; SKI and FADD ; SKI and FAS ; SKI and FOXP3 ; SKI and GUCY1A2 ; SKI and GUCY1A3 ; SKI and GUCY1B2 ; SKI and GUCY1B3 ; SKI and HAVCR2 ; SKI and HMOX2 ; SKI and IL10RA; SKI and IL10RB ; SKI and IL6R ; SKI and IL6ST; SKI and LAG3 ; SKI and LAIR1 ; SKI and PAG1 ; SKI and PDCD1 ; SKI and PPP2CA ; SKI and PPP2CB ; SKI and PRDM1 ; SKI and PTPN22 ; SKI and PTPN6 ; SKI and SIGLEC7 ; SKI and SIGLEC9 ; SKI and SIT1 ; SKIL and BATF ; SKIL and BTLA ; SKIL and CASP10 ; SKIL and CASP3 ; SKIL and CASP6 ; SKIL and CASP7 ; SKIL and CASP8 ; SKIL and CD160 ; SKIL and CD244 ; SKIL and CD96 ; SKIL and CRTAM ; SKIL and CSK ; SKIL and CTLA4 ; SKIL and EIF2AK4 ; SKIL and FADD ; SKIL and FAS ; SKIL and FOXP3 ; SKIL and GUCY1A2 ; SKIL and GUCY1A3 ; SKIL and GUCY1B2 ; SKIL and GUCY1B3 ; SKIL and HAVCR2 ; SKIL and HMOX2 ; SKIL and IL10RA ; SKIL and IL10RB ; SKIL and IL6R ; SKIL and IL6ST ; SKIL and LAG3 ; SKIL and LAIR1 ; SKIL and PAG1 ; SKIL and PDCD1 ; SKIL and PPP2CA ; SKIL and PPP2CB ; SKIL and PRDM1 ; SKIL and PTPN22 ; SKIL and PTPN6 ; SKIL and SIGLEC7 ; SKIL and SIGLEC9 ; SKIL and SIT1 ; SKIL and SKI ; SMAD10 and BATF ; SMAD10 and BTLA ; SMAD10 and CASP10 ; SMAD10 and CASP3 ; SMAD10 and CASP6 ; SMAD10 and CASP7 ; SMAD10 and CASP8 ; SMAD10 and CD160 ; SMAD10 and CD244 ; SMAD10 and CD96 ; SMAD10 and CRTAM ; SMAD10 and CSK ; SMAD10 and CTLA4 ; SMAD10 and EIF2AK4 ; SMAD10 and FADD ; SMAD10 and FAS ; SMAD10 and FOXP3 ; SMAD10 and GUCY1A2 ; SMAD10 and GUCY1A3 ; SMAD10 and GUCY1B2 ; SMAD10 and GUCY1B3 ; SMAD10 and HAVCR2 ; SMAD10 and HMOX2 ; SMAD10 and IL10RA ; SMAD10 and IL10RB ; SMAD10 and IL6R ; SMAD10 and IL6ST ; SMAD10 and LAG3 ; SMAD10 and LAIR1 ; SMAD10 and PAG1 ; SMAD10 and PDCD1 ; SMAD10 and PPP2CA; SMAD10 and PPP2CB ; SMAD10 and PRDM1 ; SMAD10 and PTPN22 ; SMAD10 and PTPN6 ; SMAD10 and SIGLEC7 ; SMAD10 and SIGLEC9 ; SMAD10 and SIT1 ; SMAD10 and SKI ; SMAD10 and SKIL ; SMAD2 and BATF ; SMAD2 and BTLA ; SMAD2 and CASP10 ; SMAD2 and CASP3 ; SMAD2 and CASP6 ; SMAD2 and CASP7 ; SMAD2 and CASP8 ; SMAD2 and CD160 ; SMAD2 and CD244 ; SMAD2 and CD96 ; SMAD2 and CRTAM ; SMAD2 and CSK ; SMAD2 and CTLA4 ; SMAD2 and EIF2AK4 ; SMAD2 and FADD ; SMAD2 and FAS ; SMAD2 and FOXP3 ; SMAD2 and GUCY1A2 ; SMAD2 and GUCY1A3 ; SMAD2 and GUCY1B2 ; SMAD2 and GUCY1B3 ; SMAD2 and HAVCR2 ; SMAD2 and HMOX2 ; SMAD2 and IL10RA ; SMAD2 and IL10RB ; SMAD2 and IL6R ; SMAD2 and IL6ST ; SMAD2 and LAG3 ; SMAD2 and LAIR1 ; SMAD2 and PAG1 ; SMAD2 and PDCD1 ; SMAD2 and PPP2CA ; SMAD2 and PPP2CB ; SMAD2 and PRDM1 ; SMAD2 and PTPN22 ; SMAD2 and PTPN6 ; SMAD2 and SIGLEC7 ; SMAD2 and SIGLEC9 ; SMAD2 and SIT1 ; SMAD2 and SKI ; SMAD2 and SKIL ; SMAD2 and SMAD10 ; SMAD3 and BATF ; SMAD3 and BTLA ; SMAD3 and CASP10 ; SMAD3 and CASP3 ; SMAD3 and CASP6 ; SMAD3 and CASP7 ; SMAD3 and CASP8 ; SMAD3 and CD160 ; SMAD3 and CD244 ; SMAD3 and CD96 ; SMAD3 and CRTAM ; SMAD3 and CSK ; SMAD3 and CTLA4 ; SMAD3 and EIF2AK4 ; SMAD3 and FADD ; SMAD3 and FAS ; SMAD3 and FOXP3 ; SMAD3 and GUCY1A2 ; SMAD3 and GUCY1A3 ; SMAD3 and GUCY1B2 ; SMAD3 and GUCY1B3 ; SMAD3 and HAVCR2 ; SMAD3 and HMOX2 ; SMAD3 and IL10RA ; SMAD3 and IL10RB ; SMAD3 and IL6R ; SMAD3 and IL6ST ; SMAD3 and LAG3 ; SMAD3 and LAIR1 ; SMAD3 and PAG1 ; SMAD3 and PDCD1 ; SMAD3 and PPP2CA ; SMAD3 and PPP2CB ; SMAD3 and PRDM1 ; SMAD3 and PTPN22 ; SMAD3 and PTPN6 ; SMAD3 and SIGLEC7 ; SMAD3 and SIGLEC9 ; SMAD3 and SIT1 ; SMAD3 and SKI ; SMAD3 and SKIL ; SMAD3 and SMAD10 ; SMAD3 and SMAD2 ; SMAD4 and BATF ; SMAD4 and BTLA ; SMAD4 and CASP10 ; SMAD4 and CASP3 ; SMAD4 and CASP6 ; SMAD4 and CASP7 ; SMAD4 and CASP8 ; SMAD4 and CD160 ; SMAD4 and CD244 ; SMAD4 and CD96 ; SMAD4 and CRTAM ; SMAD4 and CSK ; SMAD4 and CTLA4 ; SMAD4 and EIF2AK4 ; SMAD4 and FADD ; SMAD4 and FAS ; SMAD4 and FOXP3 ; SMAD4 and GUCY1A2 ; SMAD4 and GUCY1A3 ; SMAD4 and GUCY1B2 ; SMAD4 and GUCY1B3 ; SMAD4 and HAVCR2 ; SMAD4 and HMOX2 ; SMAD4 and IL10RA ; SMAD4 and IL10RB ; SMAD4 and IL6R ; SMAD4 and IL6ST ; SMAD4 and LAG3 ; SMAD4 and LAIR1 ; SMAD4 and PAG1 ; SMAD4 and PDCD1 ; SMAD4 and PPP2CA ; SMAD4 and PPP2CB ; SMAD4 and PRDM1 ; SMAD4 and PTPN22 ; SMAD4 and PTPN6 ; SMAD4 and SIGLEC7 ; SMAD4 and SIGLEC9 ; SMAD4 and SIT1 ; SMAD4 and SKI ; SMAD4 and SKIL ; SMAD4 and SMAD10 ; SMAD4 and SMAD2 ; SMAD4 and SMAD3 ; TGFBR1 and BATF ; TGFBR1 and BTLA ; TGFBR1 and CASP10 ; TGFBR1 and CASP3 ; TGFBR1 and CASP6 ; TGFBR1 and CASP7 ; TGFBR1 and CASP8 ; TGFBR1 and CD160 ; TGFBR1 and CD244 ; TGFBR1 and CD96 ; TGFBR1 and CRTAM ; TGFBR1 and CSK ; TGFBR1 and CTLA4 ; TGFBR1 and EIF2AK4 ; TGFBR1 and FADD ; TGFBR1 and FAS ; TGFBR1 and FOXP3 ; TGFBR1 and GUCY1A2 ; TGFBR1 and GUCY1A3 ; TGFBR1 and GUCY1B2 ; TGFBR1 and GUCY1B3 ; TGFBR1 and HAVCR2 ; TGFBR1 and HMOX2 ; TGFBR1 and IL10RA ; TGFBR1 and IL10RB ; TGFBR1 and IL6R ; TGFBR1 and IL6ST ; TGFBR1 and LAG3 ; TGFBR1 and LAIR1 ; TGFBR1 and PAG1 ; TGFBR1 and PDCD1 ; TGFBR1 and PPP2CA ; TGFBR1 and PPP2CB ; TGFBR1 and PRDM1 ; TGFBR1 and PTPN22 ; TGFBR1 and PTPN6 ; TGFBR1 and SIGLEC7 ; TGFBR1 and SIGLEC9 ; TGFBR1 and SIT1 ; TGFBR1 and SKI ; TGFBR1 and SKIL ; TGFBR1 and SMAD10 ; TGFBR1 and SMAD2 ; TGFBR1 and SMAD3 ; TGFBR1 and SMAD4 ; TGFBR2 and BATF ; TGFBR2 and BTLA ; TGFBR2 and CASP10 ; TGFBR2 and CASP3 ; TGFBR2 and CASP6 ; TGFBR2 and CASP7 ; TGFBR2 and CASP8 ; TGFBR2 and CD160 ; TGFBR2 and CD244 ; TGFBR2 and CD96 ; TGFBR2 and CRTAM ; TGFBR2 and CSK ; TGFBR2 and CTLA4 ; TGFBR2 and EIF2AK4 ; TGFBR2 and FADD ; TGFBR2 and FAS ; TGFBR2 and FOXP3 ; TGFBR2 and GUCY1A2 ; TGFBR2 and GUCY1A3 ; TGFBR2 and GUCY1B2 ; TGFBR2 and GUCY1B3 ; TGFBR2 and HAVCR2 ; TGFBR2 and HMOX2 ; TGFBR2 and IL10RA ; TGFBR2 and IL10RB ; TGFBR2 and IL6R ; TGFBR2 and IL6ST ; TGFBR2 and LAG3 ; TGFBR2 and LAIR1 ; TGFBR2 and PAG1 ; TGFBR2 and PDCD1 ; TGFBR2 and PPP2CA ; TGFBR2 and PPP2CB ; TGFBR2 and PRDM1 ; TGFBR2 and PTPN22 ; TGFBR2 and PTPN6 ; TGFBR2 and SIGLEC7 ; TGFBR2 and SIGLEC9 ; TGFBR2 and SIT1 ; TGFBR2 and SKI ; TGFBR2 and SKIL ; TGFBR2 and SMAD10 ; TGFBR2 and SMAD2 ; TGFBR2 and SMAD3 ; TGFBR2 and SMAD4 ; TGFBR2 and TGFBR1 ; TGIF1 and BATF ; TGIF1 and BTLA ; TGIF1 and CASP10 ; TGIF1 and CASP3 ; TGIF1 and CASP6 ; TGIF1 and CASP7 ; TGIF1 and CASP8 ; TGIF1 and CD160 ; TGIF1 and CD244 ; TGIF1 and CD96 ; TGIF1 and CRTAM ; TGIF1 and CSK; TGIF1 and CTLA4 ; TGIF1 and EIF2AK4 ; TGIF1 and FADD ; TGIF1 and FAS ; TGIF1 and FOXP3 ; TGIF1 and GUCY1A2 ; TGIF1 and GUCY1A3 ; TGIF1 and GUCY1B2 ; TGIF1 and GUCY1B3 ; TGIF1 and HAVCR2 ; TGIF1 and HMOX2 ; TGIF1 and IL10RA; TGIF1 and IL10RB ; TGIF1 and IL6R ; TGIF1 and IL6ST; TGIF1 and LAG3 ; TGIF1 and LAIR1 ; TGIF1 and PAG1; TGIF1 and PDCD1 ; TGIF1 and PPP2CA; TGIF1 and PPP2CB ; TGIF1 and PRDM1 ; TGIF1 and PTPN22 ; TGIF1 and PTPN6 ; TGIF1 and SIGLEC7 ; TGIF1 and SIGLEC9 ; TGIF1 and SIT1 ; TGIF1 and SKI ; TGIF1 and SKIL; TGIF1 and SMAD10 ; TGIF1 and SMAD2 ; TGIF1 and SMAD3 ; TGIF1 and SMAD4 ; TGIF1 and TGFBR1 ; TGIF1 and TGFBR2 ; TIGIT and BATF ; TIGIT and BTLA ; TIGIT and CASP10 ; TIGIT and CASP3 ; TIGIT and CASP6 ; TIGIT and CASP7 ; TIGIT and CASP8 ; TIGIT and CD160 ; TIGIT and CD244 ; TIGIT and CD96 ; TIGIT and CRTAM ; TIGIT and CSK ; TIGIT and CTLA4 ; TIGIT and EIF2AK4 ; TIGIT and FADD ; TIGIT and FAS ; TIGIT and FOXP3 ; TIGIT and GUCY1A2 ; TIGIT and GUCY1A3 ; TIGIT and GUCY1B2 ; TIGIT and GUCY1B3 ; TIGIT and HAVCR2 ; TIGIT and HMOX2 ; TIGIT and IL10RA ; TIGIT and IL10RB ; TIGIT and IL6R ; TIGIT and IL6ST ; TIGIT and LAG3 ; TIGIT and LAIR1 ; TIGIT and PAG1 ; TIGIT and PDCD1; TIGIT and PPP2CA; TIGIT and PPP2CB ; TIGIT and PRDM1 ; TIGIT and PTPN22 ; TIGIT and PTPN6 ; TIGIT and SIGLEC7 ; TIGIT and SIGLEC9 ; TIGIT and SIT1 ; TIGIT and SKI ; TIGIT and SKIL ; TIGIT and SMAD10 ; TIGIT and SMAD2 ; TIGIT and SMAD3 ; TIGIT and SMAD4 ; TIGIT and TGFBR1 ; TIGIT and TGFBR2 ; TIGIT and TGIF1 ; TNFRSF10A and BATF ; TNFRSF10A and BTLA ; TNFRSF10A and CASP10 ; TNFRSF10A and CASP3 ; TNFRSF10A and CASP6 ; TNFRSF10A and CASP7 ; TNFRSF10A and CASP8 ; TNFRSF10A and CD160 ; TNFRSF10A and CD244 ; TNFRSF10A and CD96 ; TNFRSF10A and CRTAM ; TNFRSF10A and CSK; TNFRSF10A and CTLA4; TNFRSF10A and EIF2AK4 ; TNFRSF10A and FADD ; TNFRSF10A and FAS ; TNFRSF10A and FOXP3 ; TNFRSF10A and GUCY1A2 ; TNFRSF10A and GUCY1A3 ; TNFRSF10A and GUCY1B2 ; TNFRSF10A and GUCY1B3 ; TNFRSF10A and HAVCR2 ; TNFRSF10A and HMOX2 ; TNFRSF10A and IL10RA ; TNFRSF10A and IL10RB ; TNFRSF10A and IL6R ; TNFRSF10A and IL6ST ; TNFRSF10A and LAG3 ; TNFRSF10A and LAIR1 ; TNFRSF10A and PAG1 ; TNFRSF10A and PDCD1 ; TNFRSF10A and PPP2CA ; TNFRSF10A and PPP2CB ; TNFRSF10A and PRDM1 ; TNFRSF10A and PTPN22 ; TNFRSF10A and PTPN6 ; TNFRSF10A and SIGLEC7 ; TNFRSF10A and SIGLEC9 ; TNFRSF10A and SIT1 ; TNFRSF10A and SKI ; TNFRSF10A and SKIL; TNFRSF10A and SMAD10 ; TNFRSF10A and SMAD2 ; TNFRSF10A and SMAD3 ; TNFRSF10A and SMAD4 ; TNFRSF10A and TGFBR1 ; TNFRSF10A and TGFBR2 ; TNFRSF10A and TGIF1 ; TNFRSF10A and TIGIT; TNFRSF10B and BATF ; TNFRSF10B and BTLA ; TNFRSF10B and CASP10 ; TNFRSF10B and CASP3 ; TNFRSF10B and CASP6 ; TNFRSF10B and CASP7 ; TNFRSF10B and CASP8 ; TNFRSF10B and CD160 ; TNFRSF10B and CD244 ; TNFRSF10B and CD96 ; TNFRSF10B and CRTAM ; TNFRSF10B and CSK ; TNFRSF10B and CTLA4 ; TNFRSF10B and EIF2AK4 ; TNFRSF10B and FADD ; TNFRSF10B and FAS ; TNFRSF10B and FOXP3 ; TNFRSF10B and GUCY1A2 ; TNFRSF10B and GUCY1A3 ; TNFRSF10B and GUCY1B2 ; TNFRSF10B and GUCY1B3 ; TNFRSF10B and HAVCR2 ; TNFRSF10B and HMOX2 ; TNFRSF10B and IL10RA ; TNFRSF10B and IL10RB ; TNFRSF10B and IL6R ; TNFRSF10B and IL6ST ; TNFRSF10B and LAG3 ; TNFRSF10B and LAIR1 ; TNFRSF10B and PAG1 ; TNFRSF10B and PDCD1 ; TNFRSF10B and PPP2CA ; TNFRSF10B and PPP2CB ; TNFRSF10B and PRDM1 ; TNFRSF10B and PTPN22 ; TNFRSF10B and PTPN6 ; TNFRSF10B and SIGLEC7 ; TNFRSF10B and SIGLEC9 ; TNFRSF10B and SIT1; TNFRSF10B and SKI ;TNFRSF10B and SKIL; TNFRSF10B and SMAD10 ; TNFRSF10B and SMAD2 ; TNFRSF10B and SMAD3 ; TNFRSF10B and SMAD4 ; TNFRSF10B and TGFBR1 ; TNFRSF10B and TGFBR2 ; TNFRSF10B and TGIF1 ; TNFRSF10B and TIGIT ; TNFRSF10B and TNFRSF10A.

By inactivating a gene it is intended that the gene of interest is not expressed in a functional protein form. The gene can be altered or deleted. For example, said protein can be mutated and remains active as a dominant negative form. In a particular embodiment, the genetic modification of the method relies on the expression, in provided cells to engineer, of one rare-cutting endonuclease such that said rare-cutting endonuclease specifically catalyzes cleavage in one targeted gene thereby inactivating said targeted gene. The nucleic acid strand breaks caused by the rare-cutting endonuclease are commonly repaired through the distinct mechanisms of homologous recombination or non-homologous end joining (NHEJ). However, NHEJ is an imperfect repair process that often results in changes to the DNA sequence at the site of the cleavage. Mechanisms involve rejoining of what remains of the two DNA ends through direct re-ligation (Critchlow and Jackson 1998) or via the so-called microhomology-mediated end joining (Ma, Kim et al. 2003). Repair via non-homologous end joining (NHEJ) often results in small insertions or deletions and can be used for the creation of specific gene knockouts. Said modification may be a substitution, deletion, or addition of at least one nucleotide. Cells in which a cleavage-induced mutagenesis event, i.e a mutagenesis event consecutive to an NHEJ event, has occurred can be identified and/or selected by well-known method in the art.

For example, said method to engineer cells comprises at least one of the following steps:
(a) providing a T-cell, preferably from a cell culture or from a blood sample;
(b) introducing into said T-cell rare-cutting endonucleases able to selectively inactivate by DNA cleavage, preferably by double-strand break at least two genes encoding immune checkpoint proteins, and
(c) expanding said cells.

Said genes encoding immune checkpoint proteins can be selected from the list as described above.

Said rare-cutting endonuclease can be a meganuclease, a Zinc finger nuclease, CRISPR/Cas9 or a TALE-nuclease. Said rare-cutting endonuclease preferably is a TALE-nuclease. By TALE-nuclease is intended a fusion protein consisting of a DNA-binding domain derived from a Transcription Activator Like Effector (TALE) and one nuclease catalytic domain to cleave a nucleic acid target sequence (Boch, Scholze et al. 2009; Moscou and Bogdanove 2009; Christian, Cermak et al. 2010; Cermak, Doyle et al. 2011; Geissler, Scholze et al. 2011; Huang, Xiao et al. 2011; Li, Huang et al. 2011; Mahfouz, Li et al. 2011; Miller, Tan et al. 2011; Morbitzer, Romer et al. 2011; Mussolino, Morbitzer et al. 2011; Sander, Cade et al. 2011; Tesson, Usal et al. 2011; Weber, Gruetzner et al. 2011; Zhang, Cong et al. 2011; Deng, Yan et al. 2012; Li, Piatek et al. 2012; Mahfouz, Li et al. 2012; Mak, Bradley et al. 2012).

In the present invention new TALE-nucleases have been designed for precisely targeting relevant genes for adoptive immunotherapy strategies. Preferred TALE-nucleases according to the disclosure are those recognizing and cleaving the target sequence selected from the group consisting of: SEQ ID NO: 77 and SEQ ID NO: 78 (PD1), SEQ ID NO: 74 to SEQ ID NO: 76 (CTLA-4). The present disclosure also relates to TALE-nuclease polypeptides which comprise an amino acid sequence selected from the group consisting of SEQ ID NO: 79 to SEQ ID NO: 88.

Additional catalytic domain can be further introduced into the cell with said rare-cutting endonuclease to increase mutagenesis in order to enhance their capacity to inactivate targeted genes. In particular, said additional catalytic domain is a DNA end processing enzyme. Non limiting examples of DNA end-processing enzymes include 5-3' exonucleases, 3-5' exonucleases, 5-3' alkaline exonucleases, 5' flap endonucleases, helicases, hosphatase, hydrolases and template-independent DNA polymerases. Non limiting examples of such catalytic domain comprise of a protein domain or catalytically active derivate of the protein domain seleced from the group consisting of hExol (EXO1_HUMAN), Yeast Exol (EXO1_YEAST), E.coli Exol, Human TREX2, Mouse TREX1, Human TREX1, Bovine TREX1, Rat TREX1, TdT (terminal deoxynucleotidyl transferase) Human DNA2, Yeast DNA2 (DNA2_YEAST). For example, said additional catalytic domain has a 3'-5'-exonuclease activity, and can be a TREX, more preferably TREX2 catalytic domain (WO2012/058458). Said catalytic domain can be encoded by a single chain TREX polypeptide (WO2013/009525). Said additional catalytic domain may be fused to a nuclease fusion protein or chimeric protein according to the disclosure optionally by a peptide linker.

Endonucleolytic breaks are known to stimulate the rate of homologous recombination. Thus, as also described herein, the genetic modification step of the method can further comprise a step of introducing into cells an exogeneous nucleic acid comprising at least a sequence homologous to a portion of the target nucleic acid sequence, such that homologous recombination occurs between the target nucleic acid sequence and the exogeneous nucleic acid. In particular, said exogenous nucleic acid can comprise first and second portions which are homologous to region 5' and 3' of the target nucleic acid sequence, respectively. Said exogenous nucleic acid then also comprises a third portion positioned between the first and the second portion which comprises no homology with the regions 5' and 3' of the target nucleic acid sequence. Following cleavage of the target nucleic acid sequence, a homologous recombination event is stimulated between the target nucleic acid sequence and the exogenous nucleic acid. Preferably, homologous sequences of at least 50 bp, preferably more than 100 bp and more preferably more than 200 bp are used within said donor matrix. In particular, the homologous sequence is preferably from 200 bp to 6000 bp, more preferably from 1000 bp to 2000 bp. Indeed, shared nucleic acid homologies are located in regions flanking upstream and downstream the site of the break and the nucleic acid sequence to be introduced should be located between the two arms.

In particular, said exogenous nucleic acid successively comprises a first region of homology to sequences upstream of said cleavage, a sequence to inactivate one targeted gene selected from the group consisting of immune checkpoint genes and a second region of homology to sequences downstream of the cleavage. Said polynucleotide introduction step can be simultaneous, before or after the introduction or expression of said rare-cutting endonuclease. Depending on the location of the target nucleic acid sequence wherein break event has occurred, such exogenous nucleic acid can be used to knock-out a gene, e.g. when exogenous nucleic acid is located within the open reading frame of said gene, or to introduce new sequences or genes of interest. Sequence insertions by using such exogenous nucleic acid can be used to modify a targeted existing gene, by correction or replacement of said gene (allele swap as a non-limiting example), or to up- or downregulate the expression of the targeted gene (promoter swap as non-limiting). As mentioned above, said additional genomic modification step can be an inactivation step comprising:
(a) introducing into said cells at least one rare-cutting endonuclease such that said rare-cutting endonuclease specifically catalyzes cleavage in one targeted sequence of the genome of said cell.
(b) Optionally introducing into said cells an exogenous nucleic acid successively comprising a first region of homology to sequences upstream of said cleavage, a sequence to be inserted in the genome of said cell and a second region of homology to sequences downstream of said cleavage,
wherein said introduced exogenous nucleic acid inactivates a gene and integrates at least one exogenous polynucleotide sequence encoding at least one recombinant protein of interest. Said exogenous polynucleotide sequence can also be integrated within a gene selected from the group consisting of immune checkpoint genes.

Said method to engineer cell further comprises an additional genomic modification step. By additional genomic modification step, is intended the introduction into cells to engineer of a Chimeric Antigen Receptor (CAR), e.g. a multi-chain CAR. Introduction of a bispecific antibody is also described in the present disclosure. Said method to engineer cell further comprises the introduction of rare-cutting endonuclease able to selectively inactivate by DNA cleavage a TCR gene as described in the present disclosure.

The disclosure also relates to TALE-nucleases. Generally, the disclosure relates to TALE-nuclease comprising:
(a) A Transcription Activator-Like Effector (TALE) DNA binding domain that has been engineered to bind a target sequence within genes selected from the group consisting of immune checkpoint genes;
(b) A cleavage domain or a cleavage half-domain.

Preferred TALE-nucleases according to the disclosure are those recognizing and cleaving the target sequence selected from the group consisting of: SEQ ID NO: 77 and SEQ ID NO: 78 (PD1) and SEQ ID NO: 74 to SEQ ID NO: 76 (CTLA-4).

Said TALE-nucleases preferably comprise a polypeptide sequence selected from the group consisting of SEQ ID NO: 79 to SEQ ID NO: 88 in order to cleave the respective target SEQ ID NO: 74 to 78.

Because some variability may arise from the genomic data from which these polypeptides derive, and also to take into account the possibility to substitute some of the amino acids present in these polypeptides without significant loss of activity (functional variants), the disclosure encompasses polypeptides variants of the above polypeptides that share at least 70%, preferably at least 80 %, more preferably at least 90 % and even more preferably at least 95 % identity with the sequences provided in this patent application.

The present disclosure is thus drawn to polypeptides comprising a polypeptide sequence that has at least 70%, preferably at least 80%, more preferably at least 90 %, 95 % 97 % or 99 % sequence identity with amino acid sequence selected from the group consisting of SEQ ID NO: 79 to SEQ ID NO: 88.

Are also comprised in the scope of the present disclosure, polynucleotides, vectors encoding the above described rare-cutting endonucleases according to the disclosure.

In the scope of the present disclosure are also encompassed isolated cells or cell lines susceptible to be obtained by said method to engineer cells, in particular T cells, in which at least one gene selected from the group consisting of immune checkpoint genes, preferably genes selected from the group of: CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3, more preferably, two of the above genes as listed previously have been inactivated. Those genes are preferably inactivated by at least one rare-cutting endonuclease. It has been shown by the inventors that the use of TALE-nucleases was particularly advantageous to achieve double inactivation in T-cells. The disclosure encompasses also an isolated T-cell comprising at least two polynucleotides, said polynucleotides encoding at least a first and second TALE-nucleases, preferably the TALE-nucleases being directed against at least two genes encoding immune checkpoint proteins, such as CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3. In another aspect of the disclosure, said isolated cell further comprises one additional genomic modification, such as the integration of at least one exogenous polynucleotide sequence.

### Non alloreactive and highly active T cells for immunotherapy

The current protocol for treatment of patients using adoptive immunotherapy is based on autologous cell transfer. Autologous therapies face substantial technical and logistic hurdles to practical application, their generation requires expensive dedicated facilities and expert personnel, they must be generated in a short time following a patient's diagnosis, and in many cases, pretreatment of the patient has resulted in degraded immune function, such that the patient's lymphocytes may be poorly functional and present in very low numbers. Ideally, one would like to use a standardized therapy in which allogeneic therapeutic cells could be pre-manufactured, characterized in detail, and available for immediate administration to patients. However, endogenous TCR specificities of allogeneic cells which recognize the host tissue as foreign, result in graft versus host disease (GvHD), which can lead to serious tissue damage and death. Thus, in order to effectively use allogeneic cells, the inactivation of TCRalpha or TCRbeta can result in the elimination of the TCR from the surface of T cells preventing recognition of alloantigen and thus GVHD.

Thus, the present invention relates to a method of engineering allogeneic T-cells, especially for immunotherapy.

In a particular embodiment, the method comprises:
(a) providing a T cell,
(b) modifying T-cells by inactivating at least:
   - a first gene encoding an immune checkpoint protein, and
   - a second gene encoding a component of the T-cell receptor (TCR)
(c) expanding said cells.

The genetic modification step of the method relies on the inactivation of one gene encoding the immune checkpoint protein PD1 and one gene selected from the group consisting of TCR alpha and TCR beta. In another embodiment, the genetic modification step of the method relies on the inactivation of two genes selected from the group consisting of [PDCD1 and TCR alpha] and [PDCD1 and TCR beta]. In another aspect of the present disclosure, the genetic modification step of the method relies on the inactivation of two genes selected from the group consisting of CTLA4 and TCR alpha, CTLA4 and TCR beta, PPP2CA and TCR alpha, PPP2CB and TCR beta, PTPN6 and TCR alpha, PTPN6 and TCR beta, PTPN22 and TCR alpha, PTPN22 and TCR beta, LAG3 and TCR alpha, LAG3 and TCR beta, HAVCR2 and TCR alpha, HAVCR2 and TCR beta, BTLA and TCR alpha, BTLA and TCR beta, CD160 and TCR alpha, CD160 and TCR beta, TIGIT and TCR alpha, TIGIT and TCR beta, CD96 and TCR alpha, CD96 and TCR beta, CRTAM and TCR alpha, CRTAM and TCR beta, LAIR1 and TCR alpha, LAIR1 and TCR beta, SIGLEC7 and TCR alpha, SIGLEC7 and TCR beta, SIGLEC9 and TCR alpha, SIGLEC9 and TCR beta, CD244 and TCR alpha, CD244 and TCR beta,TNFRSF10B and TCR alpha, TNFRSF10B and TCR beta, TNFRSF10A and TCR alpha, TNFRSF10A and TCR beta, CASP8 and TCR alpha, CASP8 and TCR beta, CASP10 and TCR alpha, CASP10 and TCR beta, CASP3 and TCR alpha, CASP3 and TCR beta, CASP6 and TCR alpha, CASP6 and TCR beta, CASP7 and TCR alpha, CASP7 and TCR beta, FADD and TCR alpha, FADD and TCR beta, FAS and TCR alpha, FAS and TCR beta, TGFBRII and TCR alpha, TGFBRII and TCR beta, TGFRBRI and TCR alpha, TGFRBRI and TCR beta, SMAD2 and TCR alpha, SMAD2 and TCR beta, SMAD3 and TCR alpha, SMAD3 and TCR beta, SMAD4 and TCR alpha, SMAD4 and TCR beta, SMAD10 and TCR alpha, SMAD10 and TCR beta, SKI and TCR alpha, SKIL and TCR beta, TGIF1 and TCR alpha, TGIF1 and TCR beta, IL10RA and TCR alpha, IL10RA and TCR beta, IL10RB and TCR alpha, IL10RB and TCR beta, HMOX2 and TCR alpha, HMOX2 and TCR beta,IL6R and TCR alpha, IL6R and TCR beta, IL6ST and TCR alpha, IL6ST and TCR beta, EIF2AK4 and TCR alpha, EIF2AK4 and TCR beta, CSK and TCR alpha, CSK and TCR beta, PAG1 and TCR alpha, PAG1 and TCR beta, SIT1 and TCR alpha, SIT1 and TCR beta, FOXP3 and TCR alpha, FOXP3 and TCR beta, PRDM1 and TCR alpha, PRDM1 and TCR beta, BATF and TCR alpha, BATF and TCR beta, GUCY1A2 and TCR alpha, GUCY1A2 and TCR beta , GUCY1A3 and TCR alpha, GUCY1A3 and TCR beta, GUCY1B2 and TCR alpha, GUCY1B2 and TCR beta, GUCY1B3 and TCR alpha, GUCY1B3 and TCR beta. In another aspect of the present disclosure, the genetic modification step of the method relies on the inactivation of more than two genes. The genetic modification is preferably operated ex-vivo.

According to the invention, said method to engineer T cells comprises at least one of the following steps:
(a) providing a T-cell, preferably from a primary cell culture or from a blood sample;
(b) introducing into said T-cell a rare-cutting endonuclease able to selectively inactivate by DNA cleavage, preferably by double-strand break respectively:
   - said gene encoding a immune checkpoint protein, and
   - at least one gene encoding a component of the T-cell receptor (TCR).
(c) expanding said cells.

In a preferred embodiment, said method comprises:
(a) providing a T-cell, preferably from a cell culture or from a blood sample;
(b) transforming said T cell with nucleic acid encoding a rare-cutting endonuclease able to selectively inactivate by DNA cleavage, preferably by double-strand break respectively:
   - said gene encoding a immune checkpoint protein and
   - at least one gene encoding a component of the T-cell receptor (TCR)
(c) expressing said rare-cutting endonucleases into said T-cells;
(d) sorting the transformed T-cells, which do not express TCR on their cell surface;
(e) expanding said cells.

In particular aspect of the disclosure, said rare-cutting endonuclease specifically targets at least one gene, preferably two genes selected from the group consisting of: CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, GUCY1B3, TCR alpha and TCR beta. In another aspect of the disclosure, more than two rare-cutting endonucleases can be expressed in cells to engineer in order to target and/or inactivate more than two genes.

Said rare-cutting endonuclease can be a meganuclease, a Zinc finger nuclease or a TALE-nuclease. In a preferred embodiment, said rare-cutting endonuclease is a TALE-nuclease.

In the present disclosure, new TALE-nucleases have been designed for precisely targeting relevant genes for adoptive immunotherapy strategies. Preferred TALE-nucleases according to the disclosure are those recognizing and cleaving the target sequence selected from the group consisting of: SEQ ID NO: 77 and SEQ ID NO: 78 (PD1), SEQ ID NO: 74 to SEQ ID NO: 76 (CTLA-4), SEQ ID NO: 37, 57 to 60 (TCRalpha), SEQ ID NO: 38 or 39 (TCRbeta). The present disclosure also relates to TALE-nuclease polypeptides which comprise an amino acid sequence selected from the group consisting of SEQ ID NO: 79 to SEQ ID NO: 88 and SEQ ID NO: 41 to 46.

The present disclosure also relates to polypeptides comprising an amino acid sequence that has at least 70%, preferably at least 80%, more preferably at least 90 %, 95 % 97 % or 99 % sequence identity with amino acid sequence selected from the group consisting of SEQ ID NO: 79 to SEQ ID NO: 88. Are also comprised in the scope of the present disclosure, polynucleotides, vectors encoding the above described rare-cutting endonucleases according to the disclosure. This method can be associated with any one of the different methods described in the present disclosure.

In preferred embodiment, inactivation of genes from the group consisting of genes encoding PD1, TCR alpha and TCR beta is done at a precise genomic location targeted by a specific TALE-nuclease, wherein said specific TALE-nuclease catalyzes a cleavage and wherein an exogenous nucleic acid successively comprising at least a region of homology and a sequence to inactivate one targeted gene selected from the group consisting of genes encoding PD1, TCR alpha and TCR beta which is integrated by homologous recombination. In another embodiment, several genes can be, successively or at the same time, inactivated by using several TALE-nucleases respectively and specifically targeting one defined gene and several specific polynucleotides for specific gene inactivation.

Also described herein is the inactivation of another gene selected from the group consisting of immune checkpoint genes, TCR alpha and TCR beta. As mentioned above, said additional genomic modification step can be an inactivation step comprising:
(a) introducing into said cells at least one rare-cutting endonuclease such that said rare-cutting endonuclease specifically catalyzes cleavage in one targeted sequence of the genome of said cell.
(b) Optionally introducing into said cells a exogenous nucleic acid successively comprising a first region of homology to sequences upstream of said cleavage, a sequence to be inserted in the genome of said cell and a second region of homology to sequences downstream of said cleavage,
wherein said introduced exogenous nucleic acid inactivates a gene and integrates at least one exogenous polynucleotide sequence encoding at least one recombinant protein of interest. In another aspect of the present disclosure, said exogenous polynucleotide sequence is integrated within a gene selected from the group consisting of immune checkpoint genes, TCR alpha and TCR beta.

Said method to engineer cell further comprises an additional genomic modification step, i.e., the introduction into cells to engineer of a Chimeric Antigen Receptor (CAR), e.g. a multi-chain CAR. Introduction of a pTalpha or functional variant thereof or a bispecific antibody into the engineered cells is also described herein, as well as the introduction of rare-cutting endonuclease able to selectively inactivate by DNA cleavage a gene encoding a target for said immunosuppressive agent.

Are also comprised in the scope of the present disclosure, polynucleotides, vectors encoding the above described rare-cutting endonucleases according to the invention.

In the scope of the present disclosure are also encompassed isolated cells or cell lines susceptible to be obtained by said method to engineer cells, in particular T cells, in which at least two genes selected from the group consisting of: CTLA4 and TCR alpha, CTLA4 and TCR beta, PPP2CA and TCR alpha, PPP2CB and TCR beta, PTPN6 and TCR alpha, PTPN6 and TCR beta, PTPN22 and TCR alpha, PTPN22 and TCR beta, PDCD1 and TCR alpha, PDCD1 and TCR beta, LAG3 and TCR alpha, LAG3 and TCR beta, HAVCR2 and TCR alpha, HAVCR2 and TCR beta, BTLA and TCR alpha, BTLA and TCR beta, CD160 and TCR alpha, CD160 and TCR beta, TIGIT and TCR alpha, TIGIT and TCR beta, CD96 and TCR alpha, CD96 and TCR beta, CRTAM and TCR alpha, CRTAM and TCR beta, LAIR1 and TCR alpha, LAIR1 and TCR beta, SIGLEC7 and TCR alpha, SIGLEC7 and TCR beta, SIGLEC9 and TCR alpha, SIGLEC9 and TCR beta, CD244 and TCR alpha, CD244 and TCR beta,TNFRSF10B and TCR alpha, TNFRSF10B and TCR beta, TNFRSF10A and TCR alpha, TNFRSF10A and TCR beta, CASP8 and TCR alpha, CASP8 and TCR beta, CASP10 and TCR alpha, CASP10 and TCR beta, CASP3 and TCR alpha, CASP3 and TCR beta, CASP6 and TCR alpha, CASP6 and TCR beta, CASP7 and TCR alpha, CASP7 and TCR beta, FADD and TCR alpha, FADD and TCR beta, FAS and TCR alpha, FAS and TCR beta, TGFBRII and TCR alpha, TGFBRII and TCR beta, TGFRBRI and TCR alpha, TGFRBRI and TCR beta, SMAD2 and TCR alpha, SMAD2 and TCR beta, SMAD3 and TCR alpha, SMAD3 and TCR beta, SMAD4 and TCR alpha, SMAD4 and TCR beta, SMAD10 and TCR alpha, SMAD10 and TCR beta, SKI and TCR alpha, SKIL and TCR beta, TGIF1 and TCR alpha, TGIF1 and TCR beta, IL10RA and TCR alpha, IL10RA and TCR beta, IL10RB and TCR alpha, IL10RB and TCR beta, HMOX2 and TCR alpha, HMOX2 and TCR beta,IL6R and TCR alpha, IL6R and TCR beta, IL6ST and TCR alpha, IL6ST and TCR beta, EIF2AK4 and TCR alpha, EIF2AK4 and TCR beta, CSK and TCR alpha, CSK and TCR beta, PAG1 and TCR alpha, PAG1 and TCR beta, SIT1 and TCR alpha, SIT1 and TCR beta, FOXP3 and TCR alpha, FOXP3 and TCR beta, PRDM1 and TCR alpha, PRDM1 and TCR beta, BATF and TCR alpha, BATF and TCR beta, GUCY1A2 and TCR alpha, GUCY1A2 and TCR beta , GUCY1A3 and TCR alpha, GUCY1A3 and TCR beta, GUCY1B2 and TCR alpha, GUCY1B2 and TCR beta, GUCY1B3 and TCR alpha, GUCY1B3 and TCR beta, have been inactivated.

According to the disclosure, those genes are preferably inactivated by at least one rare-cutting endonuclease. It has been shown by the inventors that the use of TALE-nucleases was particularly advantageous to achieve double inactivation in T-cells. The disclosure encompasses an isolated T-cell comprising at least two polynucleotides, said polynucleotides encoding at least a first and second TALE-nucleases, preferably the first TALE-nuclease being directed against a gene encoding TCR and the second being directed against a gene encoding a immune checkpoint protein, such as CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3. In another aspect of the disclosure, said isolated cell further comprises one additional genomic modification. In another aspect of the disclosure, said additional genomic modification is the integration of at least one exogenous polynucleotide sequence.

### Immunosuppressive resistant T cells

Allogeneic cells are rapidly rejected by the host immune system. It has been demonstrated that, allogeneic leukocytes present in non-irradiated blood products will persist for no more than 5 to 6 days (Boni, Muranski et al. 2008). Thus, to prevent rejection of allogeneic cells, the host's immune system has to be usually suppressed to some extent. However, in the case of adoptive immunotherapy the use of immunosuppressive drugs also have a detrimental effect on the introduced therapeutic T cells. Therefore, to effectively use an adoptive immunotherapy approach in these conditions, the introduced cells would need to be also resistant to the immunosuppressive treatment. Thus, in particular aspect of the disclosure, the method according to the present disclosure further comprises a step of modifying T-cells to make them resistant immunosuppressive agent, preferably by inactivating at least one gene encoding a target for an immunosuppressive agent. An immunosuppressive agent is an agent that suppresses immune function by one of several mechanisms of action. In other words, an immunosuppressive agent is a role played by a compound which is exhibited by a capability to diminish the extent of an immune response. As non limiting example, an immunosuppressive agent can be a calcineurin inhibitor, a target of rapamycin, an interleukin-2 α-chain blocker, an inhibitor of inosine monophosphate dehydrogenase, an inhibitor of dihydrofolic acid reductase, a corticosteroid or an immunosuppressive antimetabolite. Classical cytotoxic immunosuppressants act by inhibiting DNA synthesis. Others may act through activation of T-cells or by inhibiting the activation of helper cells. The method according to the disclosure allows conferring immunosuppressive resistance to T cells for immunotherapy by inactivating the target of the immunosuppressive agent in T cells. As non limiting examples, targets for immunosuppressive agent can be a receptor for an immunosuppressive agent such as: CD52, glucocorticoid receptor (GR), a FKBP family gene member and a cyclophilin family gene member.

In particular aspect of the disclosure, the genetic modification of the method relies on the expression, in provided cells to engineer, of one rare-cutting endonuclease such that said rare-cutting endonuclease specifically catalyzes cleavage in one targeted gene thereby inactivating said targeted gene. Said rare-cutting endonuclease can be a meganuclease, a Zinc finger nuclease or a TALE-nuclease. Preferred TALE-nucleases according to the invention are those recognizing and cleaving the target sequence selected from the group consisting of: SEQ ID NO: 1 to 6 (GR), and SEQ ID NO: 40, 61 to 65 (CD52). Said TALE-nucleases preferably comprise a polypeptide sequence selected from SEQ ID NO: 7 to SEQ ID NO: 18 and SEQ ID NO: 47 and 48.

Because some variability may arise from the genomic data from which these polypeptides derive, and also to take into account the possibility to substitute some of the amino acids present in these polypeptides without significant loss of activity (functional variants), the disclosure encompasses polypeptides variants of the above polypeptides that share at least 70%, preferably at least 80 %, more preferably at least 90 % and even more preferably at least 95 % identity with the sequences provided in this patent application.

The present aspect of the disclosure is thus drawn to polypeptides comprising a polypeptide sequence that has at least 70%, preferably at least 80%, more preferably at least 90 %, 95 % 97 % or 99 % sequence identity with amino acid sequence selected from the group consisting of SEQ ID NO: 7 to SEQ ID NO: 18 and SEQ ID NO: 47 and 48.

Are also comprised in the scope of the present aspect of the disclosure, polynucleotides, vectors encoding the above described rare-cutting endonucleases according to the invention.

### PreTalpha

Because of the nature of selection of TCRbeta chains through pairing with preTalpha during thymic development, in T cells in which TCRalpha has been inactivated, the heterologous introduction of the pTalpha transgene can result in the formation of a preTCR. This pTCR can serve as a means of T cell activation or stimulation in a manner that is non-MHC dependent, thus for example allowing continued expansion of alpha/beta Tcells following TCRalpha inactivation. Importantly, the pTCR complex displays a similar biochemical composition as the TCR in terms of associated CD3 subunits (Carrasco, Ramiro et al. 2001). In addition, in contrast to the TCR, pre-TCR signaling may occur in part by a ligand independent event. The crystal structure of the pTCR extracellular domain has provided a structural basis for the possible ligand-independence of pTCR signaling. The pTCR has been shown to form a head to tail dimer where two pTalpha-TCRbeta heterodimers associate (Pang, Berry et al. 2010).

In another aspect, the method according to the present disclosure further comprises introducing into said T-cell pTalpha (also named preTCRα) or a functional variant thereof and expanding said cells, optionally through stimulation of the CD3 complex. In a preferred embodiment, the method comprises:
a) Transforming said cells with nucleic acid encoding at least a fragment of pTalpha to support CD3 surface expression
b) Expressing said pTalpha into said cells
c) Expanding said cells optionally, optionally through stimulation of the CD3 complex.

The disclosure also relates to a method of preparing T-cells for immunotherapy comprising steps of the method for expansion for T-cell.

In particular aspect of the disclosure, the pTalpha polynucleotide sequence can be introduced randomly or else through homologous recombination, in particular the insertion could be associated with the inactivation of the TCRalpha gene.

According to the disclosure, different functional variants of pTalpha are used. A "functional variant" of the peptide refers to a molecule substantially similar to either the entire peptide or a fragment thereof. A "fragment" of the pTalpha or functional variant thereof of the present disclosure, refers to any subset of the molecule, that is, a shorter peptide. Preferred pTalpha or functional variants can be full length pTalpha or a C-terminal truncated pTalpha version. C-terminal truncated pTalpha lacks in C-terminal end one or more residues. As non limiting examples, C-terminal truncated pTalpha version lacks 18 , 48, 62, 78, 92, 110 or 114 residues from the C-terminus of the protein (SEQ ID NO: 107 to SEQ ID NO: 114). Moreover, amino acid sequence variants of the peptide can be prepared by mutations in the DNA which encodes the peptide. Such functional variants include, for example, deletions from, or insertions or substitutions of, residues within the amino acid sequence. Any combination of deletion, insertion, and substitution may also be made to arrive at the final construct, provided that the final construct possesses the desired activity, in particular the restoration of a functional CD3 complex. In preferred aspect of the disclosure, at least one mutation is introduced in the different pTalpha versions as described above to affect dimerization. As non limiting example, mutated residue can be at least W46R, D22A, K24A, R102A or R117A of the human pTalpha protein or aligned positions using CLUSTALW method on pTalpha family or homologue member. Preferably pTalpha or variant thereof as described above comprise the mutated residue W46R (SEQ ID NO:123) or the mutated residues D22A, K24A, R102A and R117A (SEQ ID NO: 124 ). In particular aspect of the disclosure, said pTalpha or variants are also fused to a signal-transducing domain such as CD28, OX40, ICOS, CD27, CD137 (4-1BB) and CD8 as non limiting examples (SEQ ID NO: 115 to SEQ ID NO: 120). The extracellular domain of pTalpha or variants as described above can be fused to a fragment of the TCRalpha protein, particularly the transmembrane and intracellular domain of TCRalpha (SEQ ID NO: 122). pTalpha variants can also be fused to the intracellular domain of TCRalpha (SEQ ID NO:121).

In another aspect of the disclosure, said pTalpha versions are fused to an extracellular ligand-binding domain and more preferably pTalpha or functional variant thereof is fused to a single chain antibody fragment (scFV) comprising the light (*V_{L}*) and the heavy (*V_{H}*) variable fragment of a target antigen specific monoclonal antibody joined by a flexible linker. As a non limiting example, amino acid sequence of pTalpha or functional variant thereof is selected from the group consisting of SEQ ID NO: 107 to SEQ ID NO: 124.

Because some variability may arise from the genomic data from which these polypeptides derive, and also to take into account the possibility to substitute some of the amino acids present in these polypeptides without significant loss of activity (functional variants), the disclosure encompasses polypeptides variants of the above polypeptides that share at least 70%, preferably at least 80 %, more preferably at least 90 % and even more preferably at least 95 % identity with the sequences provided in this patent application.

The present disclosure is thus drawn to polypeptides comprising a polypeptide sequence that has at least 70%, preferably at least 80%, more preferably at least 90 %, 95 % 97 % or 99 % sequence identity with amino acid sequence selected from the group consisting of SEQ ID NO:107 to SEQ ID NO: 124.

By TCR alpha deficient T cell is intended an isolated T cell that lacks expression of a functional TCR alpha chain. This may be accomplished by different means, as non limiting examples, by engineering a T cell such that it does not express any functional TCR alpha on its cell surface or by engineering a T cell such that it produces very little functional TCR alpha chain on its surface or by engineering a T cell to express mutated or truncated form of TCR alpha chain.

TCR alpha deficient cells can no longer be expanded through CD3 complex. Thus, to overcome this problem and to allow proliferation ofTCR alpha deficient cells, pTalpha or functional variant thereof is introduced into said cells, thus restoring a functional CD3 complex. In a preferred aspect of the disclosure, the method further comprises introducing into said T cells rare-cutting endonucleases able to selectively inactivate by DNA cleavage one gene encoding one component of the T-cell receptor (TCR). In a particular aspect of the disclosure, said rare-cutting endonuclease is a TALE-nuclease. As non limiting examples, TALE-nuclease is directed against one of the gene target sequences of TCRalpha selected from the group consisting of SEQ ID NO: 37 and SEQ ID NO: 57 to 60. Preferably, TALE-nucleases are selected from the group consisting of SEQ ID NO: 41 and SEQ ID NO: 42.

In particular aspect of the disclosure, said method for expansion of TCR alpha deficient T-cells comprises an additional genomic modification step. By additional genomic modification step, can be intended the introduction into cells to engineer of one protein of interest. Said protein of interest can be, as non limiting examples, a Chimeric Antigen Receptor (CAR), particularly CAR comprising amino acid sequence SEQ ID NO: 73, a multichain CAR, particularly multi-chain CAR comprising amino acid sequence SEQ ID NO: 125 a bispecific antibody, rare-cutting endonucleases targeting PDCD1 or CTLA-4, particularly targeting nucleic acid sequence SEQ ID NO: 74 to SEQ ID NO: 78 or a rare-cutting endonuclease targeting a target for immunosuppressive agent as described in the present disclosure.

Are also encompassed in the present disclosure polypeptides encoding pTalpha, particularly functional variants described above. In a preferred aspect, the disclosure relates to a pTalpha or functional variant thereof fused to a signal transducing domain such as CD28, OX40, ICOS, CD137 and CD8. More particularly, the disclosure relates to pTalpha functional variant comprising amino acid sequence selected form the group consisting of SEQ ID NO: 107 to SEQ ID NO: 124. Are also encompassed in the present disclosure polynucleotides, vectors encoding pTalpha or functional variants thereof described above.

In the scope of the present disclosure are also encompassed isolated cells or cell lines susceptible to be obtained by said method. In particular, said isolated cells or cell lines are obtained by introducing into said cells a pTalpha or a functional variant thereof to support CD3 surface expression. In a preferred aspect, said isolated cell or cell line are further genetically modified by inactivating TCRalpha gene. This gene is preferably inactivating by at least one rare-cutting endonuclease. In a preferred aspect of the disclosure, said rare-cutting endonuclease is TALE-nuclease.

### Multi-chain Chimeric Antigen Receptor (CAR)

The method according to the present invention further comprises introducing a multi-chain chimeric antigen receptor (CAR) particularly adapted to the production and expansion of engineered T-cells of the present invention. The multi-chain CAR can comprise at least two of the following components:
a) one polypeptide comprising the transmembrembrane domain of FcεRl alpha chain and an extracellular ligand-binding domain,
b) one polypeptide comprising a part of N- and C- terminal cytoplasmic tail and the transmembrane domain of FcεRl beta chain and/or
c) two polypeptides comprising each a part of intracytoplasmic tail and the transmembrane domain of FcεRl gamma chain, whereby different polypeptides multimerize together spontaneously to form dimeric, trimeric or tetrameric CAR.

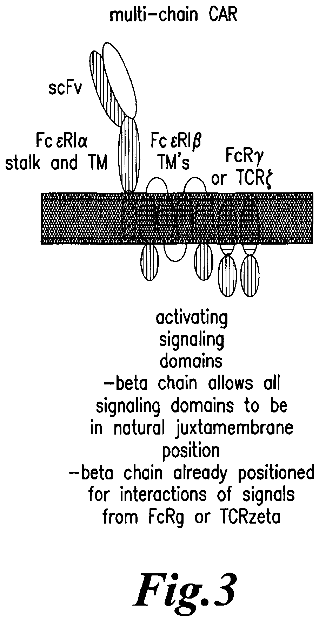

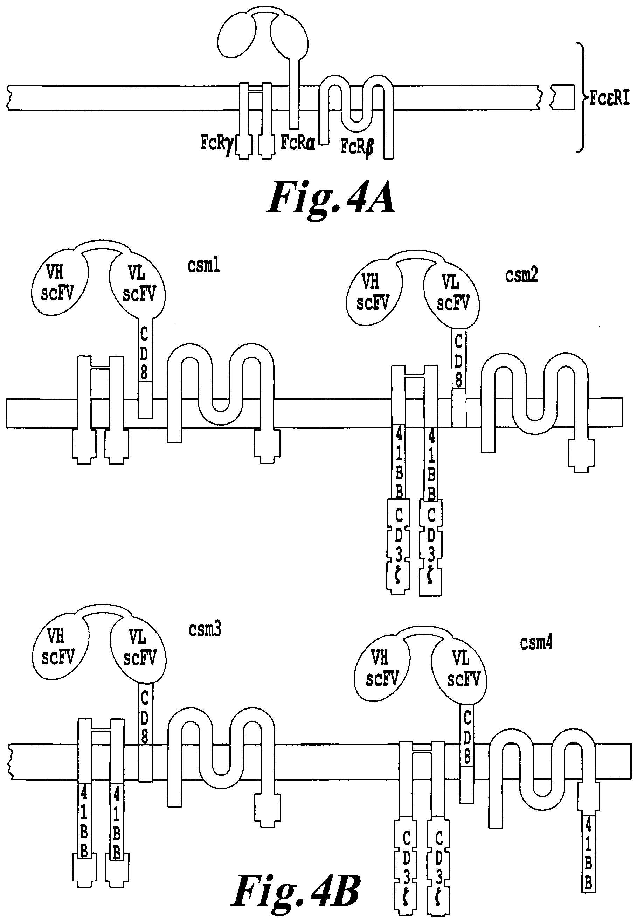

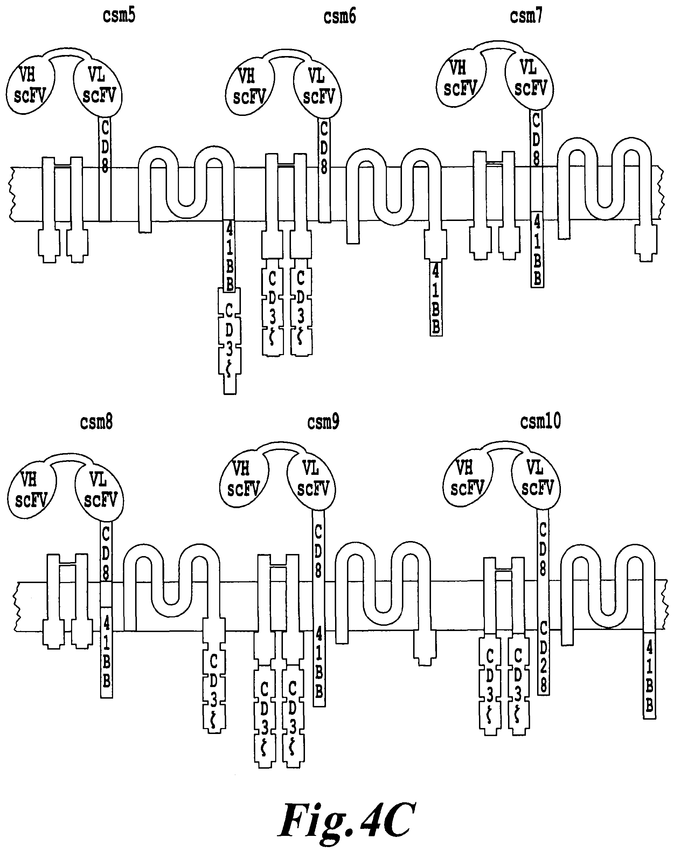

One example of tetrameric CAR is illustrated in Figure 3. Different versions of multichain CARs are represented in Figure 4. One example of multi-chain CAR comprises amino acid sequence SEQ ID NO: 125. The term "a part of" used herein refers to any subset of the molecule, that is a shorter peptide. Alternatively, amino acid sequence functional variants of the polypeptide can be prepared by mutations in the DNA which encodes the polypeptide. Such functional variants include, for example, deletions from, or insertions or substitutions of, residues within the amino acid sequence. Any combination of deletion, insertion, and substitution may also be made to arrive at the final construct, provided that the final construct possesses the desired activity, especially to exhibit a specific anti-target cellular immune activity.

In a preferred embodiment, said extracellular ligand-binding domain is a scFv. Other binding domains than scFv can also be used for predefined targeting of lymphocytes, such as camelid single-domain antibody fragments or receptor ligands like a vascular endothelial growth factor polypeptide, an integrin-binding peptide, heregulin or an IL-13 mutein, antibody binding domains, antibody hypervariable loops or CDRs as non limiting examples.

In a preferred embodiment said polypeptide of a) further comprises a stalk region between said extracellular ligand-binding domain and said transmembrane domain. The term "stalk region" used herein generally means any oligo- or polypeptide that functions to link the transmembrane domain to the extracellular ligand-binding domain. In particular, stalk region are used to provide more flexibility and accessibility for the extracellular ligand-binding domain. A stalk region may comprise up to 300 amino acids, preferably 10 to 100 amino acids and most preferably 25 to 50 amino acids. Stalk region may be derived from all or part of naturally occurring molecules, such as from all or part of the extracellular region of CD8, CD4 or CD28, or from all or part of an antibody constant region. Alternatively the stalk region may be a synthetic sequence that corresponds to a naturally occurring stalk sequence, or may be an entirely synthetic stalk sequence.

In a preferred embodiment, said polypeptide of a), b) and/or c) further comprises at least one signal-transducing domain. In a most preferred embodiment, said signal-transducing domain is selected from the group consisting of CD28, OX40, ICOS, CD137 and CD8.

In a preferred embodiment, said C-terminal cytoplasmic tail of FcεRl alpha, beta and/or gamma chain fragment further comprises TNFR-associated Factor 2 (TRAF2) binding motifs. In a most preferred embodiment, said C- terminal cytoplasmic tail of FcεRl alpha, beta and/or gamma chain is replaced by intracytoplasmic tail of costimulatory TNFR member family. Cytoplasmic tail of costimulatory TNFR family member contains TRAF2 binding motifs consisting of the major conserved motif (P/S/A)X(Q/E)E) or the minor motif (PXQXXD), wherein X is any amino acid. TRAF proteins are recruited to the intracellular tails of many TNFRs in response to receptor trimerization.

In another preferred embodiment said intracytoplasmic domain of FcεRl alpha, beta and/or gamma chain is replaced by intracytoplasmic domain of TCR zeta chain (also named CD3 zeta). In another preferred embodiment, said intracytoplasmic domain of FcεRl alpha, beta and/or gamma chain comprises at least one additional immunoreceptor tyrosine-based activation motif (ITAM). ITAMs are well defined signaling motifs found in the intracytoplasmic tail of a variety of receptors that serve as binding sites for syk/zap70 class tyrosine kinases. Examples of ITAM used in the invention include those derived from TCRzeta, FCRgamma, FCRbeta, CD3gamma, CD3delta, CD3epsilon, CD5, CD22, CD79a, CD79b, and CD66d.

As non limiting example, different versions of multi-chain CAR are illustrated in Figure 4.

In a preferred embodiment the multi-chain CAR comprise the amino acid sequence SEQ ID NO: 125. The present invention relates to polypeptides comprising a polypeptide sequence that has at least 70%, preferably at least 80%, more preferably at least 90 %, 95 % 97 % or 99 % sequence identity with amino acid sequence selected from the group consisting of SEQ ID NO: 125.

Are also comprised in the scope of the present invention, polynucleotides, vectors encoding the above described multi-chain CAR according to the invention.

In encompassed particular embodiment, the invention relates to a method of preparing Tcells for immunotherapy comprising introducing into said T-cells the different polypeptides composing said multi-chain CAR and expanding said cells.

Said method further comprises a step of genetically modifying said cells by inactivating at least one gene expressing one component of the TCR.. In a preferred embodiment, said gene is selected from the group consisting of TCRalpha and TCRbeta. Said method further comprise introducing into said T cells a rare-cutting endonuclease able to selectively inactivate by DNA cleavage said genes. In a more preferred embodiment said rare-cutting endonuclease is TALE-nuclease. Preferred TALE-nucleases according to the invention are those recognizing and cleaving the target sequence selected from the group consisting of: SEQ ID NO: 37, 57 to 60 (TCRalpha) and SEQ ID NO: 38 or 39 (TCRbeta). In another aspect of the disclosure, said method further comprises a step of genetically modifying said cells by inactivating at least one gene expressing a target for an immunosuppressive agent, such as CD52 and GR. Other TALE-nucleases according to the disclosure are those recognizing and cleaving the target sequence selected from the group consisting of SEQ ID NO: 1 to 6 (GR) and SEQ ID NO: 40, SEQ ID NO: 61 to SEQ ID NO: 65 (CD52).

Said method further comprises an additional genomic modification step. By additional genomic modification step, is intended the introduction into cells to engineer of a rare-cutting endonuclease targeting PDCD1. In particular aspect of the disclosure, said method further comprises an additional genomic modification step. By additional genomic modification step, can be intended the introduction into cells to engineer of one protein of interest. Said protein of interest can be, as non limiting examples a bispecific antibody, rare-cutting endonuclease targeting CTLA-4, a pTalpha or a functional variant thereof as described in the present disclosure.

The present invention also relates to isolated cells or cell lines susceptible to be obtained by said method to engineer cells. In particular said isolated cell comprises exogenous polynucleotide sequences encoding polypeptides composing said multi-chain CAR.

### Bispecific antibodies

According to a further aspect of the disclosure, engineered T cells obtained by the different methods as previously described can be further exposed with bispecific antibodies. Said T-cells could be exposed to bispecific antibodies *ex vivo* prior to administration to a patient or *in vivo* following administration to a patient. Said bispecific antibodies comprise two variable regions with distinct antigen properties that allow bringing the engineered cells into proximity to a target antigen. As a non limiting example, said bispecific antibody is directed against a tumor marker and lymphocyte antigen such as CD3 and has the potential to redirect and activate any circulating T cells against tumors.

### Delivery methods

The different methods described above involve introducing a protein of interest such as rare cutting endonuclease into a cell. As non-limiting example, said protein of interest can be introduced as transgenes preferably encoded by at least one plasmid vector. Polypeptides may be synthesized *in situ* in the cell as a result of the introduction of polynucleotides encoding said polypeptides into the cell. Alternatively, said polypeptides could be produced outside the cell and then introduced thereto. Methods for introducing a polynucleotide construct into cells are known in the art and including as non limiting examples stable transformation methods wherein the polynucleotide construct is integrated into the genome of the cell, transient transformation methods wherein the polynucleotide construct is not integrated into the genome of the cell and virus mediated methods. Said polynucleotides may be introduced into a cell by for example, recombinant viral vectors (e.g. retroviruses, adenoviruses), liposome and the like. For example, transient transformation methods include for example microinjection, electroporation or particle bombardment. Said polynucleotides may be included in vectors, more particularly plasmids or virus, in view of being expressed in cells. Said plasmid vector can comprise a selection marker which provides for identification and/or selection of cells which received said vector. Different transgenes can be included in one vector. Said vector can comprise a nucleic acid sequence encoding ribosomal skip sequence such as a sequence encoding a 2A peptide. 2A peptides, which were identified in the Aphthovirus subgroup of picornaviruses, causes a ribosomal "skip" from one codon to the next without the formation of a peptide bond between the two amino acids encoded by the codons (see Donnelly et al., J. of General Virology 82: 1013-1025 (2001); Donnelly et al., J. of Gen. Virology 78: 13-21 (1997); Doronina et al., Mol. And. Cell. Biology 28(13): 4227-4239 (2008); Atkins et al., RNA 13: 803-810 (2007)). By "codon" is meant three nucleotides on an mRNA (or on the sense strand of a DNA molecule) that are translated by a ribosome into one amino acid residue. Thus, two polypeptides can be synthesized from a single, contiguous open reading frame within an mRNA when the polypeptides are separated by a 2A oligopeptide sequence that is in frame. Such ribosomal skip mechanisms are well known in the art and are known to be used by several vectors for the expression of several proteins encoded by a single messenger RNA. As non-limiting example, in the present disclosure, 2A peptides have been used to express into the cell the rare-cutting endonuclease and a DNA end-processing enzyme.

A more preferred embodiment of the invention, polynucleotides encoding polypeptides according to the present invention can be mRNA which is introduced directly into the cells, for example by electroporation. The inventors determined the optimal condition for mRNA electroporation in T-cell.

The inventor used the cytoPulse technology which allows, by the use of pulsed electric fields, to transiently permeabilize living cells for delivery of material into the cells. The technology, based on the use of PulseAgile (BTX Havard Apparatus, 84 October Hill Road, Holliston, MA 01746, USA) electroporation waveforms grants the precise control of pulse duration, intensity as well as the interval between pulses (U.S. patent 6,010,613 and International PCT application WO2004083379). All these parameters can be modified in order to reach the best conditions for high transfection efficiency with minimal mortality. Basically, the first high electric field pulses allow pore formation, while subsequent lower electric field pulses allow to move the polynucleotide into the cell.

### Activation and expansion of T cells

Whether prior to or after genetic modification of the T cells, the T cells can be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 2006/0121005A1. T cells can be expanded *in vitro* or *in vivo.* Generally, the T cells of the disclosure are expanded by contact with an agent that stimulates a CD3 TCR complex and a co-stimulatory molecule on the surface of the T cells to create an activation signal for the T-cell. For example, chemicals such as calcium ionophore A23187, phorbol 12-myristate 13-acetate (PMA), or mitogenic lectins like phytohemagglutinin (PHA) can be used to create an activation signal for the T-cell. As non limiting examples, T cell populations may be stimulated *in vitro* such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody. For example, the agents providing each signal may be in solution or coupled to a surface. As those of ordinary skill in the art can readily appreciate, the ratio of particles to cells may depend on particle size relative to the target cell.

Conditions appropriate for T cell culture include an appropriate media (e.g., Minimal Essential Media or RPMI Media 1640 or, X-vivo 5, (Lonza)) that may contain factors necessary for proliferation and viability, including serum (e.g., fetal bovine or human serum), interleukin-2 (IL-2), insulin, IFN-g , 1L-4, 1L-7, GM-CSF, -10, - 2, 1L-15, TGFp, and TNF- or any other additives for the growth of cells known to the skilled artisan. Other additives for the growth of cells include, but are not limited to, surfactant, plasmanate, and reducing agents such as N-acetyl-cysteine and 2-mercaptoethanoi. Media can include RPMI 1640, A1M-V, DMEM, MEM, a-MEM, F-12, X-Vivo 1, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% C02). T cells that have been exposed to varied stimulation times may exhibit different characteristics.

### Engineered T-cells

In the scope of the present invention is also encompassed an isolated T cell obtained according to any one of the methods according to the invention. The isolated T cell according to the present invention comprises two inactivated genes, namely a first gene encoding PD1 and a second gene selected from the group consisting TCR alpha and TCR beta, and expresses a CAR such as a multi-chain CAR directed against CD19. In the scope of the present disclosure is also encompassed an isolated T cell obtained according to any one of the methods previously described. Said T-cell according to the present disclosure can be derived from a stem cell. The stem cells can be adult stem cells, embryonic stem cells, more particularly non-human stem cells, cord blood stem cells, progenitor cells, bone marrow stem cells, induced pluripotent stem cells, non-human totipotent stem cells or hematopoietic stem cells. Representative human cells are CD34+ cells. Said isolated cell can also be a dendritic cell, a NK-cell, a B-cell or a T-cell selected from the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. Prior to expansion and genetic modification of the cells of the disclosure, a source of cells can be obtained from a subject through a variety of non-limiting methods. T cells can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In certain embodiments of the present disclosure, any number of T cell lines available and known to those skilled in the art, may be used. In another embodiment, said cell can be derived from a healthy donor, from a patient diagnosed with cancer or from a patient diagnosed with an infection. In another embodiment, said cell is part of a mixed population of cells which present different phenotypic characteristics. In the scope of the present disclosure is also encompassed a cell line obtained from a transformed T- cell according to the method previously described. In a more particular embodiment, the present disclosure relates to an isolated T-cell comprising at least two inactivated genes encoding for immune checkpoint proteins, more particularly, at least two genes selected from the group consisting of: CTLA4, PPP2CA, PPP2CB, PTPN6, PTPN22, PDCD1, LAG3, HAVCR2, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3.

In another aspect of the present disclosure, said isolated cell according to the present disclosure can further comprises one inactivated gene selected from the group consisting of CD52, GR, TCR alpha and TCR beta and/or expresses a CAR, a multi-chain CAR and/or a pTalpha transgene.

### Therapeutic applications

In another embodiment, isolated cell obtained by the different methods or cell line derived from said isolated cell according to the invention can be used as a medicament. In another embodiment, said medicament can be used for treating cancer in a patient in need thereof. In another embodiment, said isolated cell according to the invention or cell line derived from said isolated cell can be used in the manufacture of a medicament for treatment of a cancer in a patient in need thereof.

In another aspect of the disclosure, the present invention relies on methods for treating patients in need thereof, said method comprising at least one of the following steps:
(a) providing a T-cell obtainable by any one of the methods previously described;
(b)Administrating said transformed T-cells to said patient,

On one aspect, said T cells of the disclosure can undergo robust *in vivo* T cell expansion and can persist for an extended amount of time.

Said treatment can be ameliorating, curative or prophylactic. It may be either part of an autologous immunotherapy or part of an allogenic immunotherapy treatment. By autologous, it is meant that cells, cell line or population of cells used for treating patients are originating from said patient or from a Human Leucocyte Antigen (HLA) compatible donor. By allogeneic is meant that the cells or population of cells used for treating patients are not originating from said patient but from a donor.

Cells that can be used with the disclosed methods are described in the previous section. Said treatment can be used to treat patients diagnosed with cancer, viral infection, autoimmune disorders or Graft versus Host Disease (GvHD). Cancers that may be treated include tumors that are not vascularized, or not yet substantially vascularized, as well as vascularized tumors. The cancers may comprise nonsolid tumors (such as hematological tumors, for example, leukemias and lymphomas) or may comprise solid tumors. Types of cancers to be treated with the CARs of the invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

It can be a treatment in combination with one or more therapies against cancer selected from the group of antibodies therapy, chemotherapy, cytokines therapy, dendritic cell therapy, gene therapy, hormone therapy, laser light therapy and radiation therapy.

The administration of the cells or population of cells according to the present invention may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous or intralymphatic injection, or intraperitoneally. In one embodiment, the cell compositions of the present invention are preferably administered by intravenous injection.

The administration of the cells or population of cells can consist of the administration of 10⁴-10⁹ cells per kg body weight, preferably 10⁵ to 10⁶ cells/kg body weight including all integer values of cell numbers within those ranges. The cells or population of cells can be administrated in one or more doses. In another aspect, said effective amount of cells are administrated as a single dose. In another aspect, said effective amount of cells are administrated as more than one dose over a period time. Timing of administration is within the judgment of managing physician and depends on the clinical condition of the patient. The cells or population of cells may be obtained from any source, such as a blood bank or a donor. While individual needs vary, determination of optimal ranges of effective amounts of a given cell type for a particular disease or conditions within the skill of the art. An effective amount means an amount which provides a therapeutic or prophylactic benefit. The dosage administrated will be dependent upon the age, health and weight of the recipient, kind of concurrent treatment, if any, frequency of treatment and the nature of the effect desired.

In another aspect, said effective amount of cells or composition comprising those cells are administrated parenterally. Said administration can be an intravenous administration. Said administration can be directly done by injection within a tumor.

In certain aspects of the present disclosure, cells are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or nataliziimab treatment for MS patients or efaliztimab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludaribine, cyclosporin, FK506, rapamycin, mycoplienolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin) (Liu et al., Cell 66:807-815, 1 1; Henderson et al., Immun. 73:316-321, 1991; Bierer et al., Citrr. Opin. mm n. 5:763-773, 93). In a further embodiment, the cell compositions of the present invention are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH, In another embodiment, the cell compositions of the present invention are administered following B-cell ablative therapy such as agents that react with CD20, e.g., Rituxan. For example, subjects may undergo standard treatment with high dose chemotherapy followed by peripheral blood stem cell transplantation. Following the transplant, subjects may receive an infusion of the expanded immune cells of the present invention. Expanded cells may also be administered before or following surgery.

### Example of method to engineer human allogeneic cells for immunotherapy

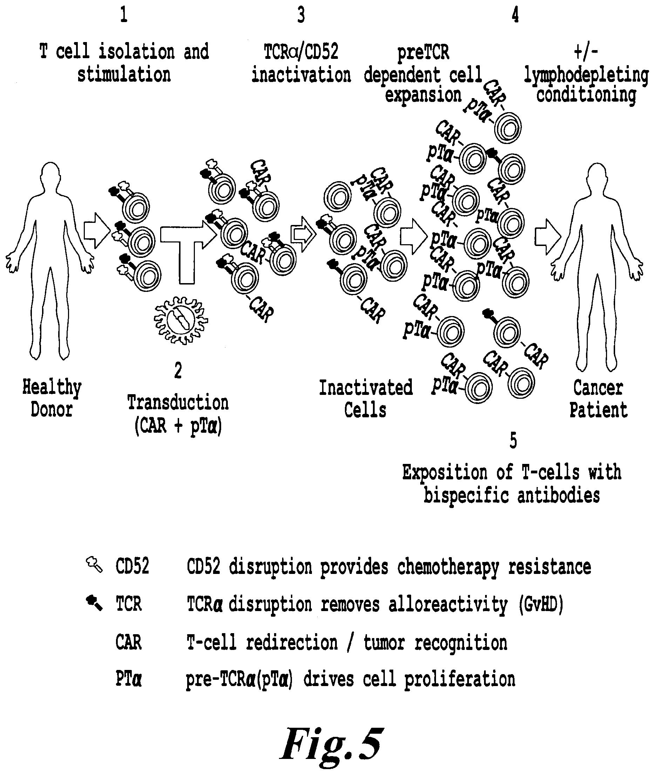

For a better understanding of the disclosure, one example of method to engineer human allogenic cells for immunotherapy is illustrated in Figure 5. The method comprising a combination of one or several of the following steps:
1. Providing T-cells from a cell culture or from a blood sample from one individual patient or from blood bank and activating said T cells using anti-CD3/C28 activator beads. The beads provide both the primary and co-stimulatory signals that are required for activation and expansion of T cells.
2.
   a) Transducing said cells with pTalpha or functional variant thereof transgene to support CD3 surface expression and allow cell expansion through stimulation of CD3 complex. TCR disruption is expected to the elimination of the TCR complex and removes alloreactivity (GvHD) but may alter allogenic cells expansion due to the loss of CD3 signaling component. Transduced cells are expected to express pTalpha chain or functional variant thereof. This pTalpha chain pairs with TCRbeta chain and CD3 signaling components to form the preTCR complex and, thus restore a functional CD3 complex and support activation or stimulation of inactivated TCRalpha cells. Transduction of T-cells with pTalpha lentiviral vector can be realized before or after TCRalpha inactivation.
   b) Transducing said cells with multi-chain CARs allow redirecting T cells against antigens expressed at the surface of target cells from various malignancies including lymphomas and solid tumors. To improve the function of co-stimulatory domain, the inventors have designed a multi-chain CAR derived from FcεRI as previously described. Transduction can be realized before or after the inactivation of TCRalpha and the other genes, such as CD52 genes.
3. Engineering non alloreactive and immunosuppressive resistant T cells:
   a) It is possible to Inactivate TCR alpha in said cells to eliminate the TCR from the surface of the cell and prevent recognition of host tissue as foreign by TCR of allogenic and thus to avoid GvHD.
   b) It is also possible to inactive one gene encoding target for immunosuppressive agent to render said cells resistant to immunosuppressive treatment to prevent graft rejection without affecting transplanted T cells. In this example, target of immunosuppressive agents is CD52 and immunosuppressive agent is a humanized monoclonal anti-CD52 antibody.
      It has been shown by the inventors that the use of TALE-nuclease by allowing higher rates of DSB events within T-cells was particularly advantageous to achieve the above double inactivation in T-cells. Preferably, TCRalpha and CD52 genes are inactivated by electoporating T cells with mRNA coding for TALE-nuclease targeting said genes. It has been found by the inventors that using mRNA resulted into high transformation rate was less harmful to T-cells and so, was critical in the process of engineering T-cells. Then, inactivated T cells are sorted using magnetic beads. For example, T cells expressing CD52 are removed by fixation on a solid surface, and inactivated cells are not exposed of the stress of being passed through a column. This gentle method increases the concentration of properly engineered T-cells.
4. Expansion *in vitro* of engineered T-cells prior to administration to a patient or *in vivo* following administration to a patient through stimulation of CD3 complex. Before administration step, patients can be subjected to an immunosuppressive treatment such as CAMPATH1-H, a humanized monoclonal antibody anti-CD52.
5. Optionally exposed said cells with bispecific antibodies *ex vivo* prior to administration to a patient or *in vivo* following administration to a patient to bring the engineered cells into proximity to a target antigen.

### Other definitions

- "As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- by "DNA target", "DNA target sequence", "target DNA sequence", "nucleic acid target sequence", "target sequence" , or "processing site" is intended a polynucleotide sequence that can be targeted and processed by a rare-cutting endonuclease according to the present disclosure. These terms refer to a specific DNA location, preferably a genomic location in a cell, but also a portion of genetic material that can exist independently to the main body of genetic material such as plasmids, episomes, virus, transposons or in organelles such as mitochondria as non-limiting example. As non-limiting examples of TALE-nuclease targets, targeted genomic sequences generally consist of two 17-bp long sequences (called half targets) separated by a 15-bp spacer. Each half-target is recognized by repeats of TALE-nucleases listed in tables 2, 6, 7 and 11 as non-limiting examples, encoded in plasmids, under the control of EF1-alpha promoter or T7 promoter. The nucleic acid target sequence is defined by the 5' to 3' sequence of one strand of said target, as indicated in tables 1, 5, 6 and 10.

- The term "transgene" means a nucleic acid sequence (encoding, e.g., one or more polypeptides), which is partly or entirely heterologous, i.e., foreign, to the host cell into which it is introduced, or, is homologous to an endogenous gene of the host cell into which it is introduced, but which can be designed to be inserted, or can be inserted, into the cell genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of the selected nucleic acid encoding polypeptide. The polypeptide encoded by the transgene can be either not expressed, or expressed but not biologically active, in cells in which the transgene is inserted.
- By "genome" it is meant the entire genetic material contained in a cell such as nuclear genome, chloroplastic genome, mitochondrial genome.
- By chimeric antigen receptor (CAR) is intended molecules that combine a binding domain against a component present on the target cell, for example an antibody-based specificity for a desired antigen (e.g., tumor antigen) with a T cell receptor-activating intracellular domain to generate a chimeric protein that exhibits a specific anti-target cellular immune activity. Generally, CAR consists of an extracellular single chain antibody (scFvFc) fused to the intracellular signaling domain of the T cell antigen receptor complex zeta chain (scFvFc:ζ) and have the ability, when expressed in T cells, to redirect antigen recognition based on the monoclonal antibody's specificity. The CAR used in the present invention is a CAR directed against CD19 antigen and can comprise as non limiting example the amino acid sequence : SEQ ID NO: 73
- By " delivery vector" or " delivery vectors" is intended any delivery vector which can be used to put into cell contact ( i.e "contacting") or deliver inside cells or subcellular compartments (i.e "introducing") agents/chemicals and molecules (proteins or nucleic acids) needed. It includes, but is not limited to liposomal delivery vectors, viral delivery vectors, drug delivery vectors, chemical carriers, polymeric carriers, lipoplexes, polyplexes, dendrimers, microbubbles (ultrasound contrast agents), nanoparticles, emulsions or other appropriate transfer vectors. These delivery vectors allow delivery of molecules, chemicals, macromolecules (genes, proteins), or other vectors such as plasmids, peptides developed by Diatos. In these cases, delivery vectors are molecule carriers. By "delivery vector" or "delivery vectors" is also intended delivery methods to perform transfection.
- The terms "vector" or "vectors" refer to a nucleic acid molecule capable of transporting another nucleic acid to which it has been linked. A "vector" herein includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).
- By "lentiviral vector" is meant HIV-Based lentiviral vectors that are very promising for gene delivery because of their relatively large packaging capacity, reduced immunogenicity and their ability to stably transduce with high efficiency a large range of different cell types. Lentiviral vectors are usually generated following transient transfection of three (packaging, envelope and transfer) or more plasmids into producer cells. Like HIV, lentiviral vectors enter the target cell through the interaction of viral surface glycoproteins with receptors on the cell surface. On entry, the viral RNA undergoes reverse transcription, which is mediated by the viral reverse transcriptase complex. The product of reverse transcription is a double-stranded linear viral DNA, which is the substrate for viral integration in the DNA of infected cells. By "integrative lentiviral vectors (or LV)", is meant such vectors as non limiting example, that are able to integrate the genome of a target cell. At the opposite by "non integrative lentiviral vectors (or NILV)" is meant efficient gene delivery vectors that do not integrate the genome of a target cell through the action of the virus integrase.
- Delivery vectors and vectors can be associated or combined with any cellular permeabilization techniques such as sonoporation or electroporation or derivatives of these techniques.
- By cell or cells is intended any eukaryotic living cells, primary cells and cell lines derived from these organisms for *in vitro* cultures.
- By "primary cell" or "primary cells" are intended cells taken directly from living tissue (i.e. biopsy material) and established for growth in vitro, that have undergone very few population doublings and are therefore more representative of the main functional components and characteristics of tissues from which they are derived from, in comparison to continuous tumorigenic or artificially immortalized cell lines.

As non limiting examples cell lines can be selected from the group consisting of CHO-K1 cells; HEK293 cells; Caco2 cells; U2-OS cells; NIH 3T3 cells; NSO cells; SP2 cells; CHO-S cells; DG44 cells; K-562 cells, U-937 cells; MRCS cells; IMR90 cells; Jurkat cells; HepG2 cells; HeLa cells; HT-1080 cells; HCT-116 cells; Hu-h7 cells; Huvec cells; Molt 4 cells.

All these cell lines can be modified by the method of the present disclosure to provide cell line models to produce, express, quantify, detect, study a gene or a protein of interest; these models can also be used to screen biologically active molecules of interest in research and production and various fields such as chemical, and therapeutics as non-limiting examples.
- by "mutation" is intended the substitution, deletion, insertion of up to one, two, three, four, five, six, seven, eight, nine, ten, eleven, twelve, thirteen, fourteen, fifteen, twenty, twenty five, thirty, fourty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- by "variant(s)", it is intended a repeat variant, a variant, a DNA binding variant, a TALE-nuclease variant, a polypeptide variant obtained by mutation or replacement of at least one residue in the amino acid sequence of the parent molecule.
- by "functional variant" is intended a catalytically active mutant of a protein or a protein domain; such mutant may have the same activity compared to its parent protein or protein domain or additional properties, or higher or lower activity.
- By "gene" is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns, a 3' untranslated region. The gene may further comprise a terminator, enhancers and/or silencers.
- As used herein, the term "locus" is the specific physical location of a DNA sequence (e.g. of a gene) on a chromosome. The term "locus" can refer to the specific physical location of a rare-cutting endonuclease target sequence on a chromosome. Such a locus can comprise a target sequence that is recognized and/or cleaved by a rare-cutting endonuclease according to the disclosure. It is understood that the locus of interest of the present disclosure can not only qualify a nucleic acid sequence that exists in the main body of genetic material (i.e. in a chromosome) of a cell but also a portion of genetic material that can exist independently to said main body of genetic material such as plasmids, episomes, virus, transposons or in organelles such as mitochondria as non-limiting examples.

The term "rare-cutting endonuclease" refers to a wild type or variant enzyme capable of catalyzing the hydrolysis (cleavage) of bonds between nucleic acids within a DNA or RNA molecule, preferably a DNA molecule. Particularly, said nuclease can be an endonuclease, more preferably a rare-cutting endonuclease which is highly specific, recognizing nucleic acid target sites ranging from 10 to 45 base pairs (bp) in length, usually ranging from 10 to 35 base pairs in length. The endonuclease according to the present disclosure recognizes and cleaves nucleic acid at specific polynucleotide sequences, further referred to as "target sequence". The rare-cutting endonuclease can recognize and generate a single- or double-strand break at specific polynucleotides sequences.

In a particular embodiment, said rare-cutting endonuclease according to the present disclosure can be a Cas9 endonuclease. Indeed, recently a new genome engineering tool has been developed based on the RNA-guided Cas9 nuclease (Gasiunas, Barrangou et al. 2012; Jinek, Chylinski et al. 2012; Cong, Ran et al. 2013; Mali, Yang et al. 2013) from the type II prokaryotic CRISPR (Clustered Regularly Interspaced Short palindromic Repeats) adaptive immune system (see for review (Sorek, Lawrence et al. 2013)). The CRISPR Associated (Cas) system was first discovered in bacteria and functions as a defense against foreign DNA, either viral or plasmid. CRISPR-mediated genome engineering first proceeds by the selection of target sequence often flanked by a short sequence motif, referred as the proto-spacer adjacent motif (PAM). Following target sequence selection, a specific crRNA, complementary to this target sequence is engineered. Trans-activating crRNA (tracrRNA) required in the CRISPR type II systems paired to the crRNA and bound to the provided Cas9 protein. Cas9 acts as a molecular anchor facilitating the base pairing of tracRNA with cRNA (Deltcheva, Chylinski et al. 2011). In this ternary complex, the dual tracrRNA:crRNA structure acts as guide RNA that directs the endonuclease Cas9 to the cognate target sequence. Target recognition by the Cas9-tracrRNA:crRNA complex is initiated by scanning the target sequence for homology between the target sequence and the crRNA. In addition to the target sequence-crRNA complementarity, DNA targeting requires the presence of a short motif adjacent to the protospacer (protospacer adjacent motif - PAM). Following pairing between the dual-RNA and the target sequence, Cas9 subsequently introduces a blunt double strand break 3 bases upstream of the PAM motif (Garneau, Dupuis et al. 2010). In the present disclosure, guide RNA can be designed for example to specifically target a gene encoding a TCR component. Following the pairing between the guide RNA and the target sequence, Cas9 induce a cleavage within TCR gene.

Rare-cutting endonuclease can also be a homing endonuclease, also known under the name of meganuclease. Such homing endonucleases are well-known to the art (Stoddard 2005). Homing endonucleases are highly specific, recognizing DNA target sites ranging from 12 to 45 base pairs (bp) in length, usually ranging from 14 to 40 bp in length.

The homing endonuclease according to the disclosure may for example correspond to a LAGLIDADG endonuclease, to a HNH endonuclease, or to a GIY-YIG endonuclease. Preferred homing endonuclease according to the present disclosure can be an I-*Crel* variant. A "variant" endonuclease, i.e. an endonuclease that does not naturally exist in nature and that is obtained by genetic engineering or by random mutagenesis can bind DNA sequences different from that recognized by wild-type endonucleases (see international application WO2006/097854).

Said rare-cutting endonuclease can be a modular DNA binding nuclease. By modular DNA binding nuclease is meant any fusion proteins comprising at least one catalytic domain of an endonuclease and at least one DNA binding domain or protein specifying a nucleic acid target sequence. The DNA binding domain is generally a RNA or DNA-binding domain formed by an independently folded polypeptide protein domain that contains at least one motif that recognizes double- or single-stranded DNA. Many such polypeptides have been described in the art having the ability to bind specific nucleic acid sequences. Such binding domains often comprise, as non limiting examples, helix-turn helix domains, leucine zipper domains, winged helix domains, helix-loop-helix domains, HMG-box domains, Immunoglobin domains, B3 domain or engineered zinc finger domain.

According to a preferred embodiment of the disclosure, the DNA binding domain is derived from a Transcription Activator like Effector (TALE), wherein sequence specificity is driven by a series of 33-35 amino acids repeats originating from *Xanthomonas* or *Ralstonia* bacterial proteins. These repeats differ essentially by two amino acids positions that specify an interaction with a base pair (Boch, Scholze et al. 2009; Moscou and Bogdanove 2009). Each base pair in the DNA target is contacted by a single repeat, with the specificity resulting from the two variant amino acids of the repeat (the so-called repeat variable dipeptide, RVD). TALE binding domains may further comprise an N-terminal translocation domain responsible for the requirement of a first thymine base (T₀) of the targeted sequence and a C-terminal domain that containing a nuclear localization signals (NLS). A TALE nucleic acid binding domain generally corresponds to an engineered core TALE scaffold comprising a plurality of TALE repeat sequences, each repeat comprising a RVD specific to each nucleotides base of a TALE recognition site. In the present disclosure, each TALE repeat sequence of said core scaffold is made of 30 to 42 amino acids, more preferably 33 or 34 wherein two critical amino acids (the so-called repeat variable dipeptide, RVD) located at positions 12 and 13 mediates the recognition of one nucleotide of said TALE binding site sequence; equivalent two critical amino acids can be located at positions other than 12 and 13 specially in TALE repeat sequence taller than 33 or 34 amino acids long. Preferably, RVDs associated with recognition of the different nucleotides are HD for recognizing C, NG for recognizing T, NI for recognizing A, NN for recognizing G or A. In another embodiment, critical amino acids 12 and 13 can be mutated towards other amino acid residues in order to modulate their specificity towards nucleotides A, T, C and G and in particular to enhance this specificity. A TALE nucleic acid binding domain usually comprises between 8 and 30 TALE repeat sequences. More preferably, said core scaffold of the present disclosure comprises between 8 and 20 TALE repeat sequences; again more preferably 15 TALE repeat sequences. It can also comprise an additional single truncated TALE repeat sequence made of 20 amino acids located at the C-terminus of said set of TALE repeat sequences, i.e. an additional C-terminal half- TALE repeat sequence.

Other engineered DNA binding domains are modular base-per-base specific nucleic acid binding domains (MBBBD) (WO2014018601). Said MBBBD can be engineered, for instance, from the newly identified proteins, namely EAV36_BURRH, E5AW43_BURRH, E5AW45_BURRH and E5AW46_BURRH proteins from the recently sequenced genome of the endosymbiont fungi *Burkholderia Rhizoxinica* (Lackner, Moebius et al. 2011). MBBBD proteins comprise modules of about 31 to 33 amino acids that are base specific. These modules display less than 40 % sequence identity with *Xanthomonas* TALE common repeats, whereas they present more polypeptides sequence variability. When they are assembled together, these modular polypeptides can although target specific nucleic acid sequences in a quite similar fashion as *Xanthomonas* TALE-nucleases. According to a preferred embodiment of the present disclosure, said DNA binding domain is an engineered MBBBD binding domain comprising between 10 and 30 modules, preferably between 16 and 20 modules. The different domains from the above proteins (modules, N and C-terminals) from Burkholderia and *Xanthomonas* are useful to engineer new proteins or scaffolds having binding properties to specific nucleic acid sequences. In particular, additional N-terminal and C-terminal domains of engineered MBBBD can be derived from natural TALE like AvrBs3, PthXo1, AvrHah1, PthA, Tallc as non-limiting examples.

"TALE-nuclease" or "MBBBD-nuclease" refers to engineered proteins resulting from the fusion of a DNA binding domain typically derived from Transcription Activator like Effector proteins (TALE) or MBBBD binding domain, with an endonuclease catalytic domain. Such catalytic domain is preferably a nuclease domain and more preferably a domain having endonuclease activity, like for instance I-Tevl, ColE7, NucA and Fok-I. In a particular embodiment, said nuclease is a monomeric TALE-Nuclease or MBBBD-nuclease. A monomeric Nuclease is a nuclease that does not require dimerization for specific recognition and cleavage, such as the fusions of engineered DNA binding domain with the catalytic domain of I-Tevl described in WO2012138927. In another particular embodiment, said rare-cutting endonuclease is a dimeric TALE-nuclease or MBBBD-nuclease, preferably comprising a DNA binding domain fused to Fokl. TALE-nuclease have been already described and used to stimulate gene targeting and gene modifications. Such engineered TALE-nucleases are commercially available under the trade name TALEN^{™} (Cellectis, 8 rue de la Croix Jarry, 75013 Paris, France).
- The term "cleavage" refers to the breakage of the covalent backbone of a polynucleotide. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. Double stranded DNA, RNA, or DNA/RNA hybrid cleavage can result in the production of either blunt ends or staggered ends.
- By "fusion protein" is intended the result of a well-known process in the art consisting in the joining of two or more genes which originally encode for separate proteins or part of them, the translation of said "fusion gene" resulting in a single polypeptide with functional properties derived from each of the original proteins.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated.
- "signal-transducing domain" or "co-stimulatory ligand" refers to a molecule on an antigen presenting cell that specifically binds a cognate co-stimulatory molecule on a T-cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A co-stimulatory ligand can include but is not limited to CD7, B7-1 (CD80), B7-2 (CD86), PD-L1, PD-L2, 4-1BBL, OX40L, inducible costimulatory igand (ICOS-L), intercellular adhesion molecule (ICAM, CD30L, CD40, CD70, CD83, HLA-G, MICA, M1CB, HVEM, lymphotoxin beta receptor, 3/TR6, ILT3, ILT4, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as but not limited to, CD27, CD28, 4-IBB, OX40, CD30, CD40, PD-1, ICOS, lymphocyte function-associated antigen-1 (LFA-1), CD2, CD7, LTGHT, NKG2C, B7-H3, a ligand that specifically binds with CD83.
- A "co-stimulatory molecule" refers to the cognate binding partner on a Tcell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor.
- A "co-stimulatory signal" as used herein refers to a signal, which in combination with primary signal, such as TCR/CD3 ligation, leads to T cell proliferation and/or upregulation or downregulation of key molecules.
- "bispecific antibody" refers to an antibody that has binding sites for two different antigens within a single antibody molecule. It will be appreciated by those skilled in the art that other molecules in addition to the canonical antibody structure may be constructed with two binding specificities. It will further be appreciated that antigen binding by bispecific antibodies may be simultaneous or sequential. Bispecific antibodies can be produced by chemical techniques (see e.g., Kranz et al. (1981) Proc. Natl. Acad. Sci. USA 78, 5807), by "polydoma" techniques (See U.S. Pat. No. 4,474,893) or by recombinant DNA techniques, which all are known per se. As a non limiting example, each binding domain comprises at least one variable region from an antibody heavy chain ("VH or H region"), wherein the VH region of the first binding domain specifically binds to the lymphocyte marker such as CD3, and the VH region of the second binding domain specifically binds to tumor antigen.
- The term "extracellular ligand-binding domain" as used herein is defined as an oligo- or polypeptide that is capable of binding a ligand. Preferably, the domain will be capable of interacting with a cell surface molecule. For example, the extracellular ligand-binding domain may be chosen to recognize a ligand that acts as a cell surface marker on target cells associated with a particular disease state. Thus examples of cell surface markers that may act as ligands include those associated with viral, bacterial and parasitic infections, autoimmune disease and cancer cells.
- The term "subject" or "patient" as used herein includes all members of the animal kingdom including non-human primates and humans.

The above written description of the invention provides a manner and process of making and using it such that any person skilled in this art is enabled to make and use the same, this enablement being provided in particular for the subject matter of the appended claims, which make up a part of the original description.

Where a numerical limit or range is stated herein, the endpoints are included. Also, all values and subranges within a numerical limit or range are specifically included as if explicitly written out.

The above description is presented to enable a person skilled in the art to make and use the invention, and is provided in the context of a particular application and its requirements. Various modifications to the preferred embodiments will be readily apparent to those skilled in the art.

Having generally described this invention, a further understanding can be obtained by reference to certain specific examples, which are provided herein for purposes of illustration only, and are not intended to be limiting unless otherwise specified.

### Examples

### Example 1: TALE-nucleases cleaving the human GR gene

6 heterodimeric TALE-nucleases targeting exons of the human GR gene were designed and produced. Table 2 below indicates the target sequences cleaved by each TALE-nuclease. GR TALE-nuclease was composed of two independent entities (called half TALE-nucleases) each containing a repeat sequence engineered to bind and cleave GR target sequences consisting of two 17-bp long sequences (called half targets) separated by a 15-bp spacer.

**Table 2: Description of the GR TALE-nucleases and sequences of the TALE-nucleases target sites in the human GR gene.**

| **Target name** | **Target sequence** | **Repeat sequence** | **Half TALE-nuclease sequence** |
|---|---|---|---|
| **GRex2** | TATTCACTGATGGACTC caaagaatcattaac TCCTGGTAGAGAAGAAA (SEQ ID NO: 1) | Repeat GRex2-LPT9-L1 (SEQ ID NO: 7) | GRex2-L TALEN (SEQ ID NO: 19) |
| | | Repeat -GRex2-LPT9-R1 (SEQ ID NO: 8) | GRex2-R TALEN (SEQ ID NO: 20) |
| **GRex3T2** | TGCCTGGTGTGCTCTGA tgaagcttcaggatg TCATTATGGAGTCTTAA (SEQ ID NO: 2) | Repeat -GRex3T2-L1 (SEQ ID NO: 9) | GRex3T2-L TALEN (SEQ ID NO: 21) |
| | | Repeat -GRex3T2-R1 (SEQ ID NO: 10) | GRex3T2-R TALEN (SEQ ID NO: 22) |
| **GRex3T4** | TGCTCTGATGAAGCTTC aggatgtcattatgg AGTCTTAACTTGTGGAA (SEQ ID NO: 3) | Repeat -GRex3T4-L1 (SEQ ID NO: 11) | GRex3T4-L TALEN (SEQ ID NO: 23) |
| | | Repeat -GRex3T4-R1 (SEQ ID NO: 12) | GRex3T4-R TALEN (SEQ ID NO: 24) |
| **GRexST1** | TGGTGTCACTGTTGGAG gttattgaacctgaa GTGTTATATGCAGGATA (SEQ ID NO: 4) | Repeat -GRex5T1-LPT8-L1 (SEQ ID NO: 13) | GRex5T1-L TALEN (SEQ ID NO: 25) |
| | | Repeat -GRex5T1-LPT8-R1 (SEQ ID NO: 14) | GRex5T1-R TALEN (SEQ ID NO: 26) |
| **GRex5T2** | TATGATAGCTCTGTTCC agactcaacttggag GATCATGACTACGCTCA (SEQ ID NO: 5) | Repeat -GRex5T2-L1 (SEQ ID NO: 15) | GRex5T2-L TALEN (SEQ ID NO: 27) |
| | | Repeat GRex5T2-R1 (SEQ ID NO: 16) | GRex5T2-R TALEN (SEQ ID NO: 28) |
| **GRex5T3** | TTATATGCAGGATATGA tagctctgttccaga CTCAACTTGGAGGATCA (SEQ ID NO: 6) | Repeat -GRex5T3-L1 (SEQ ID NO: 17) | GRex5T3-L TALEN (SEQ ID NO: 29) |
| | | Repeat -GRex5T3-R1 (SEQ ID NO: 18) | GRex5T3-R TALEN (SEQ ID NO: 30) |

The amino acid sequences of the N-terminal, C-terminal domains and repeat are based on the AvrBs3 TALE (ref: GenBank: X16130.1). The C-terminal and the N-terminal domains are separated by two BsmBl restriction sites. The repeat arrays (SEQ ID NO: 7 to 18), targeting the desired sequences (SEQ ID NO: 1 to 6) were synthesized using a solid support method composed of consecutive restriction/ligation/washing steps (International PCT application WO2013/017950). In brief, the first block (coding for a di-repeat) was immobilized on a solid support through biotin/streptavidin interaction, the second block (tri-repeat) was then ligated to the first and after SfaNl digestion a third bloc (tri-repeat) was coupled. The process was repeated using tri- or di-repeat blocks upon obtaining the desired repeat array. The product was then cloned in a classical pAPG10 cloning plasmid for amplification in *E. coli* and sequenced. The repeat array sequences thus obtained were subcloned in a yeast expression TALE vector using type IIS restriction enzymes BsmBl for the receiving plasmid and Bbvl and SfaNl for the inserted repeat sequence. DNA coding for the half TALE-nuclease, containing a TALE derived DNA binding domain fused to the catalytic domain of the Fokl restriction enzyme, was amplified in *E. coli,* recovered by standard miniprep techniques and sequenced to assess the integrity of the insert.

### Activity of GR TALE-nucleases in yeast:

Nuclease activity of the six GR-TALE-nucleases were tested at 37°C and 30°C in our yeast SSA assay previously described (International PCT Applications WO 2004/067736 and in (Epinat, Arnould et al. 2003; Chames, Epinat et al. 2005; Arnould, Chames et al. 2006; Smith, Grizot et al. 2006) on targets containing the two TALE target sequences facing each other on the DNA strand separated by a spacer of 15 bps resulting in SEQ ID NO: 1 to 6. All the yeast target reporter plasmids containing the TALE-nuclease DNA target sequences were constructed as previously described (International PCT Applications WO 2004/067736 and in (Epinat, Arnould et al. 2003; Chames, Epinat et al. 2005; Arnould, Chames et al. 2006; Smith, Grizot et al. 2006). TALE-nuclease cleavage activity levels, in yeast, of individual clones on the targets are presented in table 3.

**Table 3: Cleavage activity of the GR TALE-nucleases in yeast.**

| Values are comprised between 0 and 1. Maximal value is 1. | | | |
|---|---|---|---|
| **Target** | **Half TALE-nuclease transfected** | **yeast gal37°C** | **yeast gal30°C** |
| GRex2 | Grex2-L TALEN | 1 | 1 |
| | Grex2-R TALEN | | |
| GRex3T2 | GRex3T2-L TALEN | 0,92 | 0,87 |
| | GRex3T2-R TALEN | | |
| GRex3T4 | GRex3T4-L TALEN | 0,94 | 0,87 |
| | GRex3T4-R TALEN | | |
| GRex5T1 | GRex5T1-L TALEN | 0,48 | 0,36 |
| | GRex5T1-R TALEN | | |
| GRex5T2 | GRex5T2-L TALEN | 0,97 | 0,91 |
| | GRex5T2-R TALEN | | |
| GRex5T3 | GRex5T3-L TALEN | 1 | 0,98 |
| | GRex5T3-R TALEN | | |

### Activity of GR TALE-nucleases in HEK293 cells:

Each TALE-nuclease construct was subcloned using restriction enzyme digestion in a mammalian expression vector under the control of a pEFlalpha long promoter.

One million HEK293cells were seeded one day prior to transfection. Cells were co-transfected with 2.5 µg of each of two plasmids encoding left and right half of GRex2, GRex3T2, GRex3T4, GRex5T1, GRex5T2 or GRex5T3 TALE-nuclease recognizing the two half targets genomic sequences of interest in the GR gene under the control of EF1alpha promoter using 25µL of lipofectamine (Invitrogen) according to the manufacturer's instructions. As a control, cells were co-transfected with 2.5 µg of each of the two plasmids encoding the left and the right half of TALE-nucleases targeting the T-cell receptor alpha constant chain region (TRAC_T01) target site ((TRAC_T01-L and -R TALE-nuclease (SEQ ID NO: 41 and SEQ ID NO: 42, TRAC_T01 target site (SEQ ID NO: 37)) under the control of EF1alpha promoter. The double strand break generated by TALE-nucleases in GR coding sequence induces non homologous end joining (NHEJ), which is an error-prone mechanism. Activity of TALE-nucleases is measured by the frequency of insertions or deletions at the genomic locus targeted.

2 or 7 days post transfection cells were harvested and locus specific PCRs were performed on genomic DNA extracted using the following primers: 5'-CCATCTCATCCCTGCGTGTCTCCGACTCAG-3' (forward adaptator sequence)- 10N (TAG)- locus specific forward sequence for GR exon 2: 5'-GGTTCATTTAACAAGCTGCC-3' (SEQ ID NO: 31), for GR exon 3: 5'-GCATTCTGACTATGAAGTGA-3' (SEQ ID NO: 32) and for GR exon 5: 5'-TCAGCAGGCCACTACAGGAGTCTCACAAG-3' (SEQ ID NO: 33) and the reverse primer 5'-CCTATCCCCTGTGTGCCTTGGCAGTCTCAG-3' (reverse adaptor sequence)- locus specific reverse sequence for GR exon 2 : S'-AGCCAGTGAGGGTGAAGACG-3' (SEQ ID NO: 34), for GR exon 3 : 5'-GGGCTTTGCATATAATGGAA-3' (SEQ ID NO: 35) and for GR exon 5 : 5'-CTGACTCTCCCCTTCATAGTCCCCAGAAC-3' (SEQ ID NO: 36).

PCR products were sequenced by a 454 sequencing system (454 Life Sciences). Approximately 10,000 sequences were obtained per PCR product and then analyzed for the presence of site-specific insertion or deletion events. Table 4 indicates the percentage of the sequences showing insertions or deletions at the TALE-nuclease target site among the total number of sequences in the sample. In table 4 are listed for GRex2, GRex3T2 and GRex3T4 the results of a representative experiment.

In all cases tested, the % of mutagenesis was similar at day 7 compared to the one of the sample at day 2 post transfection. The nature of the mutagenic events was also analyzed, revealing a majority of deletions in all cases compared to insertions.

**Table 4: Percentage of targeted mutagenesis at endogenous TALE-nuclease Target sites in HEK293 cells.**

| **Target** | % Indels at 2 days with GR TALE-nuclease transfection | % Indels at 7 days with GR TALE-nuclease transfection | % Indels at 2 days with TRAC_T01 TALE-nuclease control transfection |
|---|---|---|---|
| GRex2 | 20.3 | 24.9 | 0.5 |
| GRex3T2 | 9.3 | 9.8 | 0 |
| GRex3T4 | 19 | 18.3 | 0.0 |
| GRex5T1 | 11.2 | NA | 0.7 |
| GRex5T2 | 3.4 | NA | 0 |
| GRex5T3 | 8.3 | NA | 0 |

### Activity of GR TALE-nucleases in primary T lymphocytes:

Each TALE-nuclease construct was subcloned using restriction enzyme digestion in an expression vector under the control of a T7 promoter.

mRNA encoding TALE-nucleases cleaving GR genomic sequences were synthesized from each plasmid carrying the coding sequences downstream from the T7 promoter. T lymphocytes isolated from peripheral blood were activated for 5 days using anti-CD3/CD28 activator beads (Life technologies) and 5 million cells were transfected by electroporation with 10 µg of each of 2 mRNAs encoding both half TALE-nucleases using a CytoLVT-P instrument (BTX-Harvard apparatus). T cells transfected with 10µg of each of the 2 mRNAs encoding both half TALE-nucleases targeting the CD52 gene (CD52_T02-L and -R TALEN (SEQ ID NO: 55 and 56), target sequence CD52_T02 SEQ ID NO: 40) are used as a control.

3 and 7 days after transfection, genomic DNA was isolated from transfected cells and locus specific PCRs were performed using the primers described previously. PCR products were sequenced by a 454 sequencing system (454 Life Sciences). Approximately 10,000 sequences were obtained per PCR product and then analyzed for the presence of site-specific insertion or deletion events; results are in Table 5.

**Table 5: Percentage of targeted mutagenesis at endogenous TALE-nuclease target sites in primary T lymphocytes.**

| **Target** | % Indels at day 3 with GR TALE-nuclease transfection | % Indels at day 7 with GR TALE-nuclease transfection | % Indels at day 3 with CD52 TALE-nuclease control transfection |
|---|---|---|---|
| GRex2 | 26.2 | 30.7 | 0.7 |
| GRex3T2 | 1.09 | 0.86 | 0.02 |
| GRex3T4 | 6.3 | 6.93 | 0 |
| GRex5T1 | 0.04 | 0.035 | 0.05 |
| GRex5T2 | 1.3 | 1.0 | 0.22 |
| GRex5T3 | 17.4 | NA | 0.41 |

### Example 2: TALE-nucleases cleaving the human CD52 gene, the human T-cell receptor alpha constant chain (TRAC) and the human T-cell receptor beta constant chains 1 and 2 (TRBC)

As described in example 1, heterodimeric TALE-nucleases targeting respectively CD52, TRAC and TRBC genes were designed and produced. The targeted genomic sequences consist of two 17-bp long sequences (called half targets) separated by an 11 or 15-bp spacer. Each half-target is recognized by repeats of half TALE-nucleases listed in table 6. The human genome contains two functional T-cell receptor beta chains (TRBC1 and TRBC2). During the development of alpha/beta T lymphocytes, one of these two constant chains is selected in each cell to be spliced to the variable region of TCR-beta and form a functional full length beta chain. The 2 TRBC targets were chosen in sequences conserved between TRBC1 and TRBC2 so that the corresponding TALE-nuclease would cleave both TRBC1 and TRBC2 at the same time.

**Table 6: Description of the CD52, TRAC and TRBC TALE-nucleases and sequences of the TALE-nucleases target sites in the human corresponding genes.**

| **Target** | **Target sequence** | **Repeat sequence** | **Half TALE-nuclease** |
|---|---|---|---|
| TRAC_T01 | TTGTCCCACAGATATCC Agaaccctgaccctg CCGTGTACCAGCTGAGA (SEQ ID NO: 37) | Repeat TRAC_T01-L (SEQ ID NO: 41) | TRAC_T01-L TALEN (SEQ ID NO: 49) |
| | | Repeat TRAC_T01-R (SEQ ID NO: 42) | TRAC _T01-R TALEN (SEQ ID NO: 50) |
| TRBC_T01 | TGTGTTTGAGCCATCAG aagcagagatctccc ACACCCAAAAGGCCACA (SEQ ID NO: 38) | Repeat TRBC_T01-L (SEQ ID NO: 43) | TRBC_T01-L TALEN (SEQ ID NO: 51) |
| | | Repeat TRBC_T01-R (SEQ ID NO: 44) | TRBC_T01-R TALEN (SEQ ID NO: 52) |
| TRBC_T02 | TTCCCACCCGAGGTCGC tgtgtttgagccatca GAAGCAGAGATCTCCCA (SEQ ID NO: 39) | Repeat TRBC_T02-L (SEQ ID NO: 45) | TRBC_T02-L TALEN (SEQ ID NO: 53) |
| | | Repeat TRBC_T02-R (SEQ ID NO: 46) | TRBC_T02-R TALEN (SEQ ID NO: 54) |
| CD52_T02 | TTCCTCCTACTCACCAT cagcctcctggttat GGTACAGGTAAGAGCAA (SEQ ID NO: 40) | Repeat CD52_T02-L (SEQ ID NO: 47) | CD52_T02-L TALEN (SEQ ID NO: 55) |
| | | Repeat CD52_T02-R (SEQ ID NO: 48) | CD52_T02-R TALEN (SEQ ID NO: 56) |

Other target sequences in TRAC and CD52 genes have been designed, which are displayed in Table 7.

**Table 7: Additional target sequences for TRAC and CD52 TALE-nucleases.**

| **Target** | **Target sequence** |
|---|---|
| TRAC_T02 | TTTAGAAAGTTCCTGTG atgtcaagctggtcg AGAAAAGCTTTGAAACA (SEQ ID NO: 57) |
| TRAC_T03 | TCCAGTGACAAGTCTGT ctgcctattcaccga TTTTGATTCTCAAACAA (SEQ ID NO: 58) |
| TRAC_T04 | TATATCACAGACAAAAC tgtgctagacatgag GTCTATGGACTTCAAGA (SEQ ID NO: 59) |
| TRAC_T05 | TGAGGTCTATGGACTTC aagagcaacagtgct GTGGCCTGGAGCAACAA (SEQ ID NO: 60) |
| CD52_T01 | TTCCTCTTCCTCCTAC caccatcagcctcct TTACCTGTACCATAAC (SEQ ID NO: 61) |
| CD52_T04 | TTCCTCCTACTCACCA cagcctcctgg TCTTACCTGTACCATA (SEQ ID NO: 62) |
| CD52_T05 | TCCTACTCACCATCAG ctcctggttat TTGCTCTTACCTGTAC (SEQ ID NO: 63) |
| CD52_T06 | TTATCCCACTTCTCCT ctacagatacaaact TTTTGTCCTGAGAGTC (SEQ ID NO: 64) |
| CD52_T07 | TGGACTCTCAGGACAA acgacaccagccaaa TGCTGAGGGGCTGCTG (SEQ ID NO: 65) |

### Activity of CD52-TALE-nuclease, TRAC-TALE-nuclease and TRBC-TALE-nuclease in HEK293 cells

Each TALE-nuclease construct was subcloned using restriction enzyme digestion in a mammalian expression vector under the control of pEF1alpha long promoter. One million HEK293 cells were seeded one day prior to transfection. Cells were co-transfected with 2.5 µg of each of the two plasmids encoding the TALE-nucleases recognizing the two half targets in the genomic sequence of interest in the CD52 gene, T-cell receptor alpha constant chain region (TRAC) or T-cell receptor beta constant chain region (TRBC) under the control of the EF1-alpha promoter or 5 µg of a control pUC vector (pCLS0003) using 25 µl of lipofectamine (Invitrogen) according to the manufacturer's instructions. The double stranded cleavage generated by TALE-nucleases in CD52 or TRAC coding sequences is repaired in live cells by non homologous end joining (NHEJ), which is an error-prone mechanism. Activity of TALE-nucleases in live cells is measured by the frequency of insertions or deletions at the genomic locus targeted. 48 hours after transfection, genomic DNA was isolated from transfected cells and locus specific PCRs were performed using the following primers: 5'-CCATCTCATCCCTGCGTGTCTCCGACTCAG (forward adaptor sequence)-10N (TAG)- locus specific forward sequence for CD52: 5'-CAGATCTGCAGAAAGGAAGC-3' (SEQ ID NO: 66), for TRAC: 5'-ATCACTGGCATCTGGACTCCA-3' (SEQ ID NO: 67), for TRBC1: 5'-AGAGCCCCTACCAGAACCAGAC-3' (SEQ ID NO: 68), or for TRBC2: 5'-GGACCTAGTAACATAATTGTGC-3' (SEQ ID NO: 69), and the reverse primer 5'-CCTATCCCCTGTGTGCCTTGGCAGTCTCAG (reverse adaptor sequence)- endogenous locus specific reverse sequence for CD52: 5'-CCTGTTGGAGTCCATCTGCTG-3' (SEQ ID NO: 70), for TRAC: 5'-CCTCATGTCTAGCACAGTTT-3' (SEQ ID NO: 71), for TRBC1 and TRBC2: 5'-ACCAGCTCAGCTCCACGTGGT-3' (SEQ ID NO: 72). PCR products were sequenced by a 454 sequencing system (454 Life Sciences). Approximately 10,000 sequences were obtained per PCR product and then analyzed for the presence of site-specific insertion or deletion events; results are in
8.

**Table 8: Percentages of indels for TALE-nuclease targeting CD52_T02, TRAC_T01, TRBC_T01 and TRBC_T02 targets.**

| Target | % Indels with TALE-nuclease transfection | % Indels with pUC control transfection |
|---|---|---|
| CD52_T02 | 28.0 | 0.9 |
| TRAC_T01 | 41.9 | 0.3 |
| TRBC_T01 in constant chain 1 | 3.81 | 0 |
| TRBC_T01 in constant chain 2 | 2.59 | 0 |
| TRBC_T02 in constant chain 1 | 14.7 | 0 |
| TRBC_T02 in constant chain 1 | 5.99 | 0 |

### Activity of CD52-TALE-nuclease, TRBC-TALE-nuclease and TRAC-TALE-nuclease in primary T lymphocytes

Each TALE-nuclease construct was subcloned using restriction enzyme digestion in a mammalian expression vector under the control of the T7 promoter.

mRNA encoding TALE-nuclease cleaving CD52 TRAC and TRBC genomic sequence were synthesized from plasmid carrying the coding sequences downstream from the T7 promoter. T lymphocytes isolated from peripheral blood were activated for 5 days using anti-CD3/CD28 activator beads (Life technologies) and 5 million cells were then transfected by electroporation with 10 µg of each of 2 mRNAs encoding both half TALE-nuclease (or non coding RNA as controls) using a CytoLVT-P instrument. As a consequence of the insertions and deletions induced by NHEJ, the coding sequence for CD52 and/or TRAC will be out of frame in a fraction of the cells resulting in non-functional genes. 5 days after electroporation, cells were labeled with fluorochrome-conjugated anti-CD52 or anti-TCR antibody by flow cytometry for the presence of CD52 or TCR at their cell surface. Since all T lymphocytes expanded from peripheral blood normally express CD52 and TCR, the proportion of CD52-negative or TCR-negative cells is a direct measure of TALE-nuclease activity. In table 9 are listed the results of a representative experiment. The table 9 shows the results of a representative experiment testing the efficiency of TRBC TALE-nucleases.

**Table 9: Percentages of CD52- negative, TCR-negative and CD52/TCR-double negative T lymphocytes after transfection of corresponding TALE-nuclease-expressing polynucleotides.**

| ARN transfected | % CD52-negative cells | % TCR-negative cells | % CD52/TCR double negative cells |
|---|---|---|---|
| non coding RNA | 1,21 | 1,531 | 0,111 |
| TALEN CD52_T02 | 49,2 | 1,6 | 0,78 |
| TALEN TRAC_T01 | 2,16 | 44,8 | 0,97 |
| TALEN CD52_T02 + TALEN TRAC_T01 | 29,3 | 39,6 | 15,5 |

**Table 10: Percentages of TCR-negative T lymphocytes after transfection of TRBC TALE-nuclease-expressing polynucleotides.**

| ARN transfected | % TCR-negative cells |
|---|---|
| no RNA | 1,22 |
| TALEN TRBC_T01 | 6,52 |
| TALEN TRBC_T02 | 23,5 |

### Functional analysis of T cells with targeted CD52 gene

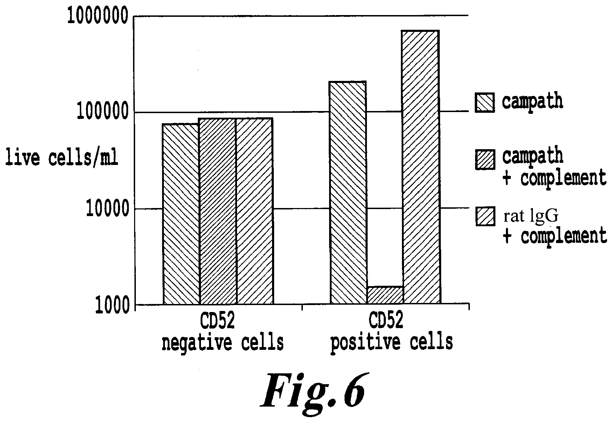

The goal of CD52 gene inactivation is to render T lymphocytes resistant to anti-CD52 antibody mediated immunosuppression. As described in the previous paragraph, T lymphocytes were transfected with mRNA encoding TALE-nuclease cleaving CD52. 7 days after transfection, cells were treated with 50µg/ml anti-CD52 monoclonal antibody (or rat IgG as control) with or without 30% rabbit complement (Cedarlane). After 2 hours of incubation at 37°C, the cells were labeled with a fluorochrome-conjugated anti-CD52 antibody together with a fluorescent viability dye (eBioscience) and analyzed by flow cytometry to measure the frequency of CD52-positive and CD52-negative cells among live cells. Figure 6 shows the result of a representative experiment, demonstrating that CD52-negative cells are completely resistant to complement-mediated anti-CD52 antibody toxicity.

### Functional analysis of T cells with targeted TRAC gene

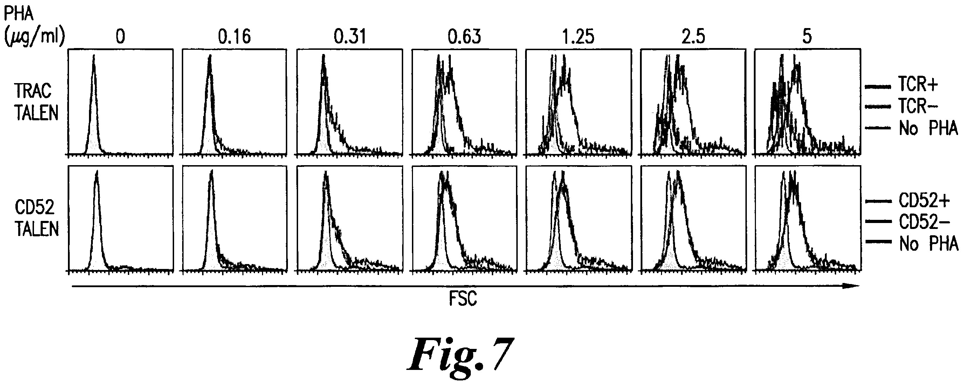

The goal of TRAC gene inactivation is to render T lymphocytes unresponsive to T-cell receptor stimulation. As described in the previous paragraph, T lymphocytes were transfected with mRNA encoding TALE-nuclease cleaving TRAC or CD52. 16 days after transfection, cells were treated with up to 5µg/ml of phytohemagglutinin (PHA, Sigma-Aldrich), a T-cell mitogen acting through the T cell receptor. Cells with a functional T-cell receptor should increase in size following PHA treatment. After three days of incubation, cells were labeled with a fluorochrome-conjugated anti-CD52 or anti-TCR antibody and analyzed by flow cytometry to compare the cell size distribution between TCR-positive and TCR-negative cells, or between CD52-positive and CD52-negative cells. Figure 7 shows that TCR-positive cells significantly increase in size after PHA treatment whereas TCR-negative cells have the same size as untreated cells indicating that TRAC inactivation rendered them unresponsive to TCR-signaling. By contrast, CD52-positive and CD52-negative increase in size to same extent.

### Functional analysis of T cells with targeted CD52 and TRAC genes

To verify that genome engineering did not affect the ability of T cells to present anti-tumor activity when provided with a chimeric antigen receptor (CAR), we transfected T cells that had been targeted with CD52-TALE-nuclease and TRAC-TALE-nuclease with 10µg of RNA encoding an anti-CD19 CAR (SEQ ID NO: 73). 24 hours later, T cells were incubated for 4 hours with CD19 expressing Daudi cells. The cell surface upregulation of CD107a, a marker of cytotoxic granule release by T lymphocytes (called degranulation) was measured by flow cytometry analysis (Betts, Brenchley et al. 2003). The results are included in Figure 8 and show that CD52-negative/TCRαβ-negative cells and CD52-positive/TCRαβ-positive have the same ability to degranulate in response to PMA /ionomycin (positive control) or CD19+ Daudi cells. CD107 upregulation is dependent on the presence of a CD19+. These data suggest that genome engineering has no negative impact on the ability of T cells to mount a controlled anti-tumor response.

### Genomic safety of CD52-TALE-nuclease and TRAC-TALE-nuclease in primary T lymphocytes

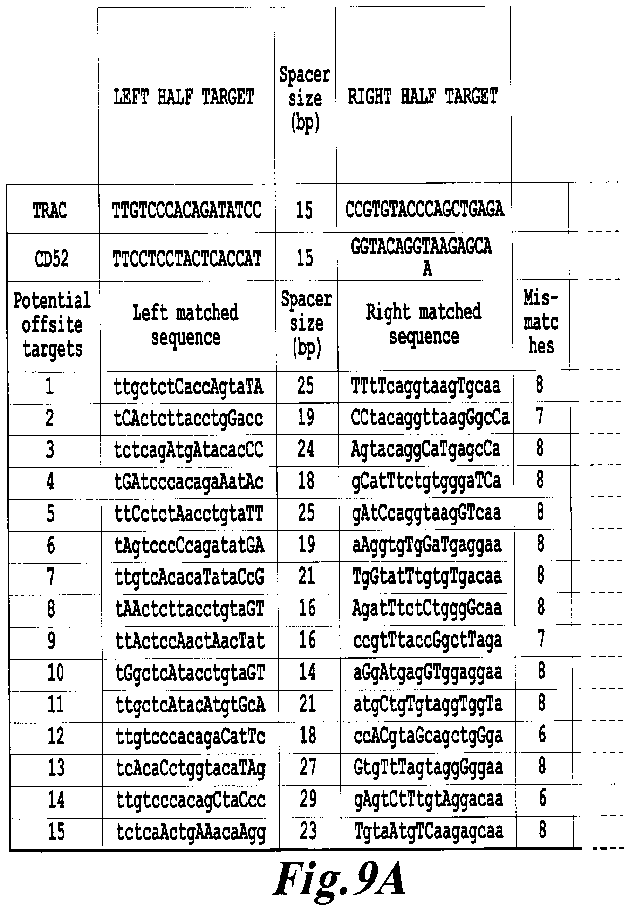

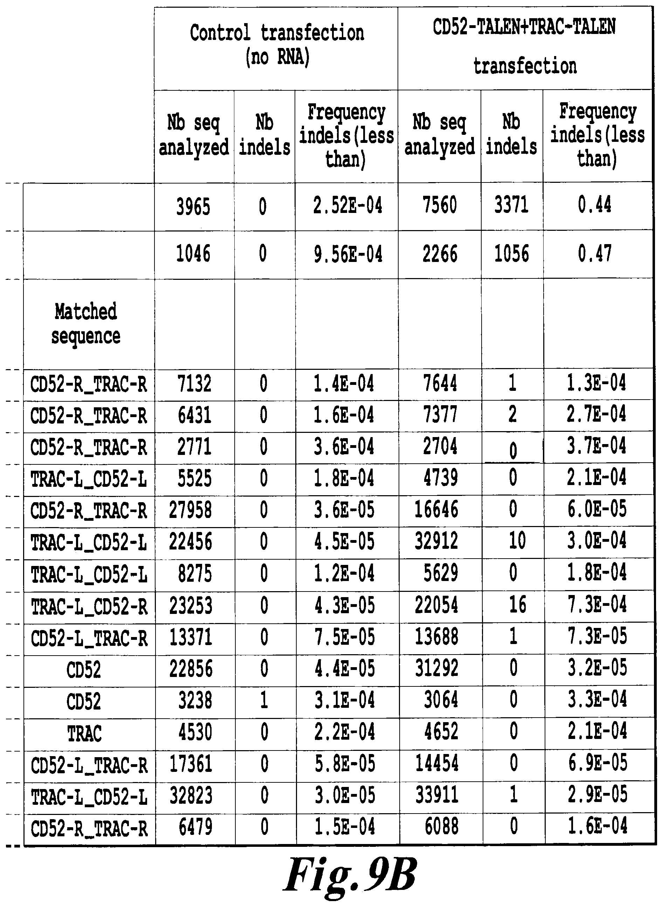

As our constructs include nuclease subunits, an important question is whether multiple TALE-nuclease transfection can lead to genotoxicity and off-target cleavage at 'close match' target sequences or by mispairing of half-TALE-nucleases. To estimate the impact of TRAC-TALE-nuclease and CD52-TALE-nuclease on the integrity of the cellular genomes, we listed sequences in the human genome that presented the potential for off-site cleavage. To generate this list, we identified all the sequences in the genome with up to 4 substitutions compared to the original half targets and then identified the pairs of potential half targets in a head to head orientation with a spacer of 9 to 30 bp from each other. This analysis included sites potentially targeted by homodimers of one half-TALE-nuclease molecule or heterodimers formed by one CD52 half TALE-nuclease and one TRAC half-TALE-nuclease. We scored the potential off-site targets based on the specificity data taking into account the cost of individual substitutions and the position of the substitutions (where mismatches are better tolerated for bases at the 3' end of the half target). We obtained 173 unique sequences with a score reflecting an estimation of the likelihood of cleavage. We selected the 15 top scores and analyzed by deep sequencing the frequency of mutations found at these loci in T cells simultaneously transfected with CD52 and TRAC TALE-nuclease and purified by magnetic separation as CD52-negative, TCRαβ-negative. Results are in Figure 9. The highest frequency of insertion/deletion is 7×10⁻⁴. These results make the putative offsite target at least 600 times less likely to be mutated than the intended targets. The TALE-nuclease reagents used in this study therefore appear extremely specific.

### Example 3: TALE-nucleases cleaving the human CTLA4 gene and the human PDCD1 gene.

As described in example 1, heterodimeric TALE-nucleases targeting respectively PDCD1 and CTLA4 genes were designed and produced. The targeted genomic sequences consist of two 17-bp long sequences (called half targets) separated by an 11 or 15-bp spacer. Each half-target is recognized by repeats of half TALE-nucleases listed in table 11.

**Table 11: Description of the CTLA4 and PDCD1 TALE-nucleases and sequences of the TALE-nucleases target sites in the human corresponding genes.**

| **Target** | **Target sequence** | **Repeat sequence** | **Half TALE-nuclease** |
|---|---|---|---|
| CTLA4_T01 | TGGCCCTGCACTCTCCT gttttttcttctctt CATCCCTGTCTTCTGCA (SEQ ID NO: 74) | Repeat CTLA4_T01-L (SEQ ID NO: 79) | CTLA4_T01-L TALEN (SEQ ID NO: 89) |
| | | Repeat CTLA4_T01-R (SEQ ID NO: 80) | CTLA4_T01-R TALEN (SEQ ID NO: 90) |
| CTLA4_T03 | TTTTCCATGCTAGCAAT gcacgtggcccagcc TGCTGTGGTACTGGCCA (SEQ ID NO : 75) | Repeat CTLA4_T03-L (SEQ ID NO: 81) | CTLA4_T03-L TALEN (SEQ ID NO: 91) |
| | | Repeat CTLA4_T03-R (SEQ ID NO: 82) | CTLA4_T03-R TALEN (SEQ ID NO: 92) |
| CTLA4_T04 | TCCATGCTAGCAATGCA cgtggcccagcctgc TGTGGTACTGGCCAGCA (SEQ ID NO: 76) | Repeat CTLA4_T04-L (SEQ ID NO: 84) | CTLA4_T04-L TALEN (SEQ ID NO: 93) |
| | | Repeat CTLA4_T04-R (SEQ ID NO: 85) | CTLA4_T04-R TALEN (SEQ ID NO: 94) |
| PDCD1_T01 | TTCTCCCCAGCCCTGCT cgtggtgaccgaagg GGACAACGCCACCTTCA (SEQ ID NO : 77) | Repeat PDCD1_T01-L (SEQ ID NO: 86) | PDCD1_T01-L TALEN (SEQ ID NO: 95) |
| | | Repeat PDCD1_T01-R (SEQ ID NO: 87) | PDCD1_T01-R TALEN (SEQ ID NO: 96) |
| PDCD1_T03 | TACCTCTGTGGGGCCAT ctccctggcccccaa GGCGCAGATCAAAGAGA (SEQ ID NO : 78) | Repeat PDCD1_T03-L (SEQ ID NO: 88) | PDCD1_T03-L TALEN (SEQ ID NO: 97) |
| | | Repeat PDCD1_T03-R (SEQ ID NO: 89) | PDCD1_T03-R TALEN (SEQ ID NO: 98 |

### Activity of CTLA4-TALE-nuclease and PDCD1-TALE-nuclease in HEK293 cells

Each TALE-nuclease construct was subcloned using restriction enzyme digestion in a mammalian expression vector under the control of the pEF1alpha long promoter. One million HEK293 cells were seeded one day prior to transfection. Cells were co-transfected with 2.5 µg of each of two plasmids encoding the TALE-nucleases recognizing the two half targets in the genomic sequence of interest in the PDCD1 and CTLA-4 gene under the control of the EF1-alpha promoter or 5 µg of a control pUC vector (pCLS0003) using 25 µl of lipofectamine (Invitrogen) according to the manufacturer's instructions. The double stranded cleavage generated by TALE-nucleases in PDCD1 or CTLA-4 coding sequences is repaired in live cells by non homologous end joining (NHEJ), which is an error-prone mechanism. Activity of TALE-nucleases in live cells is measured by the frequency of insertions or deletions at the genomic locus targeted. 48 hours after transfection, genomic DNA was isolated from transfected cells and locus specific PCRs were performed using the following primers: 5'-CCATCTCATCCCTGCGTGTCTCCGACTCAG (forward adaptor sequence)-10N (TAG)- locus specific forward sequence for CTLA4_T01: 5'-CTCTACTTCCTGAAGACCTG-3' (SEQ ID NO: 99) , for CTLA4_T03/T04: 5'-ACAGTTGAGAGATGGAGGGG-3' (SEQ ID NO: 100), for PDCD1_T01: 5'-CCACAGAGGTAGGTGCCGC-3' (SEQ ID NO: 101) or for PDCD1_T03: 5'-GACAGAGATGCCGGTCACCA-3' (SEQ ID NO: 102) and the reverse primer 5'-CCTATCCCCTGTGTGCCTTGGCAGTCTCAG (reverse adaptor sequence)- endogenous locus specific reverse sequence for CTLA4_T01: 5'-TGGAATACAGAGCCAGCCAA-3' (SEQ ID NO: 103), for CTLA4_T03/T04: 5'-GGTGCCCGTGCAGATGGAAT-3' (SEQ ID NO: 104), for PDCD1_T01: 5'-GGCTCTGCAGTGGAGGCCAG-3' (SEQ ID NO: 105) or for PDCD1_T03: 5'-GGACAACGCCACCTTCACCT-3' (SEQ ID NO: 106).

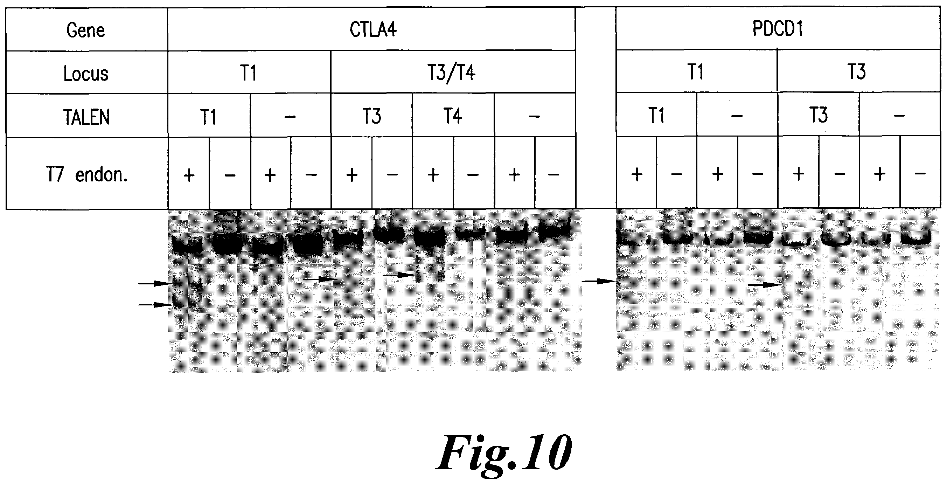

PCR products were analyzed by T7-endonuclease assay: briefly, after denaturation and reannealing of the PCR product, T7 endonuclease will specifically digest mismatched DNA composed of wild type and mutated strands. The digestion product is then resolved by polyacrylamide gel electrophoresis. The presence of a digested product is indicative of mutated sequences induced by TALE-nuclease activity. Results are displayed in Figure 10 where arrows point to the digested PCR products. They demonstrate that PDCD1_T1, PDCD1_T3, CTLA4_T1, CTLA4_T3 and CTLA4_T4 TALE-nucleases all exhibit mutagenic nuclease activity at their target sites.

### CTLA4 inactivation in primary T cells:

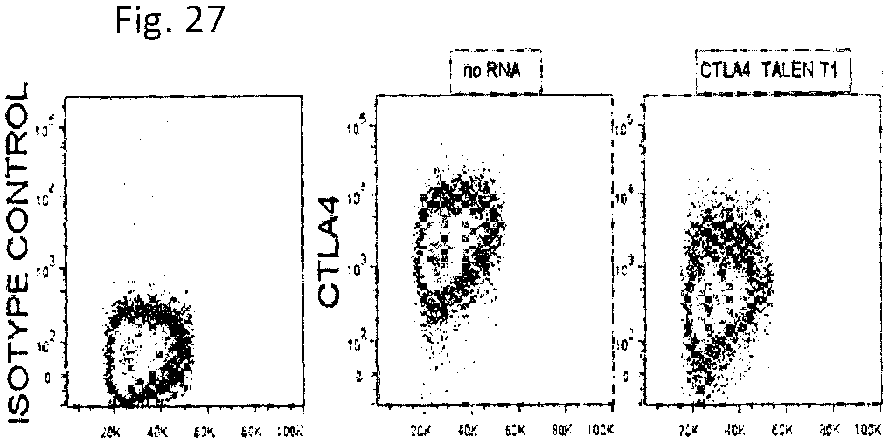

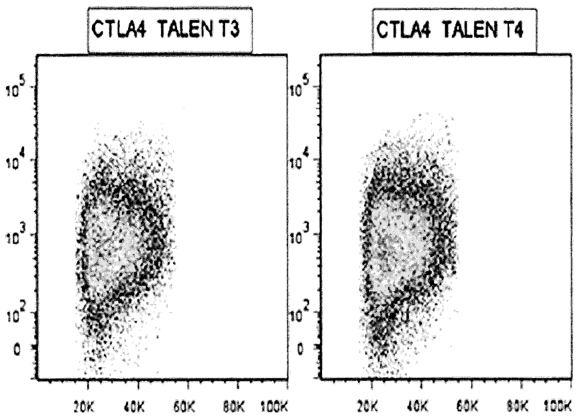

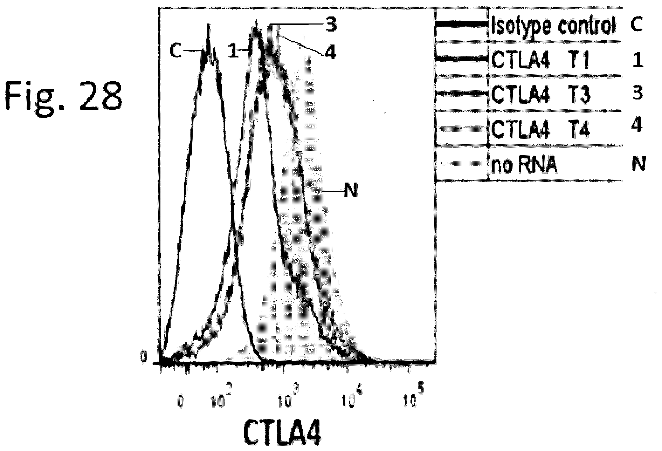

Human primary T cells were activated with CD3/28 beads. Five days later, 5×10⁶ cells were electroporated with 20 µg of RNA encoding one of three TALEN^{™} (T1, T2 and T3) designed with respect to CTLA4 gene or without RNA as control. Three days post electroporation, CTLA4 expression was measured by intracellular staining using fluorescent antibody and flow cytometry analysis (Figures 27 and 28).

All three TALEN^{™} induced downregulation of CTLA4 expression in a manner correlated with their efficiency in HEK293 cell lines (T1 was more efficient than T3 and T4).

Deep sequencing analysis of genomic DNA isolated from transfected cells using 454 technology (Roche) revealed than 96 % of CTLA4 alleles were mutated in TALEN T1-treated cells compared to 0.1% in the control sample without TALEN.

### PD1 inactivation in primary T cells:

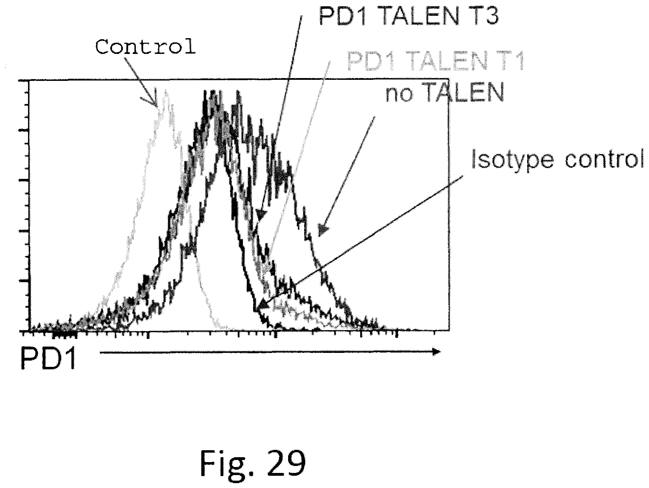

Human primary T cells were activated with CD3/28 beads. Five days later 5×10⁶ cells were electroporated with 20 µg of RNA encoding one of two TALENs specific for human PD1 gene or without RNA as control. Ten days later, cells were re-activated and 3 days post re-activation, PD1 expression was measured by surface staining using fluorescent antibody and flow cytometry analysis (see Figure 29).

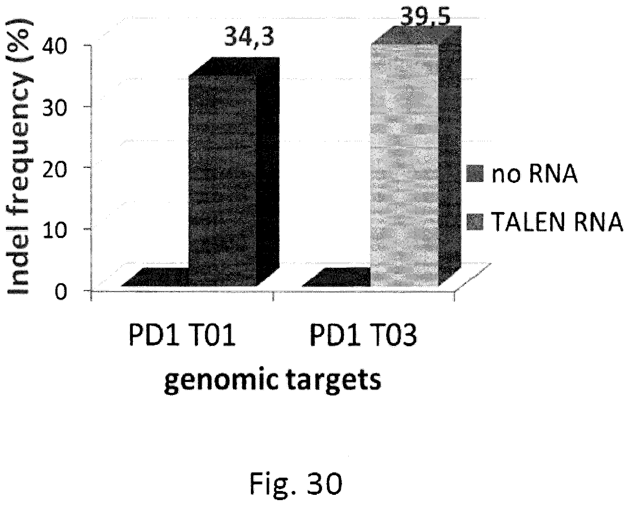

Both TALENs induced significant downregulation of PD1 expression. Deep sequencing analysis of genomic DNA isolated from cells transfected with TALEN T1 and TALEN T03 respectively using 454 technology (Roche) revealed than 34 % and 39% of PD1 alleles were mutated respectively (results shown in Figure 30).

### Enhanced anti-tumor activity PD1-TALEN treated cells:

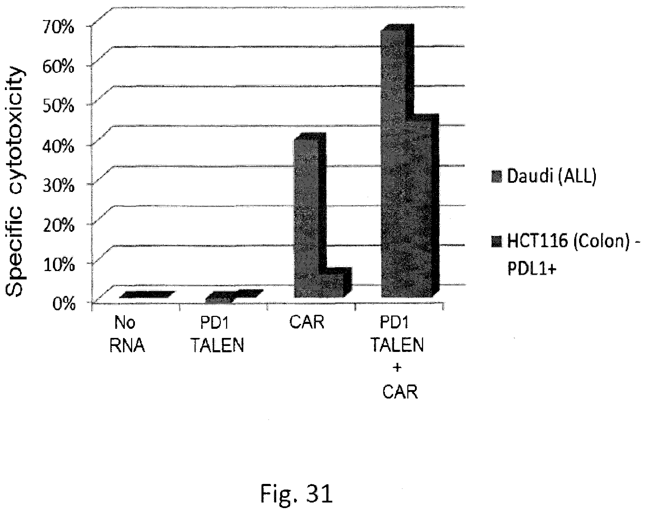

Human primary T cells were activated with CD3/28 beads. Five days later 5×10⁶ cells were electroporated with 20 µg of RNA encoding a TALEN specific for human PD1 gene or without RNA as control. A week later, cells were electroporated with mRNA encoding a chimeric antigen receptor specific for human CD19 or no RNA as negative control. The next day, their antitumor activity was measured in cellular cytotoxicity assay using CD19+ Daudi cells (vs. K562 as control) or HCT116 cells, which express PD1ligand 1 (PDL1) transduced with CD19 expression vector (vs. parental HCT116 cells as control). Cytotoxic activity was determined by comparing viability of target cells and control cells. Results are shown in the diagrams of Figure 31. PD1 TALEN transfection restored cytotoxic activity against PDL1-expressing HCT116 cells and improved cytotoxic activity against Daudi cells.

### Example 4: pTalpha permits CD3 surface expression in inactivated TCR alpha T lymphocytes:

### Description of the different preTalpha versions:

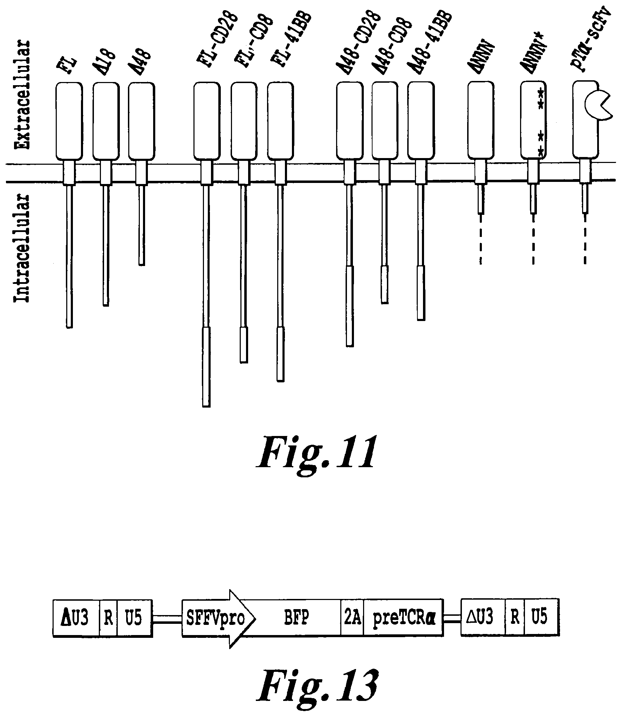

The human pTalpha gene encodes a transmembrane glycoprotein comprising an extracellular Ig-like domain, a hydrophobic transmembrane domain and a large C-terminal intracytoplasmic tail. Different versions derived from human pTalpha glycoprotein have been designed and are described in Table 12 and represented in figure 11.

**Table 12: Description of a subset of pTalpha constructs**

| **PTalpha versions** | **Description** | **SEQ ID** |
|---|---|---|
| pTalpha-FL | Full-length of human pTalpha glycoprotein | 107 |
| pTalpha-Δ18 | Truncated Human pTalpha glycoprotein lacking 18 residues from the C-terminus. | 108 |
| pTalpha-Δ48 | Truncated Human pTalpha glycoprotein lacking 48 residues from the C-terminus. | 109 |
| pTalpha-Δ62 | Truncated Human pTalpha glycoprotein lacking 62 residues from the C-terminus. | 110 |
| pTalpha-Δ78 | Truncated Human pTalpha glycoprotein lacking 78 residues from the C-terminus. | 111 |
| pTalpha-Δ92 | Truncated Human pTalpha glycoprotein lacking 92 residues from the C-terminus. | 112 |
| pTalpha-Δ110 | Truncated Human pTalpha glycoprotein lacking 110 residues from the C-terminus. | 113 |
| pTalpha-Δ114 | Truncated Human pTalpha glycoprotein lacking 114 residues from the C-terminus. | 114 |
| pTalpha-FL-CD28 | Full-length of human pTalpha glycoprotein fused in C-terminus with CD28 activation domain. | 115 |
| pTalpha-FL-CD8 | Full-length of human pTalpha glycoprotein fused in C-terminus with CD8 activation domain. | 116 |
| pTalpha-FL-4-1BB | Full-length of human pTalpha glycoprotein fused in C-terminus with 4-1BB activation domain.. | 117 |
| pTalpha-Δ48-CD28 | pTalpha-Δ48 glycoprotein fused in C-terminus with CD28 activation domain. | 118 |
| pTalpha -Δ48-CD8 | pTalpha-Δ48 glycoprotein fused in C-terminus with CD8 activation domain. | 119 |
| pTalpha -Δ48-41BB | pTalpha-Δ48 glycoprotein fused in C-terminus with 4-1BB activation domain. | 120 |
| pTalpha-Δ114/TCRα.IC | pTalpha-Δ114 glycoprotein fused in C-terminus with the intracellular domain of TCRalpha | 121 |
| pTalpha-EC/TCRα.TM.IC | pTalpha extracellular domain fused in C-terminus with the transmembrane and intracellular domain of TCRalpha. | 122 |
| pTalpha-Δ48-1xM UT | pTalpha-Δ48 glycoprotein with mutated residue W46R. | 123 |
| preTalpha-Δ48-4xM UT | pTalpha-Δ48 glycoprotein with mutated residues D22A, K24A, R102A, R117A | 124 |

The different preTalpha constructs tested include:
1) **pTalpha deletion mutants:** Different deletions were generated in the intracellular cytoplasmic tail of the human pTalpha protein (which comprises 114 amino acids) (SEQ ID NO: 107). The constructs tested include the full length version of the protein (FL) and mutants in which 18, 48, 62, 78, 92, 110 and 114 amino acids were deleted from the C-terminus of the protein (SEQ ID NO: 108 to SEQ ID NO: 114).
2) **pTalpha mutants containing intracellular activation domains:** The FL and Δ48 variants where fused to the CD8, CD28 or 41BB intracellular activation domains at their C-terminus (SEQ ID NO: 115 to SEQ ID NO: 120).
3) **pTalpha/TCRα chimeric mutants:** In one of the constructs, the TCRα intracellular domain (IC) was fused to a tail-less version (Δ114) of pTalpha (SEQ ID NO: 121). A second construct was also generated in which the pTalpha extracellular domain was fused to the transmembrane (TM) and the IC domains from TCRα (SEQ ID NO: 122).
4) **pTalpha dimerization mutants:** Some mutations have been described in the literature as being capable to alter the oligomerisation/dimerisation ability of the preTCR complex. These mutants are proposed to allow preTCR expression at the cell surface, without inducing the constitutive signaling (supposed to be induced upon preTCR oligomerization). The mutations have been introduced in the pTalphaΔ48 variant and are:
   - 1xMUT: W46R (SEQ ID NO: 123)
   - 4x MUT: D22A, K24A, R102A, R117A (SEQ ID NO: 124)

### Activity of different preTalpha constructs in TRAC inactivated Jurkat cells:

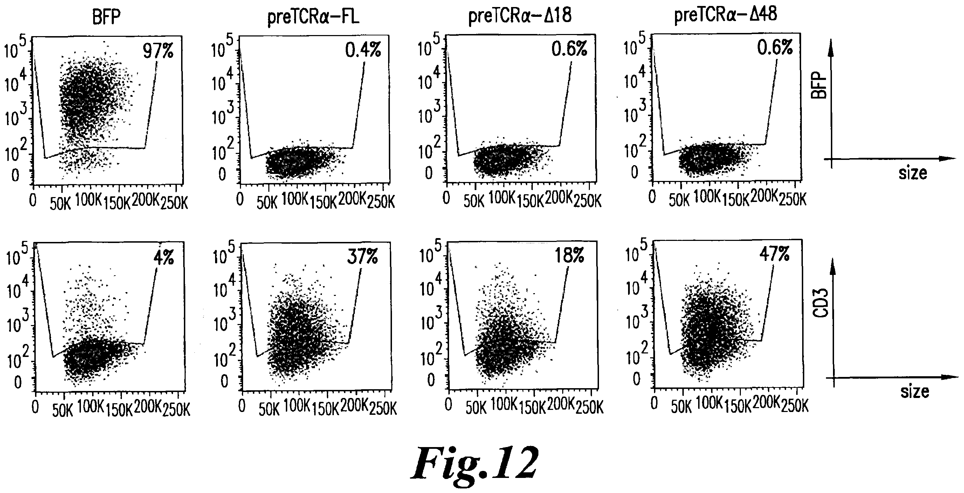

In order to screen different pTalpha variants for their ability to restore CD3 surface expression in TCRalpha inactivated cells, a cell line was generated in which the TCRalpha gene was disrupted using TALEN targeting TRAC. Jurkat cells (a T-cell leukemia cell line) were transfected with plasmids coding for the TALEN cleaving TRAC using CytoPulse electroporation, and the KO cells (TCR_{α/β}^{NEG}; CD3^{NEG}) where then purified by negative selection using CD3 magnetic beads. The KO population (JKT_KOx3 cells) was amplified and used for screening of the different pTalpha variants. Screening was performed by transfection of one million of JKT_KOx3 cells with 15 µg of plasmid coding the different pTalpha variants under control of the EF1α promoter, followed by analysis by flow cytometry of CD3 cell surface expression 48h after transfection. Figure 12 is a representative example of the transfection efficiencies (% of BFP+ cells) and activity of the FL, Δ18 and Δ48 pTalpha constructs in JKT_KOx3 cells, based on the % of CD3+ cells, determined by flow cytometry. The results from the different constructs are grouped in Table 13.

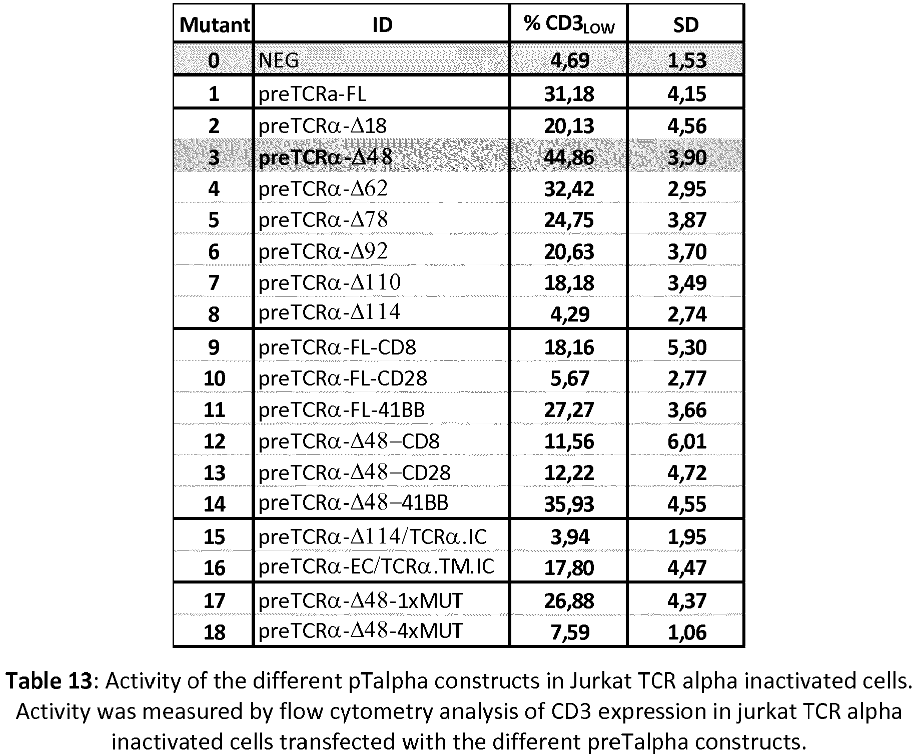

### Activity of pTalpha-FL and pTalpha-Δ48 in TCR alpha inactivated primary T lymphocytes:

In order to test the ability of pTalpha-FL and pTalpha-Δ48 versions to induce CD3 surface expression in TCR alpha inactivated T lymphocytes, pTalpha-FL and pTalpha-Δ48 coding sequences were cloned into a self-inactivating pLV-SFFV-BFP-2A-PCTRA lentiviral vector that codes for Blue Fluorescent protein (BFP) under the SFFV promoter followed by the self-cleaving T2A peptide (figure 13).

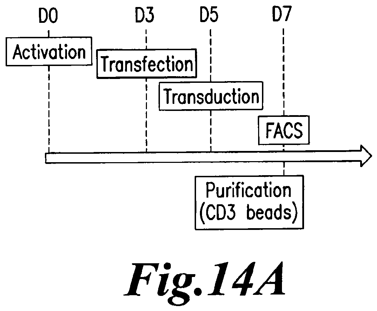

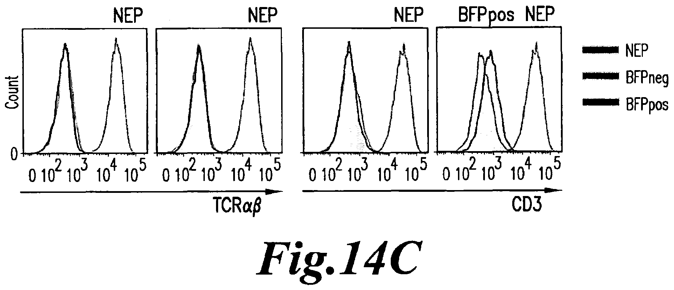

T lymphocytes isolated from peripheral blood were activated for 72 hours using anti-CD3/CD28 activator beads (Life technologies) and 4.5 million cells were transfected by electroporation with 10 µg mRNA encoding the TALE-nuclease targeting TCR alpha constant chain region (TRAC) using a CytoLVT-S instrument (BTX-Harvard Harbour). Two days after electroporation, T cells were transduced with either the LV-SFFV-BFP-2A-pTalpha-Δ48 or LV-SFFV-BFP-2A-control lentiviral vectors. CD3 negative and CD3low T cells were then purified using anti-CD3 magnetic beads (Miltenyi Biotech). This experimental protocol is represented in Figure 14A.

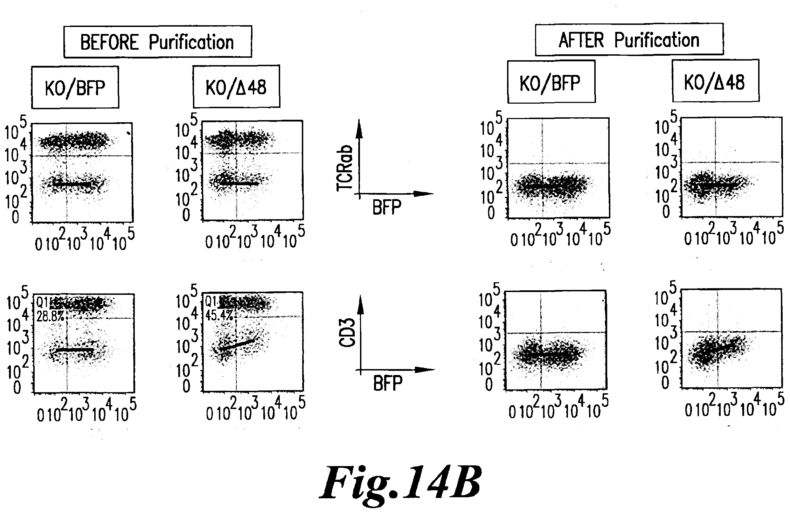

Figure 14B represents flow cytometry analysis of TCRalpha/beta, CD3 cell surface expression, and BFP expression on TCRalpha inactivated T cells (KO) transduced with either BFP-2A-pTalphaΔ48 (KO/Δ48) or control BFP lentiviral vector (KO/BFP) before and after purification with CD3 beads. TCRalpha inactivated cells transduced with the BFP-T2A-pTalpha-Δ48 vector (BFP+ cells) show higher levels of CD3 compared to non transduced cells (BFP- cells). No differences are observed among cells transduced with the control BFP vector. These results indicate that pTalpha mediates restoration of CD3 expression at the cell surface of TCRalpha inactivated cells. In contrast, TCRalpha/beta staining remains, as expected, unchanged in cells transduced or not with the pTalpha-Δ48 expressing vector.

### pTalpha-mediated CD3 expression supports activation of TCR-deficient T-cells:

To determine the capacity of pTalpha to transduce cell activation signals, expression of early and later activation markers was analyzed on TCR alpha inactivated T cells transduced with pTalpha-Δ48 and pTalpha-Δ48.41BB. TCR alpha inactivated T cells transduced with pTalpha-Δ48 and pTalpha-Δ48.41BB were generated from primary human T-cells as described in previous section and in Figure 14A.

To detect signaling via CD3, cells were re-activated using anti-CD3/CD28-coated beads 3 days after purification of TCR alpha inactivated T cells with CD3 beads (figure 14A). Cells were stained with fluorochrome-conjugated anti-CD69 (early activation marker) and anti-CD25 (late activation marker), 24 and 48 hours after re-activation respectively and analyzed by flow cytometry (Figure 15A-B). As represented in figure 15A-B, TCR alpha inactivated cells expressing pTalpha-Δ48 (KO/pTα-Δ48) or pTalpha-Δ48.41BB (KO/pTα-Δ48.BB) show upregulation of the activation markers, to levels similar to those observed in TCRalpha/beta expressing cells (NEP: non electroporated cells).

Another indicator of T cell activation is an increase in cell size which is sometimes referred to as "blasting". The capacity of the preTCR complexes to induce "blasting" was measured by flow cytometry analysis of the cell size 72 hours after re-activation using anti-CD3/CD28- beads (Figure 15C). Stimulation with anti-CD3/CD28 beads induced comparable increases in cell size in cells expressing TCRalpha/beta complexes vs. cells expressing pTalpha-Δ48 or pTalpha-Δ48.41BB. Taken together, these results suggest that preTCR complexes are competent to transduce signals that efficiently couple to the mechanisms mediating activation marker upregulation.

### pTalpha mediated CD3 expression supports expansion of TCR-deficient primary T-cells using stimulatory anti-CD3/CD28 antibodies

To evaluate the capacity of preTCR complexes to support long term cell proliferation, proliferation of cells generated as previously described was measured. Ten days after the initial activation, cells were maintained in IL2 (non-Re-act) or in IL2 with anti-CD3/CD28 beads (Re-act). For each condition, cells were counted and analyzed by flow cytometry at the different time points to estimate the number of BFP+ cells. The growth of TCRalpha inactivated cells (KO) transduced with BFP or BFP-T2A-preTCRα-Δ48 vectors was compared, and the fold induction of these cells was estimated with respect to the value obtained at day 2 post re-activation. Figure 16 shows the results obtained with two independent donors. In both cases, TCRalpha inactivated cells expressing pTalpha-Δ48 displayed greater expansion than TCR alpha inactivated cells expressing only the BFP control vector. For the second donor, TCRalpha inactivated cells expressing pTalpha-Δ48.41BB or full-length pTalpha were also included, displaying also greater expansion than TCRalpha inactivated cells expressing only the BFP control vector.

### Example 5: optimization of mRNA transfection in T cells using Cytopulse Technology.

### Determination of the optimized cytopulse program

A first set of experiments were performed on non activated PBMCs in order to determine a voltage range in which cells could be transfected. Five different programs were tested as described in Table 14.

**Table 13 : Different cytopulse programs used to determine the minimal voltage required for electroporation in PBMC derived T-cells.**

| | **Group 1** | | | | **Group 2** | | | | **Group 3** | | | |
|---|---|---|---|---|---|---|---|---|---|---|---|---|
| *Cyto-pulse program* | Pulses | V | duration (ms) | Interval (ms) | Pulses | V | duration (ms) | Interval (ms) | Pulses | V | duration (ms) | Interva (ms) |
| 1 | 1 | 600 | 0.1 | 0.2 | 1 | 600 | 0.1 | 100 | 4 | 130 | 0.2 | 2 |
| 2 | 1 | 900 | 0.1 | 0.2 | 1 | 900 | 0.1 | 100 | 4 | 130 | 0.2 | 2 |
| 3 | 1 | 1200 | 0.1 | 0.2 | 1 | 1200 | 0.1 | 100 | 4 | 130 | 0.2 | 2 |
| 4 | 1 | 1200 | 0.1 | 10 | 1 | 900 | 0.1 | 100 | 4 | 130 | 0.2 | 2 |
| 5 | 1 | 900 | 0.1 | 20 | 1 | 600 | 0.1 | 100 | 4 | 130 | 0.2 | 2 |

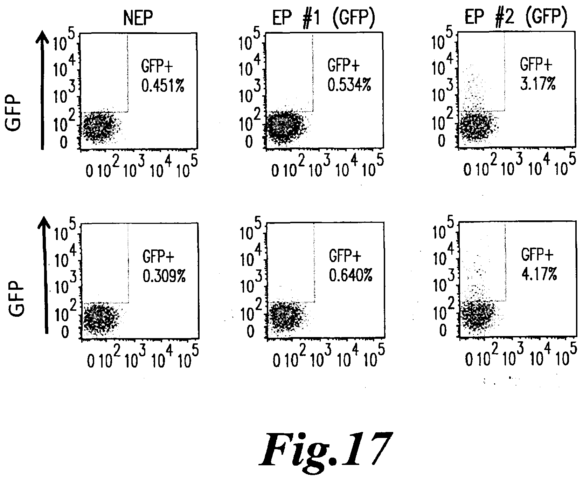

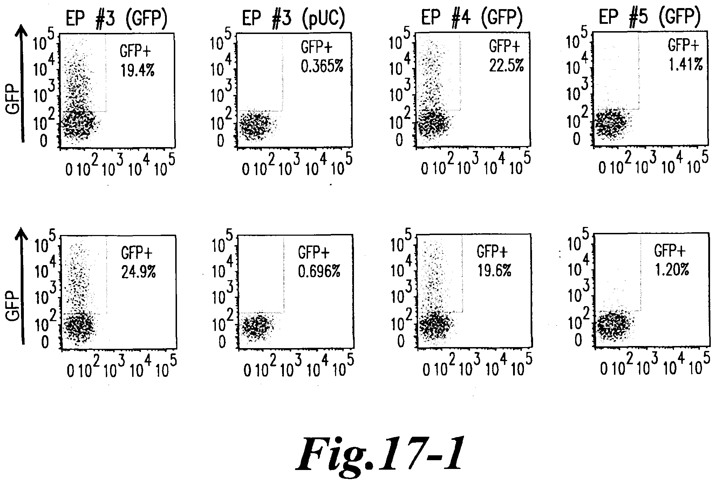

3 or 6 million of cells were electroporated in 0.4 cm gap cuvette (30 or 15×10⁶ cells/ml) with 20 µg of plasmids encoding GFP and control plasmids pUC using the different Cytopulse programs. 24 hours post electroporation, GFP expression was analyzed in electroporated cells by flow cytometry to determine the efficiency of transfection. The data shown in Figure 17 indicates the minimal voltage required for plasmid electroporation in PBMC derived T cells. These results demonstrate that the cytopulse program 3 and 4 allow an efficient transformation of T cells (EP#3 and #4).

### Electroporation of mRNA of purified Tcells activated

After determining the best cytopulse program that allows an efficient DNA electroporation of T cells, we tested whether this method was applicable to the mRNA electroporation.

5x10⁶ purified T cells preactivated 6 days with PHA/IL2 were resupended in cytoporation buffer T (BTX-Harvard apparatus) and electroporated in 0.4 cm cuvettes with 10µg of mRNA encoding GFP or 20µg of plasmids encoding GFP or pUC using the preferred cytopulse program as determined in the previous section (table 14).

**Table 15: Cytopulse program used to electroporate purified T-cells.**

| | **Group 1** | | | | **Group 2** | | | | **Group 3** | | | |
|---|---|---|---|---|---|---|---|---|---|---|---|---|
| *Cyto-pulse program* | Pulse | V | duration (ms) | Interval (ms) | Pulse | V | duration (ms) | Interval (ms) | Pulse | V | duration (ms) | Interval (ms) |
| 3 | 1 | 1200 | 0.1 | 0.2 | 1 | 1200 | 0.1 | 100 | 4 | 130 | 0.2 | 2 |

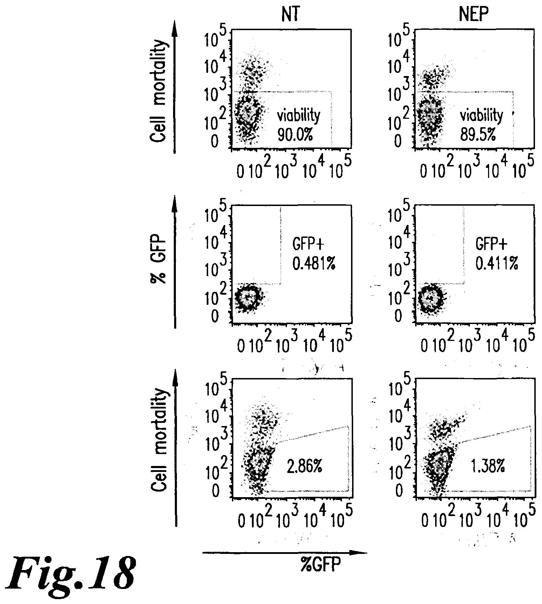

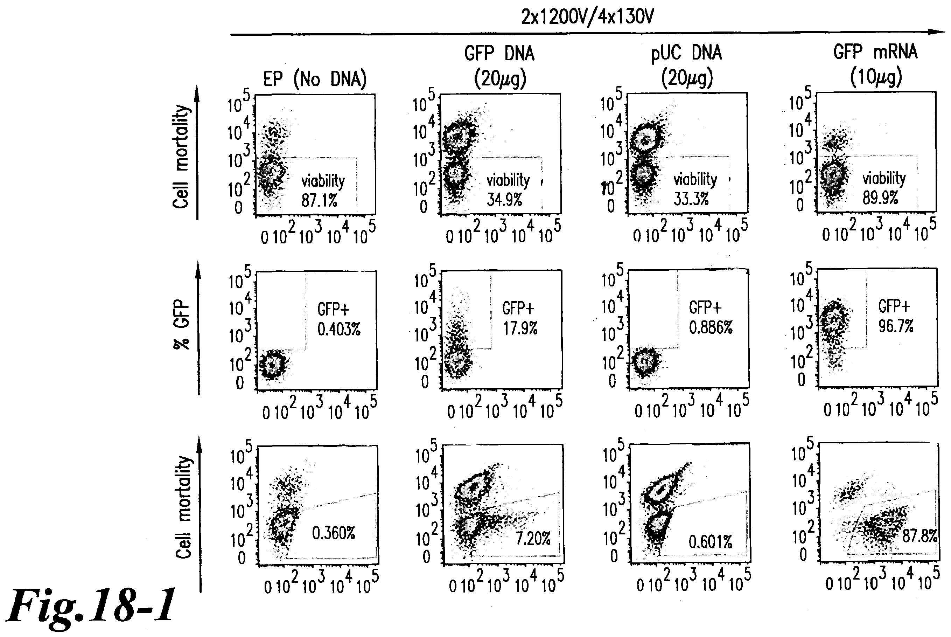

48h after transfection cells were stained with viability dye (eFluor-450) and the cellular viability and % of viable GFP+ cells was determined by flow cytometry analysis (Figure 18).

The data shown in Figure 18 indicates that the electroporation of RNA with the optimal condition determined here is no toxic and allows transfection of more than 95% of the viable cells.

In synthesis, the whole dataset shows that T-cells can be efficiently transfected either with DNA or RNA. In particular, RNA transfection has no impact on cellular viability and allows uniform expression levels of the transfected gene of interest in the cellular population.

Efficient transfection can be achieved early after cellular activation, independently of the activation method used (PHA/IL-2 or CD3/CD28-coated-beads). The inventors have succeeded in transfecting cells from 72h after activation with efficiencies of >95%. In addition, efficient transfection of T cells after thawing and activation can also be obtained using the same electroporation protocol.

### mRNA electroporation in primary human T cells for TALE-nuclease functional expression

After demonstrating that mRNA electroporation allow efficient expression of GFP in primary human T cells, we tested whether this method was applicable to the expression of other proteins of interest. Transcription activator-like effector nucleases (TALE-nuclease) are site-specific nucleases generated by the fusion of a TAL DNA binding domain to a DNA cleavage domain. They are powerful genome editing tools as they induce double-strand breaks at practically any desired DNA sequence. These double-strand breaks activate Non-homologous end-joining (NHEJ), an error-prone DNA repair mechanism, potentially leading to inactivation of any desired gene of interest. Alternatively, if an adequate repair template is introduced into the cells at the same time, TALE-nuclease-induced DNA breaks can be repaired by homologous recombination, therefore offering the possibility of modifying at will the gene sequence.

We have used mRNA electroporation to express a TALE-nuclease designed to specifically cleave a sequence in the human gene coding for the alpha chain of the T cell antigen receptor (TRAC). Mutations induced in this sequence are expected to result in gene inactivation and loss of TCRαβ complex from the cell surface. TRAC TALE-nuclease RNA or non coding RNA as control are transfected into activated primary human T lymphocytes using Cytopulse technology. The electroporation sequence consisted in 2 pulses of 1200 V followed by four pulses of 130 V as described in Table 15.

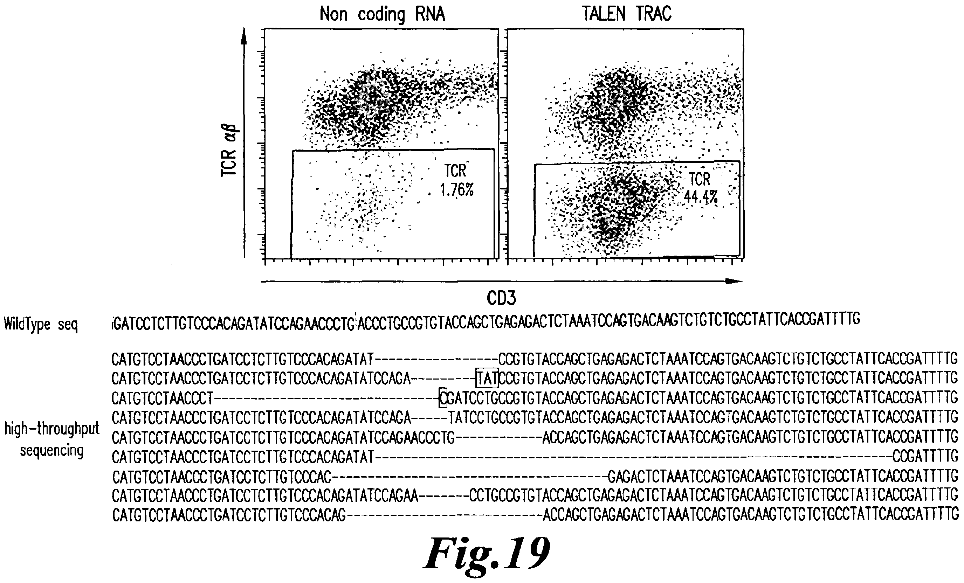

By flow cytometry analysis of TCR surface expression 7 days post electroporation (Figure 19, top panel), we observed that 44% of T cells lost the expression of TCRαβ. We analyzed the genomic DNA of the transfected cells by PCR amplification of the TRAC locus followed by 454 high throughput sequencing. 33% of alleles sequenced (727 out of 2153) contained insertion or deletion at the site of TALE-nuclease cleavage. Figure 19 (bottom panel) shows examples of the mutated alleles.

These data indicate that electroporation of mRNA using cytopulse technology results in functional expression of TRAC TALE-nuclease.

### Electroporation of T cells with a monocistronic mRNA encoding for an anti-CD19 single chain chimeric antigen receptor (CAR):

5×10⁶ T cells preactivated several days (3-5) with anti-CD3/CD28 coated beads and IL2 were resuspended in cytoporation buffer T, and electroporated in 0.4cm cuvettes without mRNA or with 10µg of mRNA encoding a single chain CAR (SEQ ID NO: 73) using the program described in Table 15.

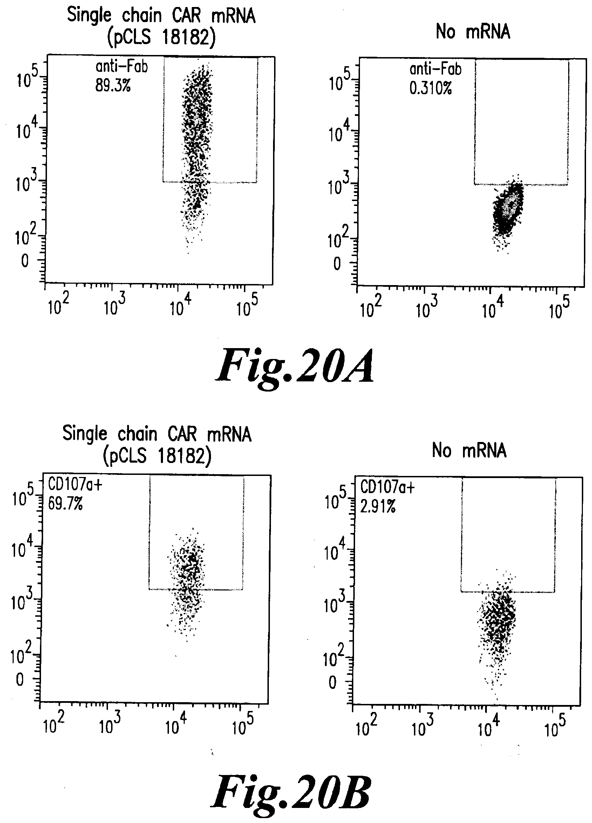

24 hours post electroporation, cells were stained with a fixable viability dye eFluor-780 and a PE-conjugated goat anti mouse IgG F(ab')2 fragment specific to assess the cell surface expression of the CAR on the live cells. The data is shown in the figure 20. A indicates that the vast majority of the live T cells electroporated with the monocitronic mRNA described previously express the CAR at their surface. 24 hours post electroporation, T cells were cocultured with Daudi (CD19⁺) cells for 6 hours and analyzed by flow cytometry to detect the expression of the degranulation marker CD107a at their surface (Betts, Brenchley et al. 2003).

The data shown in figure 20 indicates that the majority of the cells electroporated with the monocistronic mRNA described previously degranulate in the presence of target cells expressing CD19. These results clearly demonstrate that the CAR expressed at the surface of electroporated T cells is active.

### Electroporation of T cells with a polycistronic mRNA encoding for an anti-CD19 multisubunit chimeric antigen receptor (CAR):

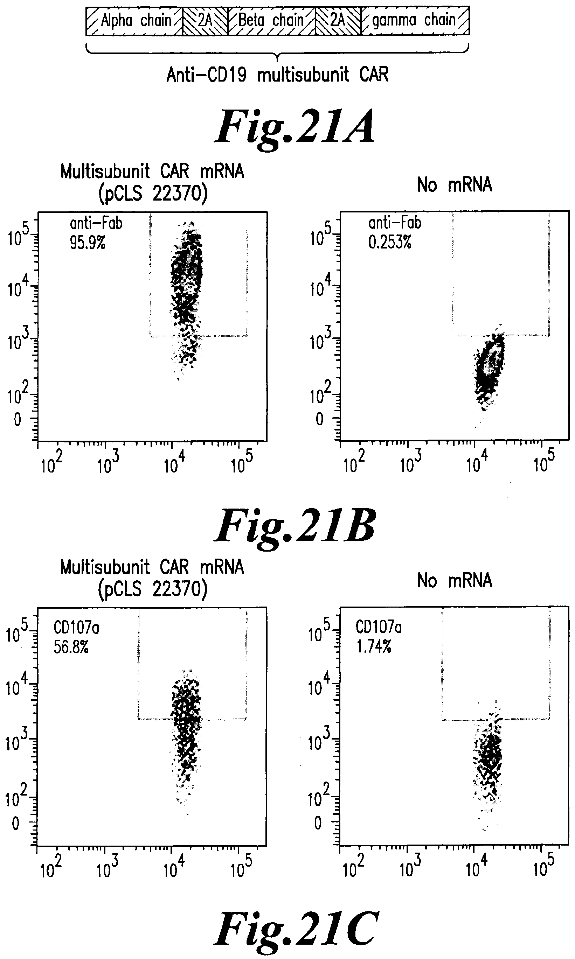

5×10⁶ T cells preactivated several days (3-5) with anti CD3/CD28 coated beads and IL2 were electroporated in cytoporation buffer T, and electroporated in 0.4cm cuvettes without mRNA or with 45µg of mRNA encoding a multi-chain CAR (SEQ ID NO: 125, encoded by SEQ ID NO: 126, Figure 21A and figure 4B (csm4)) using the program as described in Table 15.

24 hours post electroporation, cells were stained with a fixable viability dye eFluor-780 and a PE-conjugated goat anti mouse IgG F(ab')2 fragment specific to assess the cell surface expression of the CAR on the live cells. The data shown in Figure 21 indicates that the vast majority of the live T cells electroporated with the polycistronic mRNA described previously express the CAR at their surface.

24 hours post electroporation, T cells were cocultured with Daudi (CD19⁺) for 6 hours and analyzed by flow cytometry to detect the expression of the degranulation marker CD107a at their surface. The data shown in Figure 21 indicates that the majority of the cells electroporated with the polycistronic mRNA described previously degranulate in the presence of target cells expressing CD19. These results clearly demonstrate that the CAR expressed at the surface of electroporated T cells is active.

### List of references

Arnould, S., P. Chames, et al. (2006). "Engineering of large numbers of highly specific homing endonucleases that induce recombination on novel DNA targets." J Mol Biol 355(3): 443-58.

Betts, M. R., J. M. Brenchley, et al. (2003). "Sensitive and viable identification of antigen-specific CD8+ T cells by a flow cytometric assay for degranulation." J Immunol Methods 281(1-2): 65-78.

Boch, J., H. Scholze, et al. (2009). "Breaking the code of DNA binding specificity of TAL-type III effectors." Science 326(5959): 1509-12.

Boni, A., P. Muranski, et al. (2008). "Adoptive transfer of allogeneic tumor-specific T cells mediates effective regression of large tumors across major histocompatibility barriers." Blood 112(12): 4746-54.

Brahmer, J. R., C. G. Drake, et al. (2010). "Phase I study of single-agent anti-programmed death-1 (MDX-1106) in refractory solid tumors: safety, clinical activity, pharmacodynamics, and immunologic correlates." J Clin Oncol 28(19): 3167-75.

Bucks, C. M., J. A. Norton, et al. (2009). "Chronic antigen stimulation alone is sufficient to drive CD8+ T cell exhaustion." J Immunol 182(11): 6697-708.

Carrasco, Y. R., A. R. Ramiro, et al. (2001). "An endoplasmic reticulum retention function for the cytoplasmic tail of the human pre-T cell receptor (TCR) alpha chain: potential role in the regulation of cell surface pre-TCR expression levels." J Exp Med 193(9): 1045-58.

Cermak, T., E. L. Doyle, et al. (2011). "Efficient design and assembly of custom TALEN and other TAL effector-based constructs for DNA targeting." Nucleic Acids Res 39(12): e82.

Chames, P., J. C. Epinat, et al. (2005). "In vivo selection of engineered homing endonucleases using double-strand break induced homologous recombination." Nucleic Acids Res 33(20): e 178.

Christian, M., T. Cermak, et al. (2010). "Targeting DNA double-strand breaks with TAL effector nucleases." Genetics 186(2): 757-61.

Cong, L., F. A. Ran, et al. (2013). "Multiplex genome engineering using CRISPR/Cas systems." Science 339(6121): 819-23.

Critchlow, S. E. and S. P. Jackson (1998). "DNA end-joining: from yeast to man." Trends Biochem Sci 23(10): 394-8.

Deltcheva, E., K. Chylinski, et al. (2011). "CRISPR RNA maturation by trans-encoded small RNA and host factor RNase III." Nature 471(7340): 602-7.

Deng, D., C. Yan, et al. (2012). "Structural basis for sequence-specific recognition of DNA by TAL effectors." Science 335(6069): 720-3.

Dessarthe, B., A. Thedrez, et al. (2013). "CRTAM receptor engagement by Necl-2 on tumor cells triggers cell death of activated Vgamma9Vdelta2 T cells." J Immunol 190(9): 4868-76.

Epinat, J. C., S. Arnould, et al. (2003). "A novel engineered meganuclease induces homologous recombination in yeast and mammalian cells." Nucleic Acids Res 31(11): 2952-62.

Garneau, J. E., M. E. Dupuis, et al. (2010). "The CRISPR/Cas bacterial immune system cleaves bacteriophage and plasmid DNA." Nature 468(7320): 67-71.

Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.

Geissler, R., H. Scholze, et al. (2011). "Transcriptional activators of human genes with programmable DNA-specificity." PLoS One 6(5): e19509.

Grange, M., G. Verdeil, et al. (2013). "Active STATS regulates T-bet and eomesodermin expression in CD8 T cells and imprints a T-bet-dependent Tc1 program with repressed IL-6/TGF-beta1 signaling." J Immunol 191(7): 3712-24.

Heinrich, P. C., I. Behrmann, et al. (2003). "Principles of interleukin (IL)-6-type cytokine signalling and its regulation." Biochem J 374(Pt 1): 1-20.

Huang, P., A. Xiao, et al. (2011). "Heritable gene targeting in zebrafish using customized TALENs." Nat Biotechnol 29(8): 699-700.

Ikehara, Y., S. K. Ikehara, et al. (2004). "Negative regulation of T cell receptor signaling by Siglec-7 (p70/AIRM) and Siglec-9." J Biol Chem 279(41): 43117-25.

Jena, B., G. Dotti, et al. (2010). "Redirecting T-cell specificity by introducing a tumor-specific chimeric antigen receptor." Blood 116(7): 1035-44.

Jinek, M., K. Chylinski, et al. (2012). "A programmable dual-RNA-guided DNA endonuclease in adaptive bacterial immunity." Science 337(6096): 816-21.

Lackner, G., N. Moebius, et al. (2011). "Complete genome sequence of Burkholderia rhizoxinica, an Endosymbiont of Rhizopus microsporus." J Bacteriol 193(3): 783-4.

Li, L., M. J. Piatek, et al. (2012). "Rapid and highly efficient construction of TALE-based transcriptional regulators and nucleases for genome modification." Plant Mol Biol 78(4-5): 407-16.

Li, T., S. Huang, et al. (2011). "Modularly assembled designer TAL effector nucleases for targeted gene knockout and gene replacement in eukaryotes." Nucleic Acids Res 39(14): 6315-25.

Lozano, E., M. Dominguez-Villar, et al. (2011). "The TIGIT/CD226 axis regulates human T cell function." J Immunol 188(8): 3869-75.

Ma, J. L., E. M. Kim, et al. (2003). "Yeast Mre11 and Rad1 proteins define a Ku-independent mechanism to repair double-strand breaks lacking overlapping end sequences." Mol Cell Biol 23(23): 8820-8.

Mahfouz, M. M., L. Li, et al. (2012). "Targeted transcriptional repression using a chimeric TALE-SRDX repressor protein." Plant Mol Biol 78(3): 311-21.

Mahfouz, M. M., L. Li, et al. (2011). "De novo-engineered transcription activator-like effector (TALE) hybrid nuclease with novel DNA binding specificity creates double-strand breaks." Proc Natl Acad Sci U S A 108(6): 2623-8.

Mak, A. N., P. Bradley, et al. (2012). "The crystal structure of TAL effector PthXo1 bound to its DNA target." Science 335(6069): 716-9.

Mali, P., L. Yang, et al. (2013). "RNA-guided human genome engineering via Cas9." Science 339(6121): 823-6.

Meyaard, L., G. J. Adema, et al. (1997). "LAIR-1, a novel inhibitory receptor expressed on human mononuclear leukocytes." Immunity 7(2): 283-90.

Miller, J. C., S. Tan, et al. (2011). "A TALE nuclease architecture for efficient genome editing." Nat Biotechnol 29(2): 143-8.

Morbitzer, R., P. Romer, et al. (2011). "Regulation of selected genome loci using de novo-engineered transcription activator-like effector (TALE)-type transcription factors." Proc Natl Acad Sci U S A 107(50): 21617-22.

Moscou, M. J. and A. J. Bogdanove (2009). "A simple cipher governs DNA recognition by TAL effectors." Science 326(5959): 1501.

Mussolino, C., R. Morbitzer, et al. (2011). "A novel TALE nuclease scaffold enables high genome editing activity in combination with low toxicity." Nucleic Acids Res 39(21): 9283-93.

Nicoll, G., J. Ni, et al. (1999). "Identification and characterization of a novel siglec, siglec-7, expressed by human natural killer cells and monocytes." J Biol Chem 274(48): 34089-95.

Pang, S. S., R. Berry, et al. (2010). "The structural basis for autonomous dimerization of the pre-T-cell antigen receptor." Nature 467(7317): 844-8.

Pardoll, D. and C. Drake (2012). "Immunotherapy earns its spot in the ranks of cancer therapy." J Exp Med 209(2): 201-9.

Pardoll, D. M. (2012). "The blockade of immune checkpoints in cancer immunotherapy." Nat Rev Cancer 12(4): 252-64.

Park, T. S., S. A. Rosenberg, et al. (2011). "Treating cancer with genetically engineered T cells." Trends Biotechnol 29(11): 550-7.

Quigley, M., F. Pereyra, et al. (2010). "Transcriptional analysis of HIV-specific CD8+ T cells shows that PD-1 inhibits T cell function by upregulating BATF." Nat Med 16(10): 1147-51.

Robert, C. and C. Mateus (2011). "[Anti-CTLA-4 monoclonal antibody: a major step in the treatment of metastatic melanoma]." Med Sci (Paris) 27(10): 850-8.

Rodriguez, P. C., D. G. Quiceno, et al. (2007). "L-arginine availability regulates T-lymphocyte cell-cycle progression." Blood 109(4): 1568-73.

Sander, J. D., L. Cade, et al. (2011). "Targeted gene disruption in somatic zebrafish cells using engineered TALENs." Nat Biotechnol 29(8): 697-8.

Shin, H., S. D. Blackburn, et al. (2009). "A role for the transcriptional repressor Blimp-1 in CD8(+) T cell exhaustion during chronic viral infection." Immunity 31(2): 309-20.

Smith, J., S. Grizot, et al. (2006). "A combinatorial approach to create artificial homing endonucleases cleaving chosen sequences." Nucleic Acids Res 34(22): e149.

Sorek, R., C. M. Lawrence, et al. (2013). "CRISPR-mediated Adaptive Immune Systems in Bacteria and Archaea." Annu Rev Biochem.

Stoddard, B. L. (2005). "Homing endonuclease structure and function." Q Rev Biophys 38(1): 49-95.

Tesson, L., C. Usal, et al. (2011). "Knockout rats generated by embryo microinjection of TALENs." Nat Biotechnol 29(8): 695-6.

Thaventhiran, J. E., A. Hoffmann, et al. (2012). "Activation of the Hippo pathway by CTLA-4 regulates the expression of Blimp-1 in the CD8+ T cell." Proc Natl Acad Sci U S A 109(33): E2223-9.

Torikai H. et al. (14 June 2012) Blood, vol. 119, no. 24, pages 5697-5705.

Uttenthal B.J. et al (June 2012) Journal of Gene Medecine, JOHN WILEY & SONS, INC, US, vol. 14, no. 6, pages 386-399.

Weber, E., R. Gruetzner, et al. (2011). "Assembly of designer TAL effectors by Golden Gate cloning." PLoS One 6(5): e19722.

Wolchok, J. D., H. Kluger, et al. "Nivolumab plus ipilimumab in advanced melanoma." N Engl J Med 369(2): 122-33.

Zhang, F., L. Cong, et al. (2011). "Efficient construction of sequence-specific TAL effectors for modulating mammalian transcription." Nat Biotechnol 29(2): 149-53.

Zhang, J. Q., G. Nicoll, et al. (2000). "Siglec-9, a novel sialic acid binding member of the immunoglobulin superfamily expressed broadly on human blood leukocytes." J Biol Chem 275(29): 22121-6.

## Claims

1. A method of preparing human primary T-cell(s) for immunotherapy comprising:
(a) providing human T-cells, from a primary cell culture or from a blood sample;
(b) genetically modifying said T-cells by introducing into said T-cells rare-cutting endonucleases able to selectively inactivate by DNA cleavage at least two genes:
- one gene encoding the immune checkpoint protein PD1, and
- one gene encoding TCR alpha orTCR beta, and
(c) expanding said cells,
wherein said method further comprises introducing into said T-cells a chimeric antigen receptor (CAR) directed against CD19 antigen.

2. The method according to claim 1, wherein said rare-cutting endonucleases are encoded by mRNA.

3. The method according to any one of claim 1 to 2 which comprises introducing one or more rare-cutting endonuclease(s) into said cell in step (b) by way of RNA electroporation.

4. The method according to any one of claims 1 to 3, wherein said at least one rare-cutting endonuclease is a TALE-nuclease.

5. The method according to claim 4, wherein said at least one TALE-nuclease is directed against one of the gene target sequences of PD1 selected from SEQ ID NO: 77 and SEQ ID NO: 78.

6. The method according to claim 1, wherein said chimeric antigen receptor is a multi-chain chimeric antigen receptor.

7. The method of claim 6, wherein said CAR comprises at least one signal-transducing domain, wherein said signal-transducing domain is CD137 (4-1BB).

8. The method of anyone of claims 1-7, wherein said CAR comprises the amino acid sequence SEQ ID NO: 73.

9. The method according to any one of claims 1 to 8, wherein said T-cell(s) in step a) are derived from inflammatory T-lymphocytes, cytotoxic T-lymphocytes, regulatory T-lymphocytes or helper T-lymphocytes and/or from CD4+ T-lymphocytes and/or CD8+ T-lymphocytes.

10. An isolated human primary T-cell obtainable by the method according to any one of claims 1 to 9 or a subculture thereof, in which at least two genes have been inactivated, a first gene encoding the immune checkpoint protein PD1, and a second gene encoding TCR alpha or TCR beta and further comprising an exogenous polynucleotide sequence encoding a Chimeric Antigen Receptor directed against CD19 antigen.

11. The isolated T-cell according to claim 10 for use as a medicament.

12. An isolated T-cell for use according to claim 11 for use as a therapeutic product for treating a cancer.

13. An isolated T-cell for use according to claim 11, for use as a therapeutic product for treating lymphoma or leukemia.

14. A pharmaceutical composition comprising at least one isolated human T-cell according to claim 10.

15. A population of modified human T-cells prepared by the method according to any one of claims 1 to 9 for use in a therapeutic treatment.

16. The population for use according to claim 15, wherein said patient is diagnosed with cancer.

## Patentansprüche

1. Verfahren zur Vorbereitung von menschlichen primären T-Zellen für die Immuntherapie, umfassend:
(a) Bereitstellen von menschlichen T-Zellen aus einer primären Zellkultur oder aus einer Blutprobe;
(b) genetisches Modifizieren der T-Zellen durch Einführen von selten schneidenden Endonuklease in die T-Zellen, die in der Lage sind, selektiv mindestens zwei Gene durch DNA-Spaltung zu inaktivieren:
- ein Gen, das das Immun-Checkpoint-Protein PD1 codiert, und
- ein Gen, das TCR alpha oder TCR beta codiert, und
(c) Erweitern der Zellen,
wobei das Verfahren ferner das Einführen eines gegen das CD19-Antigen gerichteten chimären Antigenrezeptors (CAR) in die T-Zellen umfasst.

2. Verfahren nach Anspruch 1, wobei die selten schneidenden Endonuklease durch mRNA codiert sind.

3. Verfahren nach einem der Ansprüche 1 bis 2, das das Einführen einer oder mehrerer selten schneidenden Endonuklease(s) in die Zelle in Schritt (b) mittels RNA-Elektroporation umfasst.

4. Verfahren nach einem der Ansprüche 1 bis 3, wobei die mindestens eine selten schneidende Endonuklease eine TALE-Nuklease ist.

5. Verfahren nach Anspruch 4, wobei die mindestens eine TALE-Nuklease gegen eine der Genzielsequenzen von PD1, ausgewählt aus SEQ ID NO: 77 und SEQ ID NO: 78, gerichtet ist.

6. Verfahren nach Anspruch 1, wobei der chimäre Antigenrezeptor ein mehrkettiger chimärer Antigenrezeptor ist.

7. Verfahren nach Anspruch 6, wobei das CAR mindestens eine signalübertragende Domäne umfasst, wobei die signalübertragende Domäne CD137 (4-1BB) ist.

8. Verfahren nach einem der Ansprüche 1-7, wobei das CAR die Aminosäuresequenz SEQ ID NO: 73 umfasst.

9. Verfahren nach einem der Ansprüche 1 bis 8, wobei die T-Zelle(n) in Schritt a) von entzündlichen T-Lymphozyten, zytotoxischen T-Lymphozyten, regulatorischen T-Lymphozyten oder Helfer-T-Lymphozyten und/oder von CD4+ T-Lymphozyten und/oder CD8+ T-Lymphozyten stammen.

10. Isolierte humane primäre T-Zelle, erhältlich nach dem Verfahren nach einem der Ansprüche 1 bis 9, oder eine Subkultur davon, in der mindestens zwei Gene inaktiviert wurden, ein erstes Gen, das für das Immun-Checkpoint-Protein PD1 codiert, und ein zweites Gen, das für TCR alpha oder TCR beta codiert, und die ferner eine exogene Polynukleotidsequenz umfasst, die für einen gegen CD19-Antigen gerichteten chimären Antigenrezeptor codiert.

11. Isolierte T-Zelle nach Anspruch 10 zur Verwendung als Medikament.

12. Isolierte T-Zelle zur Verwendung nach Anspruch 11 zur Verwendung als therapeutisches Produkt zur Behandlung eines Krebses.

13. Isolierte T-Zelle zur Verwendung nach Anspruch 11, zur Verwendung als therapeutisches Produkt zur Behandlung von Lymphomen oder Leukämie.

14. Pharmazeutische Zusammensetzung, die mindestens eine isolierte menschliche T-Zelle umfasst, nach Anspruch 10.

15. Population modifizierter menschlicher T-Zellen, zubereitet nach dem Verfahren nach einem der Ansprüche 1 bis 9 zur Verwendung in einer therapeutischen Behandlung.

16. Population zur Verwendung nach Anspruch 15, wobei bei dem Patienten Krebs diagnostiziert wird.

## Revendications

1. Procédé de préparation de lymphocyte(s) T primaire(s) humain(s) à des fins d'immunothérapie comprenant :
(a) la fourniture de lymphocytes T humains, provenant d'une culture de cellules primaires ou d'un échantillon de sang ;
(b) la modification génétique desdits lymphocytes T en introduisant dans lesdits lymphocytes T des endonucléases à sites rares capables d'inactiver sélectivement par clivage de l'ADN au moins deux gènes :
- un gène codant pour la protéine de point de contrôle immunitaire PD1, et
- un gène codant pour un TCR alpha ou un TCR bêta, et
(c) la multiplication desdits lymphocytes,
dans lequel ledit procédé comprend en outre l'introduction dans lesdits lymphocytes T d'un récepteur antigénique chimère (CAR) dirigé contre l'antigène CD19.

2. Procédé selon la revendication 1, dans lequel lesdites endonucléases à sites rares sont codées par un ARNm.

3. Procédé selon l'une quelconque des revendications 1 à 2 qui comprend l'introduction d'une ou de plusieurs endonucléase(s) à sites rares dans ledit lymphocyte dans l'étape (b) par électroporation d'ARN.

4. Procédé selon l'une quelconque des revendications 1 à 3, dans lequel ladite au moins une endonucléase à sites rares est une nucléase TALE.

5. Procédé selon la revendication 4, dans lequel ladite au moins une nucléase TALE est dirigée contre l'une des séquences géniques cibles de PD1 sélectionnées parmi SEQ ID NO : 77 et SEQ ID NO : 78.

6. Procédé selon la revendication 1, dans lequel ledit récepteur antigénique chimère est un récepteur antigénique chimère multichaîne.

7. Procédé selon la revendication 6, dans lequel ledit CAR comprend au moins un domaine de transduction du signal, dans lequel ledit domaine de transduction du signal est CD137 (4-1BB).

8. Procédé selon l'une quelconque des revendications 1 à 7, dans lequel ledit CAR comprend la séquence d'acides aminés SEQ ID NO : 73.

9. Procédé selon l'une quelconque des revendications 1 à 8, dans lequel lesdits lymphocytes T dans l'étape a) sont dérivés de lymphocytes T inflammatoires, de lymphocytes T cytotoxiques, de lymphocytes T régulateurs ou de lymphocytes T auxiliaires et/ou de lymphocytes T CD4+ et/ou de lymphocytes T CD8+.

10. Lymphocyte T primaire humain isolé pouvant être obtenu par le procédé selon l'une quelconque des revendications 1 à 9 ou une sous-culture de celui-ci, dans lequel au moins deux gènes ont été inactivés, un premier gène codant pour la protéine de point de contrôle immunitaire PD1, et un second gène codant pour un TCR alpha ou un TCR bêta et comprenant en outre une séquence polynucléotidique exogène codant pour un récepteur antigénique chimère dirigé contre l'antigène CD19.

11. Lymphocyte T isolé selon la revendication 10 pour une utilisation comme médicament.

12. Lymphocyte T isolé pour une utilisation selon la revendication 11 pour une utilisation comme produit thérapeutique pour traiter un cancer.

13. Lymphocyte T isolé pour une utilisation selon la revendication 11, pour une utilisation comme produit thérapeutique pour traiter un lymphome ou une leucémie.

14. Composition pharmaceutique comprenant au moins un lymphocyte T humain isolé selon la revendication 10.

15. Population de lymphocytes T humains modifiés préparés par le procédé selon l'une quelconque des revendications 1 à 9 pour une utilisation dans un traitement thérapeutique.

16. Population pour une utilisation selon la revendication 15, dans laquelle ledit patient est diagnostiqué avec un cancer.
